(19) Europäisches Patentamt
European Patent Office
Office européen des brevets

(11) EP 1 463 794 B1

(12) **EUROPEAN PATENT SPECIFICATION**

(45) Date of publication and mention of the grant of the patent:
**17.06.2009 Bulletin 2009/25**

(21) Application number: **03701299.4**

(22) Date of filing: **09.01.2003**

(51) Int Cl.:
***C11D 1/645*** *(2006.01)* ***A61K 8/49*** *(2006.01)*
***A61Q 5/02*** *(2006.01)* ***A61Q 5/12*** *(2006.01)*

(86) International application number:
**PCT/US2003/000717**

(87) International publication number:
**WO 2003/060048 (24.07.2003 Gazette 2003/30)**

(54) **IMMIDAZOLINE QUATS**

QUARTERNÄRE IMIDAZOLINVERBINDUNGEN

COMPOSES QUATERNAIRES D'IMIDAZOLINE

(84) Designated Contracting States:
**DE FR GB IT**

(30) Priority: **09.01.2002 US 347170 P**
**13.08.2002 US 403039 P**

(43) Date of publication of application:
**06.10.2004 Bulletin 2004/41**

(73) Proprietor: **Croda, Inc.**
**Edison, NJ 08837 (US)**

(72) Inventors:
- **PEREIRA, Abel**
**Bridgewater, New Jersey 08807 (US)**
- **BARINOVA, Helena, S.**
**Iselin, NJ 08830 (US)**

(74) Representative: **Humphries, Martyn**
**Croda Europe Limited**
**Intellectual Property Department**
**Wilton Centre**
**Wilton**
**Redcar TS10 4RF (GB)**

(56) References cited:
**EP-A- 0 023 335** **JP-A- 58 144 174**
**JP-A- 60 081 376** **US-A- 4 102 795**

- **PATENT ABSTRACTS OF JAPAN vol. 017, no. 391 (C-1087), 22 July 1993 (1993-07-22) & JP 05 070302 A (MIKASA KAGAKU KOGYO KK), 23 March 1993 (1993-03-23)**
- **PLOOG, U.; PETZOLD, M.; UPHUES, G.: "Neuartige Imidazoline und ihre Derivate aus Fettsäuren und Hydroxyalkylaminen" FETTE, SEIFEN, ANSTRICHMITTEL, vol. 82, no. 2, 1980, pages 57-59, XP009051077**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 463 794 B1

## Description

CROSS-REFERENCE TO RELATED APPLICATIONS

This patent application claims the benefit of the filing date of the United States Provisional Application No. 60/347,170, filed January 9, 2002, and the benefit of the filing date of the United States Provisional Application No. 60/403,037, filed August 13, 2002.

BACKGROUND ART

Compounds containing an immidazoline ring

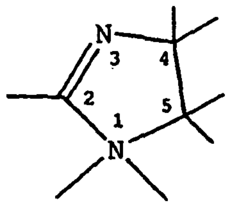

are known in the art. They are disclosed, for example, in U.S. Patents Nos. 4,851,141, 4,452,732, 4,247,538, 4,206,195, 4,187,289, 4,149,551, and 4,102,795.

U.S. Patent No. 4,102,795 discloses compositions for softening fabrics or hair that include immidazoline-based quarternary compounds ("quats") of the formula

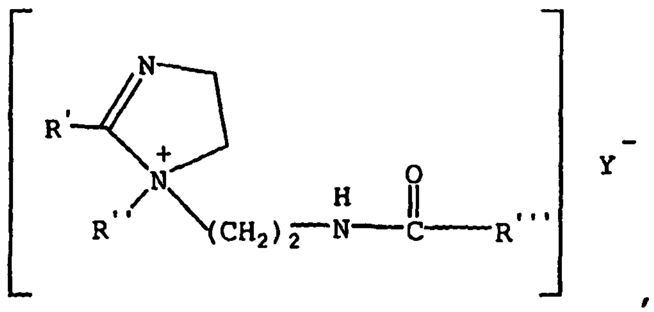

where R' and R''' are alkyl groups having 11 to 22 carbon atoms or β-hydroxyalkyl groups having from 13 to 24 carbon atoms; R'' is a lower alkyl group having 1 to 3 carbon atoms, benzyl group, or the group $-(C_2H_4O)_nH$, where n is 1 to 3; and $Y^-$ is halogen or monoalkyl sulfate. The '795 patent mentions the possibility of mixtures of these compounds, but does not describe any specific mixtures.

U.S. Patent No. 4,452,732 discloses a shampoo containing several components, including immidazoline-based quaternary compounds of the formula

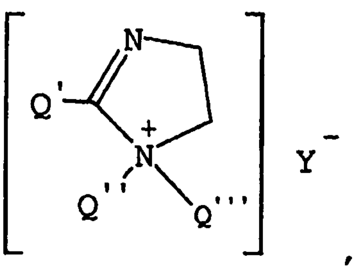

where the groups Q' and Q''' is/are a hydrocarbon group(s) containing 16 to 22 carbon atoms, preferably, 16 to 18 carbon atoms; Q'' is $C_1$-$C_4$ alkyl or hydroxyalkyl group; and $Y^-$ is a compatible anion. The possibility of mixtures is mentioned,

again without specificity.

U.S. Patent No. 4,247,538 discloses compositions for shampooing and conditioning hair that contain several components, including immidazoline-based compounds of the formula

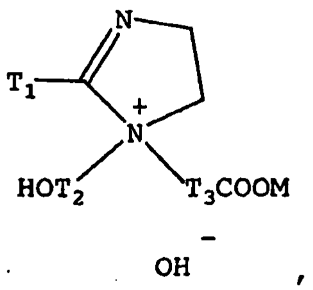

where $T_1$ is an alkyl group having 12 to 18 carbon atoms; $T_2$ is an alkylene or hydroxyalkylene group having 1 to 4 carbon atoms; and M is a water-soluble cation. The '538 patent does not disclose mixtures of these compounds.

JP-A-58 144 174 discloses fabric softening compositions containing 2-hexadecyl-1-methyl-1-[(2-hexadecanoylamino) ethyl]imidazolium chloride (Example 1). A cosmetic or personal care composition having a $C_{16}$-$C_{30}$ substitution content of compositions of the present invention, however, is not disclosed.

SUMMARY OF THE INVENTION

In one aspect, the present invention provides compositions that include a mixture of dialkyl immidazoline quats, where the mixture as a whole has a specified substitution content. Various embodiments are disclosed. In one embodiment, at least a portion of the mixture includes at least one dialkyl immidazoline quat having at least one $C_{16}$-$C_{30}$ alkyl group; the $C_{16-30}$ substitution content of the mixture being from about 10% to about 95% with respect to $C_{10+}$ reference substitution range. In another embodiment, at least a portion of the mixture includes at least one of the dialkyl immidazoline quats has at least one $C_{20}$-$C_{30}$ alkyl group; the $C_{20}$-$_{30}$ substitution content of the mixture being from about 10% to about 95% with respect to $C_{10+}$ reference substitution range. In another embodiment, at least a portion of the mixture includes at least one of the dialkyl immidazoline quats has at least one $C_{20}$-$C_{24}$ alkyl group; the $C_{20-24}$ substitution content of the mixture being from about 10% to about 95% with respect to $C_{10+}$ reference substitution range.

In another aspect, the invention provides compositions that include a mixture of monoalkyl immidazoline quats, where the mixture as a whole has specified substitution content. Various embodiments are disclosed.

In another aspect, the invention provides immidazoline quat compounds of certain chemical structure. Various embodiments are disclosed.

Methods of making immidazoline quats and quat mixtures, personal care and cosmetic products and formulations that contain the immidazoline quats and quat mixtures, methods of making such personal care and cosmetic products and formulations, and methods of using the such immidazoline quats, quat mixtures, and personal care and cosmetic products and formulations are also provided. Various embodiments of the methods are disclosed.

BEST MODE FOR CARRYING OUT INVENTION

For the purposes of the present invention, various terms used herein are defined as follows. A "compound" is a distinct chemical substance having molecules of the same chemical structure. A "compound" is not a mixture of molecules having different chemical structures. A "composition" may include one compound or a mixture of compounds.

An "alkyl group" is any substituent group that includes a chain of one or more carbon atoms. An alkyl group may terminate in alkyl functionality (*e.g.*, $-CH_3$) or non-alkyl functionality (*e.g.*, -Br). Likewise, an alkyl group may connect to the rest of the molecule (MOL) through alkyl functionality (*e.g.*, $-CH_2-$ in $MOL-CH_2CH_3$) or non-alkyl functionality (*e.g.*, $-SO_2-$ in $MOL-SO_2C_3H_8$). Purely for purposes of illustration, each of the groups $-(CH_2)_3-OH$, $-(CH_2)_4-CH_3$, $-CH_3$, and $-C(O)-(CH_2)_5-CH_3$, is an alkyl group. An "alkyl radical" is a chain of one or more carbon atoms connected to one another. Purely for purposes of illustration, the alkyl groups $-(CH_2)_3-OH$, $-(CH_2)_4-CH_3$, and $-C(O)-(CH_2)_5-CH_3$ contain alkyl radicals of the structures $-(CH_2)_3-$, $-(CH_2)_4-CH_3$, and $-(CH_2)_5-CH_3$, respectively.

Carbon chains of alkyl groups and alkyl radicals, and alkyl groups and radicals themselves are described as "$C_x$-$C_y$." An alkyl group containing a $C_x$-$C_y$ alkyl radical is referred to as $C_x$-$C_y$ alkyl. Such description encompasses carbon chains of every length ranging from x to y carbon atoms, inclusive. For example, the description of an alkyl radical as "$C_{10}$-$C_{20}$" encompasses all alternative carbon chains having from 10 to 20 carbon atoms, including carbon chains having 10, 11, 12, 13, 14, 15, 16, 17, 18, 19, and 20 carbon atoms.

Terms such as alkylhydroxy, alkylcarboxy, carboxyalkyl, and the like, are used throughout. Purely for purposes of illustration, an alkylhydroxy group contains a hydroxy group and an alkyl radical, and connects to the rest of the molecule through the alkyl radical; a carboxyalkyl group contains an alkyl radical and a carboxy functionality that connects the carboxy group to the rest of the molecule; an alkylcarboxy group connects to the rest of the molecule through an alkyl radical and terminates in a carboxy functionality. Purely for purposes of illustration, "$C_{10}$-$C_{30}$ alkyl" defines a range of alkyl groups containing alkyl radicals having from 10 to 30 carbon atoms and "$C_{10}$-$C_{30}$ alkylhydroxy" defines a range of alkyl groups containing a hydroxy group and alkyl radicals having from 10 to 30 carbon atoms.

In the compounds described herein, and consistent with the definitions set forth above, the alkyl groups and alkyl radicals, when present, may be substituted or unsubstituted, straight chain or branched, saturated or unsaturated. The substituents of the alkyl groups and alkyl radicals described herein, when present, may include lower alkyl, which contain alkyl radicals having from 1 to 8 carbon atoms (e.g., methyl, ethyl, n-propyl, i-propyl, and butyl); halogenated lower alkyl, such as trifluoromethyl, perfluoroethyl, chloromethyl, and dichloromethyl; arylalkyl, such as benzyl; alkylaryl, such as p-methylbenzyl; halo, such as fluoro, chloro and bromo; carboxy, such as acetoxy and ethylcarboxy; alkylcarboxy, such as acetoxymethyl and acetoxyethyl; arylacetoxy, such as acetylbenzyl; hydroxy; alkoxy, such as methoxy, ethoxy and propoxy; and alkylhydroxy, such as hydroxymethyl and hydroxyethyl.

"Dialkyl immidazoline quats" are compounds the molecules of which include an immidazoline ring, a quaternary nitrogen atom, and two alkyl groups having 10 or more carbon atoms (two $C_{10+}$ alkyl groups). "Monoalkyl immidazoline quats" are compounds the molecules of which include an immidazoline ring, a quaternary nitrogen atom, and one alkyl group having 10 or more carbon atoms (one $C_{10+}$ alkyl group).

Mixtures of quat compounds are described herein in terms of their substitution content, which is a characteristic of the quat mixture as a whole. The substitution content of a quat mixture is a ratio, expressed in the percentage terms, of the molar content of the alkyl groups that fall within a specified substitution range to the molar content of the alkyl groups that fall within a broader, reference substitution range. The molar content values for both the specified substitution range and the reference substitution range are measured for the quat mixture as a whole.

The specified substitution ranges are denoted as "$C_{x-y}$", indicating a range alkyl groups or alkyl radicals having from x to y carbon atoms. The reference substitution ranges are denoted as "$C_{X-Y}$" or "$C_{10+}$". "$C_{10+}$" indicates a range of alkyl group or alkyl radicals having 10 or more carbon atoms. "$C_{X-Y}$" indicates a range of alkyl groups or alkyl radicals having from X to Y carbon atoms.

The quat mixtures are described in terms of their "$C_{x-y}$ content" or "$C_{x-y}$ substitution content". The $C_{10+}$ reference range is the default substitution range. Thus, unless specified otherwise, a $C_{x-y}$ substitution content of a quat mixture (abbreviated in the exemplified mixtures throughout as "$S_{x-y}$") is the ratio, expressed in the percentage terms, of the molar content of alkyl groups that fall within a $C_{x-y}$ range ("$M_{x-y}$") to the molar content of the alkyl groups that fall within the $C_{10+}$ range ("$M_{10+}$"): $S_{x-y} = M_{x-y} / M_{10+} \times 100\%$, where both $M_{x-y}$ and $M_{10+}$ are measured for the mixture as a whole. If any reference range other than $C_{10+}$ is used to describe a quat mixture (e.g., $C_{x-y}$ range), the substitution content of the mixture (abbreviated in the exemplified mixtures as "$S_{x-y/X-Y}$") and is the ratio of the molar content of alkyl groups that fall within a specified $C_{x-y}$ range ("$M_{x-y}$") to the molar content of the alkyl groups that fall within the $C_{X-Y}$ reference range ("$M_{X-Y}$") : $S_{x-y/X-Y}= M_{x-y} / M_{X-Y} \times 100\%$.

To illustrate, consider the mixture M1 that contains a single molecule of dialkyl quat A1 and a single molecule of different dialkyl immidazoline quat A2. By definition, each of dialkyl quats A1 and A2 has two $C_{10+}$ alkyl groups. Suppose, the molecule of quat A1 has one $C_{20}$ alkyl group and one $C_{12}$ alkyl group, and the molecule of quat A2 has two $C_{20}$ alkyl groups. Suppose also, the mixture M1 is to be characterized in terms of its $C_{16-30}$ substitution content ($S_{16-30}$(M1)), *e.g.*, the narrower, specified range is $C_{16-30}$ and the broader, reference range is $C_{10+}$.

The $C_{16-30}$ substitution content of the mixture M1 can be calculated as: $S_{16-30}(M1) = M_{16-30}(M1)/M_{10+}(M1) \times 100\%$, where $M_{16-30}$ is the $C_{16-30}$ molar content of the mixture M1 and $M_{10+}$(M1) is the $C_{10+}$ molar content of the mixture M1. Since the solar concentrations of quats A1 and A2 in the mixture M1 are identical (one molecule each), the relative molar concentration may be disregarded, and the absolute numbers of alkyl groups falling within each range may be used instead. Thus, the $C_{16-30}$ substitution content of the mixture M1 as a whole may be calculated as: $S_{16-30}(M1)= N_{16-30}(M1)/N_{10}+(M1) \times 100\%$, where $N_{16-30}$(M1) is the number of alkyl groups in the mixture M1 that fall within the $C_{16-30}$ range and $N_{10+}$(M1) is the number of alkyl groups in the mixture M1 falling within the $C_{10+}$ range.

The first step is to calculate the values of $N_{16-30}$ and $N_{10+}$ for the mixture. To determine $N_{16-30}$ and $N_{10+}$, a substituent group is counted every time it falls within the $C_{10+}$ and $C_{16-30}$ ranges, respectively, for all molecules in the mixture. The same group may be counted more than once. The $C_{20}$ group falls within both the $C_{16-30}$ range and the $C_{10}$+ range and thus should be counted in calculating both $N_{16-30}$(M1) and $N_{10+}$(M1), while the $C_{12}$ group fall only within the $C_{10+}$ range and therefore should be counted only in calculating $N_{10+}$(M1). Performing the calculation for the mixture M1 as a whole, $N_{16-30}$ is 3 (one $C_{20}$ group of quat A1 and two $C_{20}$ group of the quat A2) and $N_{10}$+ is 4 (all four groups are in the $C_{10+}$ range) Therefore, $S_{16-30}$ for the mixture M1 is 75% (3/4 x 100%).

For more complex quat mixtures, molar concentrations of quats in the mixture are taken into account. A non-limiting example illustrates calculation of substitution content for mixture M2 of dialkyl quats A3, A4, and A5. The mixture

M2 is characterized in terms of its $C_{20-30}$ substitution content (the specified substitution range is $C_{20-30}$ and the reference substitution range is $C_{10+}$) :

TABLE 1*

| I | II ($N_{20-30}$) | III ($P_{20-30}$) | IV ($M_0$/$M_{10+}$) | V ($M_{20-30}$) | VI ($S_{20-30}$) |
|---|---|---|---|---|---|
| Quat | Number of alkyl groups in the quat molecule falling in the $C_{20-30}$ range | $C_{20-30}$ molecular content for each quat ((II)/2) | Moles | $C_{20-30}$ molar contribution of each quat ((III) x ((IV)) and $C_{20-30}$ molar content of the mixture | $C_{20-30}$ substitution content of the mixture |
| A3 | 1 (one) | 0.5 | 2 | 1 (0.5 x 2) | |
| A4 | 2 (two) | 1 | 0.75 | 0.75 (1 x 0.75) | |
| A5 | 0 (none) | 0 | 0.75 | 0 (0 x 0.75) | |
| M2 | | | 3.5 | 1.75 | 50% (1.75/3.5) |

*Column (I) identifies quat components of the mixture M2. Column (II) indicates how many alkyl groups of each quat molecule fall within the $C_{20-30}$ range (note that dialkyl quats have two groups in the $C_{10+}$ range ($N_{10+}$ is 2)). Column (IV) provides molar amounts of each quat in the mixture M2 and the total number of quat moles in the mixture M2. Columns (III), (V) and (VI) are explained below.

To calculate the $C_{20-30}$ substitution content ($S_{20-30}$) of the mixture M2, the first step is to determine the $C_{20-30}$ content the molecule of each quat based on its chemical structure. Such substitution content is referred to as $C_{20-30}$ "molecular content" and denoted as "$P_{20-30}$". The $C_{20-30}$ molecular content of a quat is determined by dividing the number of $C_{20}$-$C_{30}$ alkyl groups ($N_{20-30}$) by the number of $C_{10+}$ alkyl groups ($N_{10+}$) in molecule of the quat: $P_{20-30} = N_{20-30}/ N_{10+}$.

In the example, the nature of substitution for quats in the mixture is provided in column (II). The molecule of quat A3 has 1 (one) group that falls in the $C_{20-30}$ range. The number of groups in the $C_{10}$+ range is 2 (two) for all dialkyl quats. Thus, the $C_{20-30}$ molecular content of quat A3 ($P_{20-30}$ (A3)) is 1/2 = 0.5. The $C_{20-30}$ molecular content values for quats A3, A4, and A5 are calculated in the same manner by dividing the values in column (II) by 2, and are shown in column (III).

Next, the $C_{20-30}$ molar contribution of each quat component of the mixture ($M_{20-30}$) is calculated. For this purpose, each quat's $C_{20-30}$ molecular content ($P_{20-30}$, column (III)) is multiplied by the number of moles of the corresponding quat in the mixture ($M_0$, column (IV)): $M_{20-30} = P_{20-30} \times M_0$. The results of the calculations are shown in column (V). In effect, the product of the multiplication is the molar amount of $C_{20-30}$ alkyl groups contributed by each quat component of the mixture M2.

The $C_{20-30}$ molar content of the mixture M2 as a whole ($M_{20-30}$(M2)) is the sum of the $C_{20-30}$ molar contributions of individual quats: $M_{20-30}$ (M2) = $M_{20-30}$ (A3) + $M_{20-30}$ (A4) + $M_{20-30}$ (A5). Referring to column (V), quat A3 contributes 1 mole of $C_{20}$-$C_{30}$ groups ($M_{20-30}$(A3) = 1), quat A4 contributes 0. 75 moles ($M_{20-30}$(A4) = 0.75), and quat A5 contributes 0 moles of $C_{20}$-$C_{30}$ groups ($M_{20-30}$ (A5) = 0). Therefore, the $C_{20-30}$ molar content of the mixture M2 is 1.75 ($M_{20-30}$(M2) = 1 + 0.75 + 0).

The sum of the $C_{10+}$ molar contributions of individual quats is the $C_{10+}$ molar content of the mixture M2 as a whole ($M_{10+}$ (M2)): $M_{10+}$ (M2) = $M_{10+}$ (A3) + $M_{10+}$ (A4) + $M_{10+}$ (A5). Since all dialkyl quats have two alkyl groups in the $C_{10+}$ range, the $C_{10+}$ molar content ($M_{10+}$) of a dialkyl quat component is identical to the number of moles of the quat component ($M_0$). Referring to column (IV), quat A3 contributes 2 mole of $C_{10+}$ groups ($M_{0+}$ (A3) = 2), quat A4 contributes 0.75 moles ($M_{0+}$(A4) = 0.75), and quat A5 contributes 0.75 moles of $C_{10+}$ groups ($M_{0+}$ (A5) = 0.75). Therefore, the $C_{10+}$ molar content of the mixture M2 is 3.5 ($M_{10+}$ (M2) = 2 + 0.75 + 0.75). Finally, the $C_{20-30}$ substitution content of the mixture M2 can be calculated: $S_{20-30}$ (M2) = $M_{20-30}$ (M2) /$M_{10+}$(M2) = 1.75/3.5 = 50% (column (VI)).

Another non-limiting example illustrates calculation of the substitution content for mixtures of monoalkyl quats. Table 2 shows mixture M3 of monoalkyl quats B1, B2, and B3. The mixture M3 is characterized in terms of its $C_{20-24}$ substitution content (the specified substitution range is $C_{20-24}$ and the reference substitution range is $C_{10+}$).

TABLE 2

| I | II ($N_{20-24}$) | III ($P_{20-24}$) | IV ($M_0$//$M_{10+}$) | V ($M_{20-24}$) | VI ($S_{20-24}$) |
|---|---|---|---|---|---|
| Quat | Number of alkyl groups (the $C_{20-24}$ range) that fall in the quat molecule | $C_{20-24}$ molecular content for each quat (II/1) | Moles | $C_{20-24}$ molar contribution of each quat (III x IV) and $C_{20-24}$ molar content the mixture | $C_{20-24}$ substitution content of the mixture (V/IV) |
| B1 | 1 | 1 | 1.5 | 1.5 (1 x 1.5) | |
| B2 | 1 | 1 | 1 | 1 (1 x 1) | |
| B3 | 0 | 0 | 2.5 | 0 (0 x 2.5) | |
| M3 | | | 5 | 2.5 | 2.5/5 x 100% = 50% |

The $C_{20-24}$ substitution content of the mixture M3 ($S_{20-24}$ (M3)) is calculated as follows similarly to the calculations described in reference to the mixture of Table 1:

1. Determine the $C_{20-24}$ molecular content for each monoalkyl quat: $P_{20-24} = N_{20-24}/ N_{10+}$. Since $N_{10+}$ is 1 (one) for all monoalkyl quats, the $C_{20-24}$ molecular content for each quat is calculated as: $P_{20-24} = N_{20-24}/1$. The results are shown in column (III). It is evident that $P_{x-y} = N_{x-y}$ for monoalkyl quats in general. Thus, $N_{x-y}$ may be used instead of $P_{x-y}$ and column (III) is omitted in later examples of monoalkyl quat mixtures.
2. Determine the $C_{20-24}$ molar contributions of quat components by multiplying the number of $C_{20}$-$C_{24}$ groups by molar amount for each quat component: $M_{20-24} = N_{20-24} \times M_0$. The $N_{20-24}$ values in column (II) are multiplied by the corresponding $M_0$ values in column (IV). The results of the calculations are in column (V).
3. Determine the $C_{20-24}$ molar content of the mixture M3 by adding the $C_{20-24}$ molar contributions of the quat components: $M_{20-24}$ (M3) = $M_{20-24}$ (B1) + $M_{20-24}$ (B2) + $M_{20-24}$ (B3). The $M_{20-24}$ values for each quat component in column (V) are added. The calculated $C_{20-24}$ molar content of the mixture M3 is also shown in column (V).
4. Determine the $C_{10+}$ molar content of the mixture M3 by adding the $C_{10+}$ molar contributions of the quat components. Since all monoalkyl quats have one alkyl group in the $C_{10+}$ range, molar amounts of the quats are used: $M_{10+}$(M3) = $M_0$(B1) + $M_0$(B2) + $M_0$(B3). The $M_0$ values for each quat component in column (IV) are added. The calculated $C_{10+}$ molar content of the mixture M3 is shown in column (IV).
5. Determine the $C_{20-24}$ substitution content of the mixture M3 by dividing the $C_{20-24}$ molar content of the mixture M3 by the $C_{10+}$ molar content of the quats in the mixture: $S_{20-24}$ (M3) = $M_{20-24}$ (M3) /$M_{10+}$ (M3). The calculated $C_{20-24}$ substitution content of the mixture M3 is shown in column (VI).

The above definitions and calculation methodologies are used throughout to describe various aspects and embodiments of the invention.

In accordance with one aspect, the invention provides compositions that include mixtures of at least two different dialkyl immidazoline quats; the mixture having specified substitution content. In addition to dialkyl immidazoline quats, the compositions of this aspect of the invention may include other quaternary and non-quaternary compounds. If desired, monoalkyl immidazoline quats and/or other quaternary compounds are substantially excluded from the composition.

In one embodiment, the mixture includes at least one dialkyl immidazoline quat with one or both alkyl groups being $C_{16-30}$ alkyl groups. Thus, at least a portion of the mixture has $C_{16}$-$C_{30}$ alkyl groups, providing the mixture as a whole with $C_{16-30}$ substitution content greater than zero. In this embodiment, the $C_{16-30}$ substitution content of the mixture is from about 10% to about 95%, more preferably, from about 15% to about 80%, yet more preferably, from about 20% to about 70%, yet more preferably, from about 35% to about 60%.

In another embodiment, the mixture includes at least one dialkyl immidazoline quat with one or both alkyl groups being $C_{20}$-$C_{30}$ alkyl groups. Thus, at least a portion of the mixture has $C_{20}$-$C_{30}$ alkyl groups, providing the mixture as a whole with $C_{20-30}$ substitution content greater than zero. In this embodiment, the $C_{20-30}$ substitution content of the mixture is from about 10% to about 95%, more preferably, from about 15% to about 80%, yet more preferably, from about 20% to about 70%, yet more preferably, from about 35% to about 60%.

In yet one embodiment, the mixture includes at least one dialkyl immidazoline quat with one or both alkyl groups being $C_{20}$-$C_{24}$ alkyl groups. Thus, at least a portion of the mixture has $C_{20}$-$C_{24}$ alkyl groups, providing the mixture as a whole with $C_{20-24}$ substitution content greater than zero. In this embodiment, the $C_{20-24}$ substitution content of the mixture is from about 10% to about 95%, more preferably, from about 15% to about 80%, yet more preferably, from about 20%

to about 70%, yet more preferably, from about 35% to about 60%.

Dialkyl immidazoline quats in the mixtures may be selected from compounds of the formula (I):

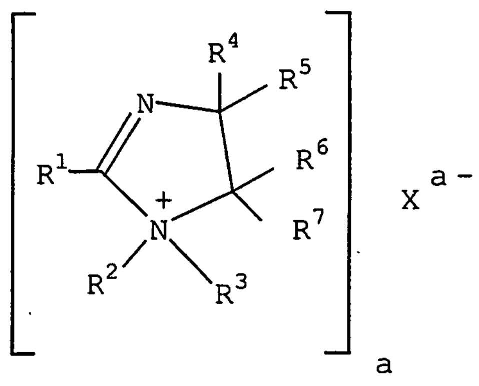

(I) ,

where

X is a salt-forming anion, such as chloride, bromide, iodide, fluoride, sulfate, methyl sulfate, methanebenzylsulfonate, phosphate, nitrite, nitrate, carboxylate, or a mixture thereof, preferably, chloride or methyl sulfate; a is the ionic charge of X;

$R^1$, $R^2$, and $R^3$ are independently hydrogen, $C_1$-$C_{30}$ alkyl, $C_1$-$C_{30}$ alkylhydroxy, $C_1$-$C_{30}$ alkyl amido $R_{(C1-C6)}$ wherein $R_{(C1-C6)}$ is a $C_1$-$C_6$ alkylene or benzyl, $C_1$-$C_{30}$ alkylaryl amido $R_{(C1-C6)}$ or $C_1$-$C_{30}$ alkylhydroxy amido $R_{(C1-C6)}$;

two of $R^1$, $R^2$, and $R^3$ are independently $C_{10}$-$C_{30}$ alkyl, $C_{10}$-$C_{30}$ alkylhydroxy, $C_{10}$-$C_{30}$ alkyl amido $R_{(C1-C6)}$, $C_{10}$-$C_{30}$ alkylaryl amido $R_{(C1-C6)}$, or $C_{10}$-$C_{30}$ alkylhydroxy amido $R_{(C1-C6)}$, and the remaining one of $R^1$, $R^2$ and $R^3$ is hydrogen, $C_1$-$C_8$ alkyl, $C_1$-$C_8$ alkylhydroxy, $C_1$-$C_8$ alkyl amido $R_{(C1-C6)}$, $C_1$-$C_8$ alkylaryl amido $R_{(C1-C6)}$, or $C_1$-$C_8$ alkylhydroxy amido $R_{(C1-C6)}$;

$R^4$, $R^5$, $R^6$, and $R^7$, same or different, are independently hydrogen, alkyl, arylalkyl, alkylaryl, halogen, including bromo and chloro, acetoxy, alkylacetoxy, arylacetoxy, carboxy, alkylcarboxy, hydroxy, or alkoxyhydroxy, preferably, $R^4$, $R^5$, $R^6$, and $R^7$, same or different, are hydrogen or $C_1$-$C_8$ alkyl.

More preferably, the dialkyl immidazoline quats in the mixtures are selected from the compounds of the formula (I) in which $R^1$ is $C_{10}$-$C_{30}$ alkyl or $C_{10}$-$C_{30}$ alkylhydroxy, $R^2$ is $C_1$-$C_6$ alkyl, $R^3$ is $C_{10}$-$C_{30}$ alkyl amido $R_{(C1-C6)}$ or $C_{10}$-$C_{30}$ alkylhydroxy amido $R_{(C1-C6)}$, and $R^4$, $R^5$, $R^6$, and $R^7$ are independently hydrogen or $C_1$-$C_8$ alkyl. Yet more preferably, $R^1$ is $C_{10}$-$C_{30}$ alkyl, $R^2$ is methyl, $R^3$ is $C_{10}$-$C_{30}$ alkyl amido $C_1$-$C_3$ alkylene or $C_{10}$-$C_{30}$ alkylhydroxy amido $C_1$-$C_3$ alkylene, $R^4$, $R^5$, $R^6$, and $R^7$ are hydrogen, and X is chloride or methyl sulfate.

The portion providing the mixture with substitution content greater than $C_{10+}$, such as the $C_{16-30}$, $C_{20-30}$ or $C_{20-24}$ substitution contents, may contain compounds of the formula (I) in which $R^1$ is $C_{16}$-$C_{30}$ alkyl or $C_{16}$-$C_{30}$ alkylhydroxy, and $R^3$ is $C_{10}$-$C_{30}$ alkyl amido $R_{(C1-C6)}$; or compounds of the formula (I) in which $R^1$ is $C_{10}$-$C_{30}$ alkyl or $C_{10}$-$C_{30}$ alkylhydroxy, and $R^3$ is $C_{16}$-$C_{30}$ alkyl amido $R_{(C1-C6)}$; or compounds of the formula (I) in which both $R^1$ and $R^3$ are in the $C_{16-30}$ substitution range. The portion of the mixture may have the same relative variations of the content of the portion for $C_{16}$-$C_{30}$ and $C_{20}$-$C_{24}$ substituents.

Non-limiting examples of dialkyl quats of the formula (I) are quats (1), (2), (3), and (4):

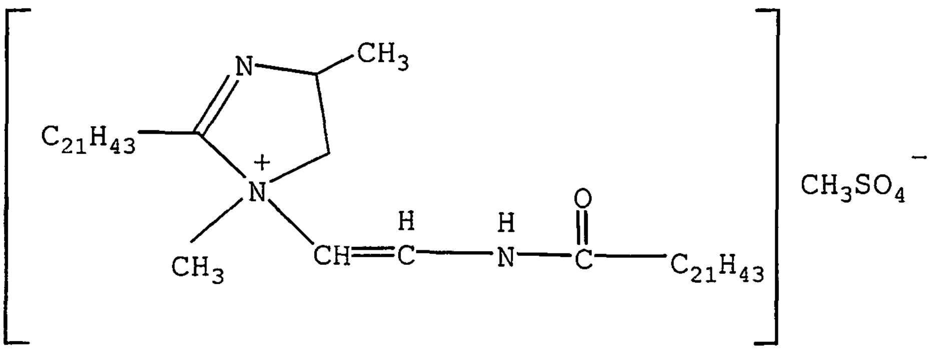

(1) ,

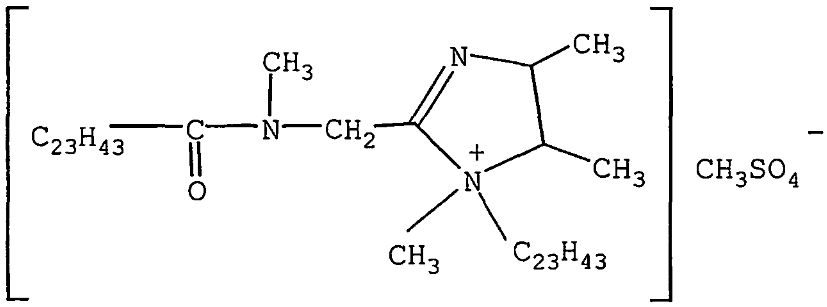

(2) ,

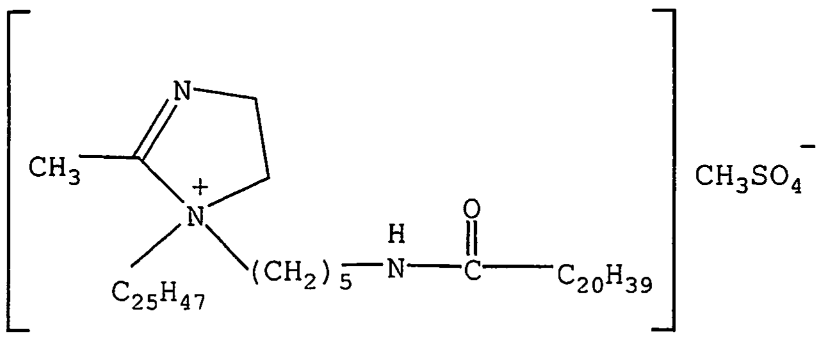

(3) ,

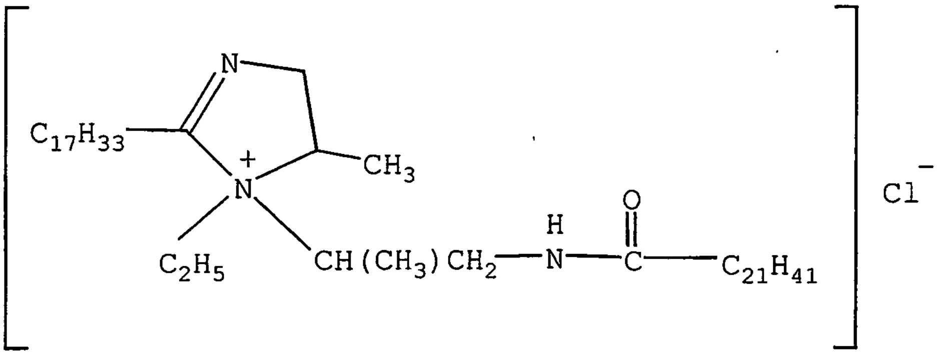

(4)

In another non-limiting example, Table 3 shows mixture M4 of dialkyl quats (1), (2), (3), and (4) and the calculation of the mixture's substitution content. The mixture M4 is characterized in terms of its $C_{20-30}$ substitution content (the specified substitution range is $C_{20-30}$ and the reference substitution range is $C_{10+}$).

TABLE 3*

| I | II ($N_{20-30}$) | III ($P_{20-30}$ = II/2) | IV ($M_0$//$M_{10+}$) | V ($M_{20-30}$ = III x IV) | VI ($S_{20-30}$ = V/IV) |
|---|---|---|---|---|---|
| 1 | 1 | 0.5 | 1 | 0.5 (0.5 x 1) | |
| 2 | 2 | 1 | 2 | 2 (1 x 2) | |
| 3 | 2 | 1 | 2 | 2 (1 x 2) | |
| 4 | 1 | 0.5 | 3 | 1.5 (0.5 x 3) | |
| M4 | | | 8 | 6 | 75% (6/8 x 100%) |
| * Explanations for calculating the $C_{20-30}$ content are provided with reference to Table 1. | | | | | |

The invention also provides compositions that include a mixture of dialkyl immidazoline quats of the formula (II):

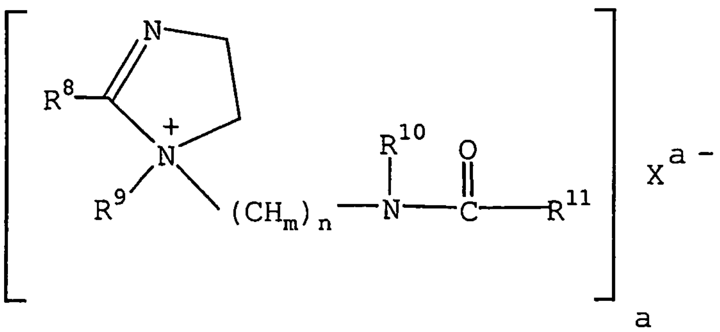

(II)

where

X and a are defined above with reference to the formula (I);
n varies from 1 to 3, preferably, n is 2;
m is 1 or 2, preferably, m is 2;
$R^8$ and $R^{11}$, which may be the same or different, are $C_{16}$-$C_{30}$ alkyl;
$R^9$ is hydrogen or $C_1$-$C_3$ alkyl, preferably, methyl;

$R^{10}$ is hydrogen, alkyl, arylalkyl, alkylaryl, halogen, including bromo and chloro, acetoxy, alkylacetoxy, arylacetoxy, carboxy, alkylcarboxy, hydroxy, or alkoxyhydroxy, preferably, hydrogen or lower alkyl, more preferably, hydrogen.

In addition to dialkyl immidazoline quats of the formula (II), the compositions of this embodiment may include other quaternary and non-quaternary compounds. If desired, monoalkyl immidazoline quats and/or other quaternary compounds may be substantially excluded from the composition.

In one embodiment, at least a portion of the mixture includes at least one quat of the formula (II) in which one or both of $R^8$ and $R^{11}$ is /are $C_{16}$-$C_{24}$ alkyl group(s), and the $C_{16-24}$ substitution content of the mixture with respect to the $C_{16-30}$ reference substitution range varies from about 10% to about 95%, more preferably, from about 15% to about 80%, more preferably, from about 20% to about 80%, yet more preferably, from about 35% to about 60%.

In another embodiment, at least a portion of the mixture includes at least one quat of the formula (II) in which one or both of $R^8$ and $R^{11}$ is/are $C_{20}$-$C_{24}$ alkyl group (s) and the $C_{20-24}$ substitution content of the mixture with respect to the $C_{16-30}$ reference substitution range varies from about 10% to about 95%, more preferably, from about 15% to about 80%, yet more preferably, from about 20% to about 70%, yet more preferably, from about 35% to about 60%.

Non-limiting examples of dialkyl immidazoline quats of the formula (II) are quats (5), (6), (7), and (8):

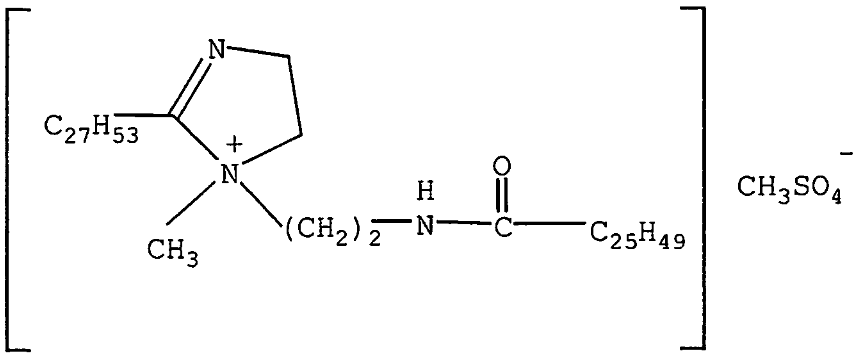

(5) ,

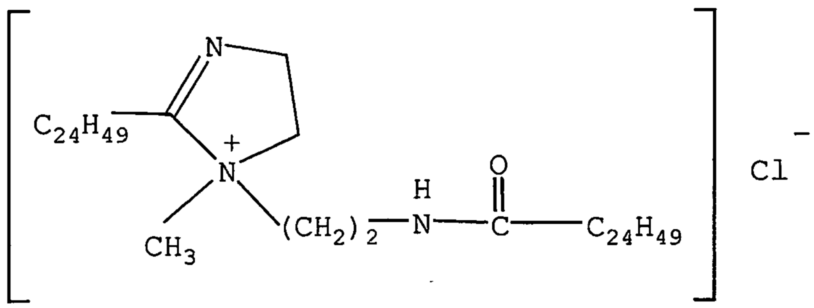

(6) ,

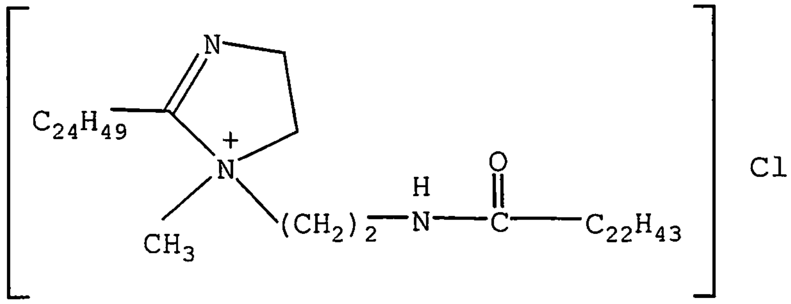

(7)

,

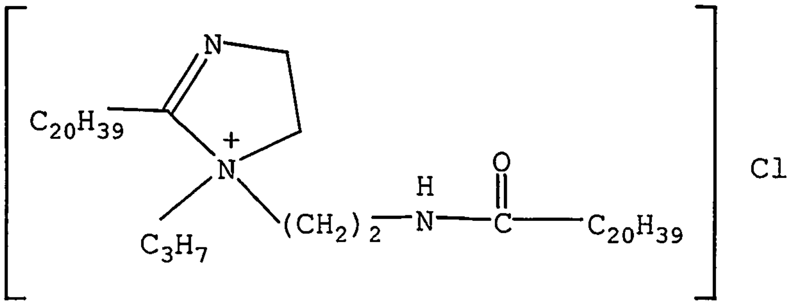

(8)

.

Other non-limiting examples of the quats of the formula (II) are quats (9)-(16) shown in Table 4:

TABLE 4*

| Compound | $R^8$ | $R^{11}$ |
|---|---|---|
| 9 | $C_{18}H_{35}$ | $C_{18}H_{35}$ |
| 10 | $C_{21}H_{40}$ | $C_{21}H_{40}$ |
| 11 | $C_{22}H_{40}$ | $C_{22}H_{40}$ |
| 12 | $C_{16}H_{29}$ | $C_{18}H_{35}$ |
| 13 | $C_{23}H_{40}$ | $C_{23}H_{4}$ |
| 14 | $C_{18}H_{35}$ | $C_{22}H_{41}$ |
| 15 | $C_{18}H_{33}$ | $C_{18}H_{33}$ |
| 16 | $C_{22}H_{40}$ | $C_{22}H_{40}$ |
| *$R^9$ is methyl, $R^{10}$ is hydrogen, m is 2, and n is 2. | | |

In a non-limiting example, Table 5 shows mixture M5 of dialkyl quats (9), (10), and (11) and the calculation of the mixture's substitution content. The mixture M5 is characterized in terms of its $C_{20-24/16-30}$ substitution content (the specified range is $C_{20-24}$ and the reference range is $C_{16-30}$). The $C_{16-30}$ range is used as the reference range, instead of the default $C_{10+}$ reference range, since $R^8$ and $R^{11}$ are $C_{16}$-$C_{30}$ alkyl groups. The combined molar content of $R^8$ and $R^{11}$ groups in the mixture M5 is used in the calculations:

TABLE 5

| I (Quat) | II ($N_{20-24}$) | III ($P_{20-24}$ = II/2) | IV ($M_0$// $M_{10+}$) | V ($M_{20-24}$ = III x IV) | VI ($S_{20-24/16-30}$ = V/IV) |
|---|---|---|---|---|---|
| 9 | 0 | 0 | 1 | 0 (0 x 1) | |
| 10 | 2 | 1 | 0.75 | 0.75 (0.75 x 1) | |
| 11 | 2 | 1 | 0.25 | 0.25 (0.25 x 1) | |
| M5 | | | 2 | 1 | 50% (1/2 x 100%) |

Other non-limiting examples of quat mixtures and calculations of their $C_{20-24}$ content are shown in Tables 6 and 7:

TABLE 6

| I (Quat) | II ($N_{20-24}$) | III ($P_{20-24}$ = II/2) | IV ($M_0$//$M_{10}$ +) | V ($M_{20-24}$ = III x IV) | VI ($S_{20-24/16-30}$ = V/IV) |
|---|---|---|---|---|---|
| 12 | 0 | 0 | 1 | 0 (0 x 1) | |
| 13 | 2 | 1 | 2.5 | 2.5( 1x 2.5) | |
| M6 | | | 3.5 | 2.5 | 71.4% (2.5/3.5 x 100%) |

TABLE 7

| I (Quat) | II ($N_{20-24}$) | III ($P_{20-24}$ = II/2) | IV ($M_0$//$M_{10}$ +) | V ($M_{20-24}$ = III x IV) | VI ($S_{20-24/16-30}$ = V/IV) |
|---|---|---|---|---|---|
| 14 | 1 | 0.5 | 2 | 1 (0.5 x 2) | |
| 15 | 0 | 0 | 1 | 0 (0 x 1) | |
| 16 | 2 | 1 | 1 | 1 (1 x 1) | |
| M7 | | | 4 | 2 | 50% (2/4 x 100%) |

In another aspect, the invention provides a composition that include a dialkyl immidazoline quat of the formula (IA):

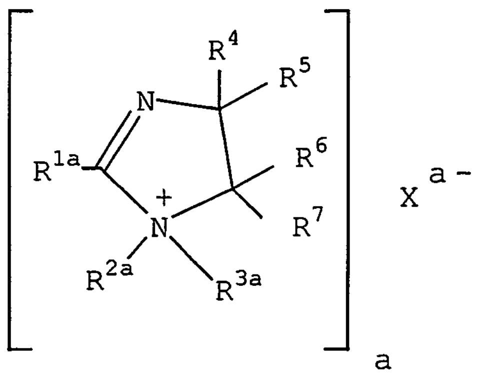

(IA)

,

where X is a salt-forming anion, such as chloride, bromide, iodide, fluoride, sulfate, methyl sulfate, methanebenzylsulfonate, phosphate, nitrite, nitrate, carboxylate, and mixtures thereof;

a is the ionic charge of X;

$R^{1a}$, $R^{2a}$, and $R^{3a}$ are independently hydrogen, $C_1$-$C_{36}$ alkyl, $C_1$-$C_{36}$ alkylhydroxy, $C_1$-$C_{36}$ alkyl amido $R_{(C1-C6)}$, $C_1$-$C_{36}$ alkylaryl amido $R_{(C1-C6)}$ or $C_1$-$C_{36}$ alkylhydroxy amido $R_{(C1-C6)}$, $R_{(C1-C6)}$ being $C_1$-$C_6$ alkylene or benzyl;

two of $R^{1a}$, $R^{2a}$, and $R^{3a}$ are independently $C_{10}$-$C_{36}$ alkyl, $C_{10}$-$C_{36}$ alkylhydroxy, $C_{10}$-$C_{36}$ alkyl amido $R_{(C1-C6)}$, $C_{10}$-$C_{36}$ alkylaryl amido $R_{(C1-C6)}$ or $C_{10}$-$C_{36}$ alkylhydroxy amido $R_{(C1-C6)}$;
the remaining one of $R^{1a}$, $R^{2a}$ and $R^{3a}$ is hydrogen, $C_1$-$C_8$ alkyl, $C_1$-$C_8$ alkylhydroxy, $C_1$-$C_8$ alkyl amido $R_{(C1-C6)}$, $C_1$-$C_8$ alkylaryl amido $R_{(C1-C6)}$ or $C_1$-$C_8$ alkylhydroxy amido $R_{(C1-C6)}$; $R^4$, $R^5$, $R^6$, and $R^7$, same or different, are independently hydrogen, alkyl, arylalkyl, alkylaryl, fluoro, bromo, chloro, iodo, acetoxy, alkylacetoxy, arylacetoxy, carboxy, alkylcarboxy, hydroxy or alkoxyhydroxy;
with the proviso that the dialkyl immidazoline quat does not have the formula

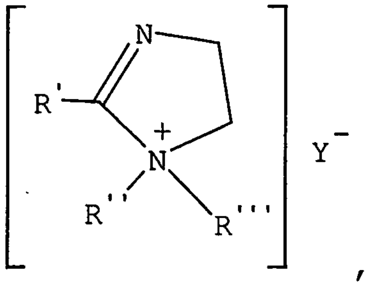

,

where R' is $C_{11}$-$C_{22}$ alkyl or $C_{13}$-$C_{24}$ β-alkyl hydroxy; R" is $C_1$-$C_6$ alkyl; and R"' is $C_{12}$-$C_{20}$ alkyl or $C_{11}$-$C_{22}$ alkyl amido $C_1$-$C_3$ alkylene.

In one embodiment of this aspect, the dialkyl immidazoline quat is a compound of the formula (III):

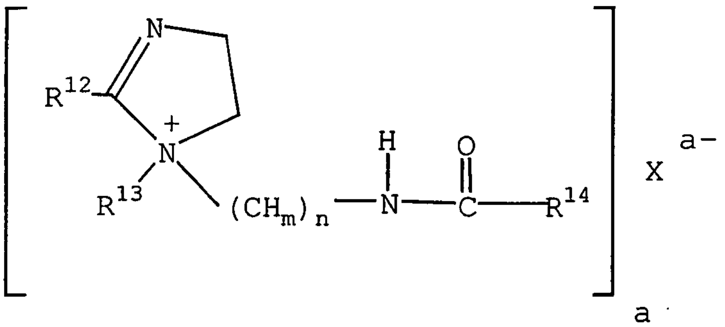

(III)

,

where

n varies from 1 to 3, preferably, n is 2;
m is 1 or 2, preferably, m is 2;
$R^{12}$ and $R^{14}$, same or different, are $C_{24}$-$C_{30}$ alkyl; and
$R^{13}$ is hydrogen or $C_1$-$C_3$ alkyl.

In yet another aspect, the invention provides compositions that include mixtures of at least two monoalkyl immidazoline quats with specified substitution content. In addition to monoalkyl immidazoline quats, the compositions may include other quaternary and non-quaternary compounds. If desired, dialkyl immidazoline quats and/or other quaternary compounds may be substantially excluded from the composition.

In one embodiment, the mixture contains monoalkyl immidazoline quats, at least one of which has a $C_{16}$-$C_{30}$ alkyl group; the $C_{16-30}$ substitution content of the mixture being from about 10% to about 95%, preferably, from about 15% to about 85%, more preferably, from about 20% to about 80%, yet more preferably, from about 25% to about 75%.

In another embodiment, the mixture contains monoalkyl immidazoline quats at least one of which has a $C_{18}$-$C_{26}$ alkyl group; the $C_{18-26}$ substitution content of the mixture being from about 10% to about 95%, preferably, from about 15% to about 85%, more preferably, from about 20% to about 80%, yet more preferably, from about 25% to about 75%.

In yet another embodiment, the mixture contains monoalkyl immidazoline quats at least one of which has a $C_{20}$-$C_{24}$ alkyl group; the $C_{20-24}$ substitution content of the mixture being from about 10% to about 90%, preferably, from

about 15% to about 85%, more preferably, from about 20% to about 80%, yet more preferably, from about 25% to about 75%.

Monoalkyl quats in the mixtures may have the formula (IV):

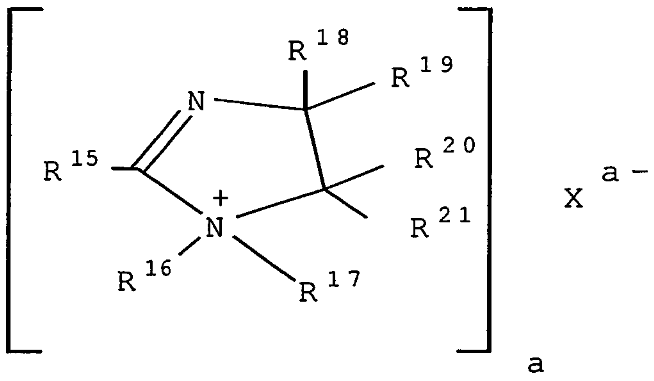

(IV)

,

where X and a are defined in reference to formula (I);

$R^{15}$, $R^{16}$ and $R^{17}$ are independently hydrogen, $C_1$-$C_{30}$ alkyl, $C_1$-$C_{30}$ alkylhydroxy, $C_1$-$C_{30}$ alkyl amido $R_{(C1-C6)}$, $C_1$-$C_{30}$ alkylaryl amido $R_{(C1-C6)}$, or $C_1$-$C_{30}$ alkylhydroxy amido $R_{(C1-C6)}$, wherein $R_{(C1-C6)}$ is a $C_1$-$C_6$ alkylene or benzyl; one of $R^{15}$, $R^{16}$ and $R^{17}$ is $C_1$-$C_{30}$ alkyl, $C_{10}$-$C_{30}$ alkylhydroxy, $C_{10}$-$C_{30}$ alkyl amido $R_{(C1-C6)}$, $C_{10}$-$C_{30}$ alkylaryl amido $R_{(C1-C6)}$ or $C_{10}$-$C_{30}$ alkylhydroxy, amido $R_{(C1-C6)}$;

the remaining two of $R^{15}$ $R^{16}$ and $R^{17}$ are independently hydrogen, $C_1$-$C_8$ alkyl, $C_1$-$C_8$ alkylhydroxy, $C_1$-$C_8$ alkyl amido $R_{(C1-C6)}$, $C_1$-$C_8$ alkylaryl amido $R_{(C1-C6)}$, or $C_1$-$C_8$ alkylhydroxy amido $R_{(C1-C6)}$ ;

$R^{18}$, $R^{19}$, $R^{20}$, and $R^{21}$, same or different, are independently hydrogen, alkyl, arylalkyl, alkylaryl, halogen, including bromo and chloro, acetoxy, alkylacetoxy, arylacetoxy, carboxy, alkylcarboxy, hydroxy, or alkoxyhydroxy; preferably, $R^{18}$, $R^{19}$, $R^{20}$, and $R^{21}$, same or different, are hydrogen or $C_1$-$C_8$ alkyl.

More preferably, $R^{15}$ is $C_{10}$-$C_{30}$ alkyl or alkylhydroxy, yet more preferably, $C_{14}$-$C_{30}$ alkyl or alkylhydroxy, yet more preferably, $R^{15}$ is $C_{16}$-$C_{30}$ alkyl or or alkylhydroxy, yet more preferably, $R^{15}$ is $C_{20}$-$C_{30}$ alkyl or alkylhydroxy;

$R^{16}$ is $C_1$-$C_6$ alkyl, yet more preferably, $C_1$-$C_3$ alkyl, yet more preferably, methyl;

$R^{17}$ is $C_1$-$C_8$ alkyl, $C_1$-$C_8$ alkylhydroxy, $C_1$-$C_8$ alkyl amido $R_{(C1-C6)}$, or $C_1$-$C_8$ alkylhydroxy amido $R_{(C1-C6)}$, more preferably, $C_1$-$C_8$ alkyl amido $C_1$-$C_3$ alkylene or $C_1$-$C_8$ alkylhydroxy.

Non-limiting examples of monoalkyl quats of the formula (IV) are quats (17), (18), and (19):

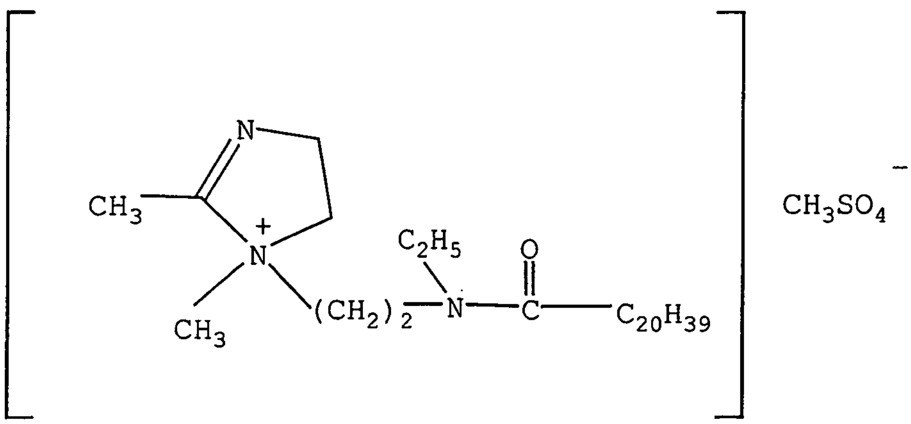

(17)

,

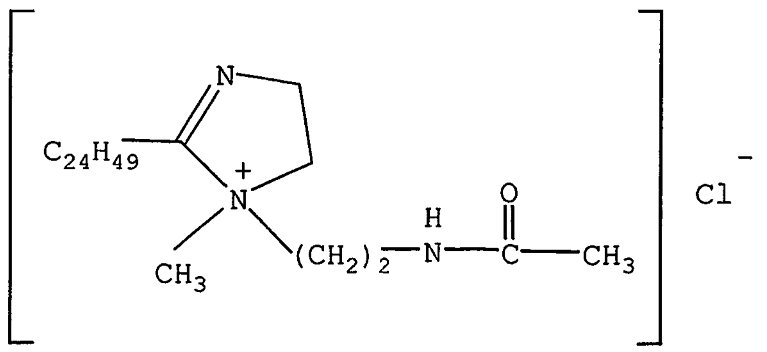

(18) ,

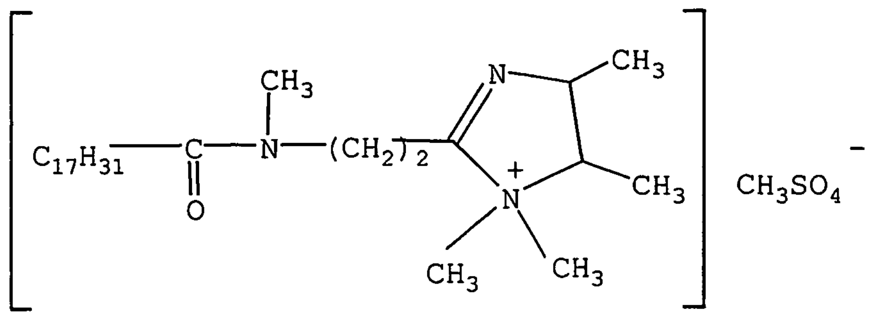

(19) .

More preferably, monoalkyl quats in the mixtures have the formula (V)

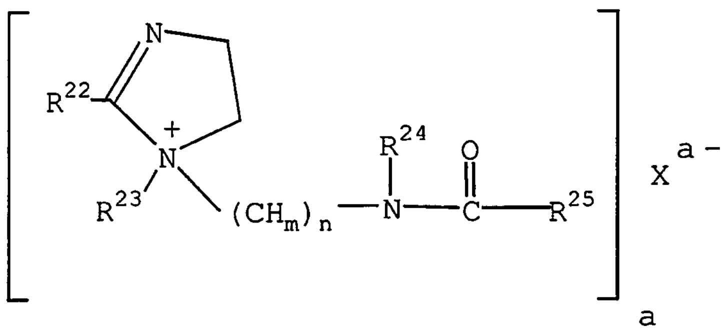

(V) ,

or formula (VI)

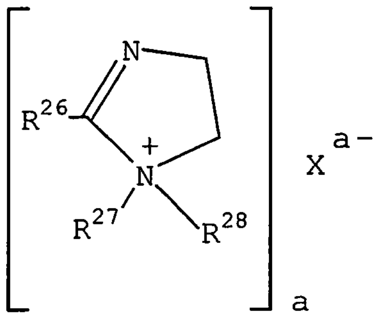

(VI) ,

where X and a are as defined above with reference to the formula (I);
$R^{22}$ and $R^{26}$ are independently $C_{16}$-$C_{30}$ alkyl;
$R^{23}$ and $R^{27}$ are each hydrogen or $C_1$-$C_3$ alkyl;
$R^{24}$ is hydrogen, alkyl, arylalkyl, alkylaryl, halogen, including bromo and chloro, acetoxy, alkylacetoxy, arylacetoxy, carboxy, alkylcarboxy, hydroxy, or alkoxyhydroxy;
$R^{25}$ and $R^{28}$ are each $C_1$-$C_8$ alkyl or $C_1$-$C_8$ alkylhydroxy;
n varies from 1 to 3; and
m is 1 or 2.

Yet more preferably, $R^{24}$ is hydrogen or lower alkyl, yet more preferably, hydrogen; n is 2; and m is 2.

Non-limiting examples of monoalkyl quats of the formula (V) are quats (20) and (21):

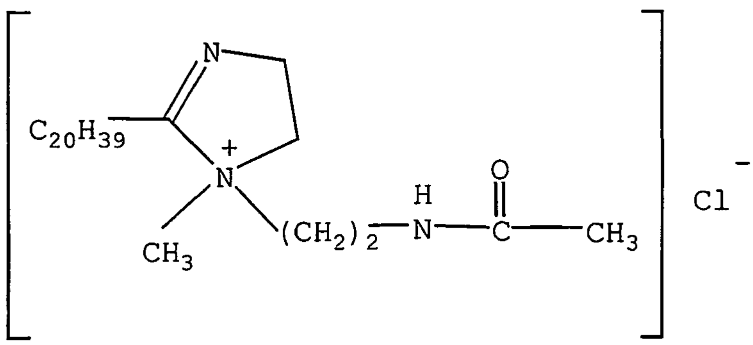

(20) ,

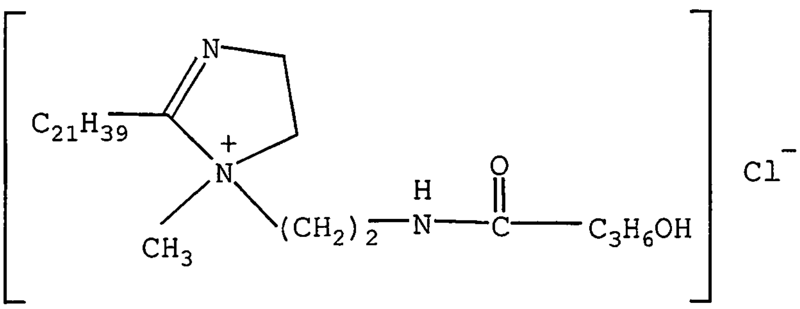

(21) .

Non-limiting examples of monoalkyl quats of the formula (VI) are quats (22) and (23):

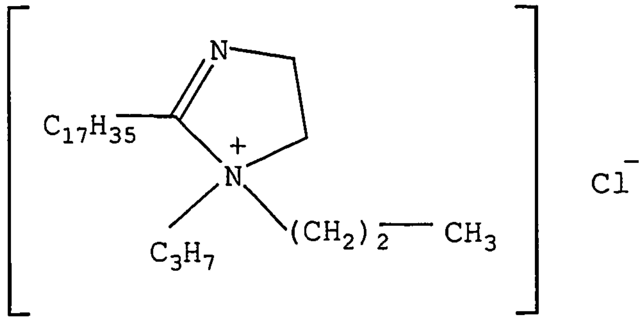

(22) ,

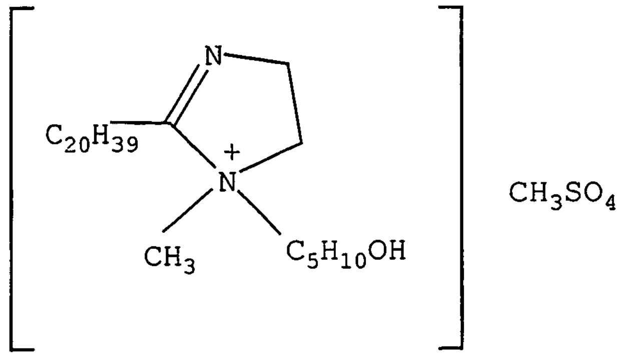

(23) .

Other non-limiting examples of monoalkyl quats of the formula (V) are quats (24)-(28) shown in Table 8:

TABLE 8*

| Quat | $R^{22}$ |
|---|---|
| 24 | $C_{18}H_{35}$ |
| 25 | $C_{21}H_{40}$ |
| 2 6 | $C_{22}H_{41}$ |
| 27 | $C_{18}H_{33}$ |
| 28 | $C_{22}H_{40}$ |
| *$R^{23}$ is $CH_3$; $R^{24}$ is hydrogen, $R^{25}$ is $CH_3$ | |

In non-limiting examples, Tables 9 and 10 show mixtures M8 and M9, respectively, of quats (24)-(28) and the calculations of their substitution content. Both mixtures are characterized in terms of their $C_{20-24}$ content (the specified range is $C_{20-24}$ and the reference range is $C_{10+}$).

TABLE 9*

| I (Quat) | II ($N_{20-24}$) | IV ($M_0$//$M_{10+}$) | V ($M_{20-20}$ = II x IV) | VI ($S_{20-24}$ = V/IV) |
|---|---|---|---|---|
| 24 | 0 | 1 | 0 (0 x 1) | |
| 25 | 1 | 1.5 | 1.5 (1 x 1.5) | |
| M8 | | 2.5 | 1.5 | 60% (1.5/2.5x 100%) |
| *$N_{10+}$ is 1; column (III) is omitted | | | | |

TABLE 10*

| I (Quat) | II ($N_{20-4}$ | IV ($M_0$//$M_{10+}$) | V ($M_{20-24}$ = II x IV) | VI ($S_{20-24}$ = V/IV) |
|---|---|---|---|---|
| 26 | 1 | 2 | 2 (1 x 2) | |
| 27 | 0 | 1 | 0 (0 x 1) | |
| 28 | 1 | 1 | 1 (1 x 1) | |
| M9 | | 4 | 3 | 75% (3/4) x 100% |

Monoalkyl immidazoline quats and dialkyl immidazoline quats may be prepared in a number of ways, including methods known to those skilled in the art. One of suitable preparation methods is described in U.S. Patent No. 4,855,440, incorporated herein by reference in its entirety. A possible synthetic route involves a reaction of a carboxylic acid, anhydride, or natural or synthetic oil, with a desired dialkyltriamine (for dialkyl quats preparation) or N-alkyl-N-alkylamino-diamine (for monoalkyl quats preparation), followed by quaternization of the resulting immidazoline intermediate.

Reaction Scheme 1 shows an example of the synthetic route for preparation of certain dialkyl immidazoline quats, specifically, 1-methyl-1-(alkyl-acylamido-) ethyl)-2-alkyl immidazolinium methyl sulfates, via a reaction between one mole of diethylene triamine and two moles of a fatty carboxylic acid (or acids), followed by a quaternization with dimethyl sulfate:

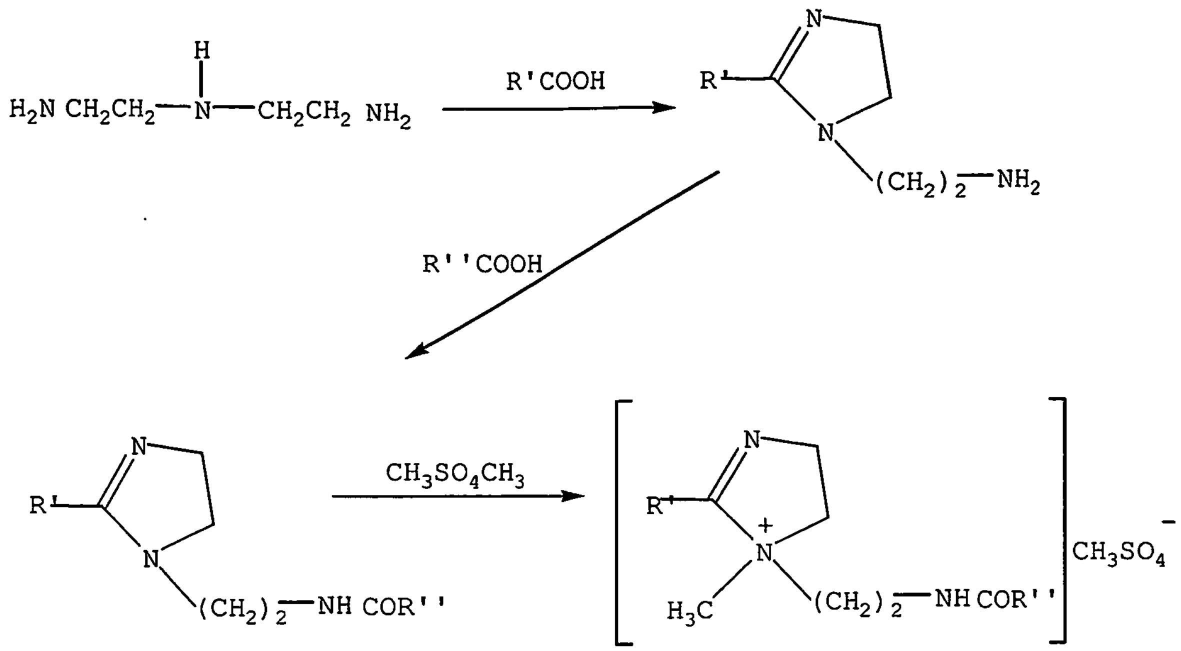


**Reaction Scheme 1** ,

where R' and R" are alkyl groups having more than 10 carbon atoms, or groups containing alkyl radicals having more than 10 carbon atoms.

Likewise, Reaction Scheme 2 shows an example of the synthetic route for preparation of certain monoalkyl immidazoline quats, specifically, 1-methyl-1-(lower alkyl)-2-alkyl immidazolinium chlorides, via a reaction between N-lower alkyl-N-ethyleneamino diamine and a fatty carboxylic acid(s), followed by a quaternization with methyl chloride:

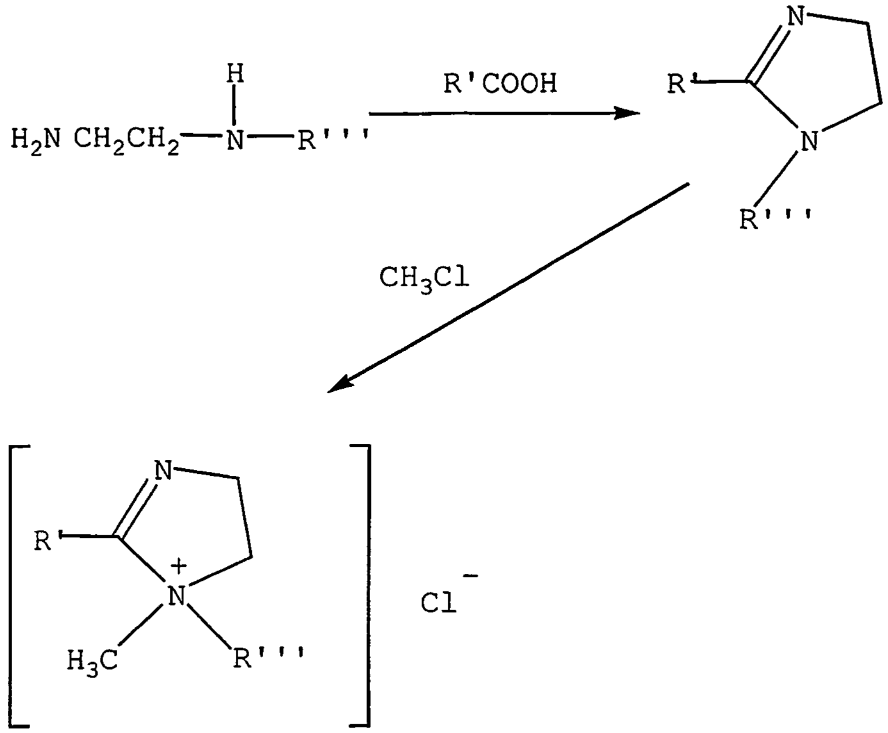


**Reaction Scheme 2** ,

where R' is an alkyl group or a group containing an alkyl radical having more than 10 carbon atoms, and R"' is an alkyl group or a group containing an alkyl radical having 1 to 8 carbon atoms.

The Reaction Schemes 1 and 2 are non-limiting examples. Various other immidazoline quats may be obtained via different synthetic routes known to those skilled in the art and/or by varying the starting materials and the reactants in the examplyfied routes. For example, with respect to the synthetic route shown in the Reaction Scheme 1, the carboxylic acids R'COOH and R"COOH may be the same or different and/or may contain a variety of groups R' and R". The nature of R' and R " substitution in the carboxylic acids may be used to vary the R' and R " substituents in the resulting dialkyl immidazoline compounds. Likewise, the groups R' and R"' may be varied in the route shown in the Reaction Scheme 2.

The carboxylic groups R'CO- and R"CO- may be derived from a variety of sources. Thus, essentially pure carboxylic acids may be used. The suitable carboxylic acids having $C_{18}$-$C_{24}$ alkyl groups include, for example, arachidic ($C^{20}$, including the carboxylic carbon[1], and 0 double bonds in the alkyl group ($C^{20}$: 0) erucic ($C^{22}$ : 1) , behemic ($C^{22}$:0), gadoleic ($C^{20}$:1), erucic ($C^{22}$:1), arachadonic ($C^{20}$:4), culpodonic ($C^{22}$:5), eicosapentaenoic ($C^{20}$:5), docosahexaenoic acid ($C^{22}$:6), tetrcosanoic ($C^{24}$:0); and nervonic ($C^{24}$:1). Other carboxylic acids, including acids having any desirable alkyl substitution may also be used.

[1] Subscripts with reference to the number of carbon atoms indicate the number of carbon atoms without the carboxylic group carbon; the superscripts indicate the number of carbon atoms of the carboxylic group including the carboxylic carbon. Thus, $C^{20}$ is $C_{19}$COO--.

The mixtures of quats may be obtained in any manner. For example, artificial mixtures of carboxylic acids may be used in reaction schemes 1 and 2. If a mixture of carboxylic acids is used, the reaction usually provides a corresponding mixture of compounds with R'/R " substitution content similar or identical to the R'/R" distribution in the mixture.

The use of artificial mixtures of pure carboxylic acids may not be economically feasible. Rather, the mixtures of carboxylic acids derived from a single source containing various carboxylic groups, such as natural or synthetic oils, triglycerides, and the like, are used. For example, such mixtures may be obtained in commercial quantities via saponification of ester-containing natural or synthetic substances.

In fact, carboxylic acids may be directly replaced in the reactions above by ester-containing natural or synthetic oil or a similar substance. Similarly to the use of carboxylic acid mixtures, the reaction between the ester-containing oil and a diamine or triamine usually provides a mixture of compounds with R'/R " substitution content similar or identical to the R'/R " distribution in the oil.

Preferably, the compositions of the invention contain compounds derived from natural and synthetic oils, fatty acids and/or triglycerides.

Thus, in yet another aspect, the invention provides a product of a reaction between

a) a compound of the formula

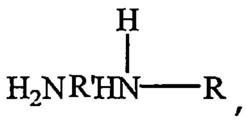

where R' is $C_1$-$C_3$ alkylene, preferably -$CH_2CH_2$-group, and R is R'$NH_2$, $C_1$-$C_{30}$ alkyl, or $C_1$-$C_{30}$ alkylhydroxy, preferably. R is -$CH_2CH_2NH_2$ or -$CH_2CH_2OH$; and

b) a mixture of natural or synthetic oil-derived carboxylic acids or a natural or synthetic oil.

Oils that may be used directly or which may provide oil-derived mixtures of carboxylic acids include, for example, HEAR oil, cod liver oil, herring oil, menhaden oil, mustard seed oil, pilchard oil, hear oil, salmon oil, sardine oil and shark liver oil. Of course, other oils and similar substances may also be used. For listing of such substances, see 1 "Bailey's Industrial Oil and Fat Products" (Daniel Swern, John Wiley & Sons, 4th Ed. 1979), at pages 416-417, 447, 449-450, and 452, which are hereby incorporated by reference.

The substitution content of any quat mixture, including those derived from the natural or synthetic oils, may be relatively easily characterized in terms of the weight percentages of certain substituents in the mixture. Any analytical methods known to those skilled in the art may be used, such as High Performance Liquid Chromatography or Gas Chromatography, where suitable, to determine the identity and the weight proportions of the quat components in the mixture. On the basis of such analysis, the "substitution content" of the mixture as defined herein (*e.g.*, $C_{16\text{-}24}$ or $C_{24\text{-}24}$ contents), may be easily derived from the weight percentages and molecular weights of the quat components by using the calculation methodology described herein.

Table 11 shows known approximate weight percentages of some of the $C^{20}$+ components in some of the common oils:

TABLE 11

| Substance | $C^{20}$:0 | $C^{20}$:1 | $C^{20}$:4 | $C^{20}$:5 | $C^{20}$:0 | $C^{22}$:1 | $C^{22}$:5 | $C^{22}$:6 | $C^{24}$:0 |
|---|---|---|---|---|---|---|---|---|---|
| Cod liver oil | 8.8-14.6 % | | | 2.6-9% | | 4.6-13.3% | 1-2% | 8.6-19% | |
| Herring oil | | 1.5-19.2% | | 4.6-10.2% | | 2.8-19.9% | 1-3.7% | 3.8-24.1 % | |
| Menhaden oil | | 0.9-2.7% | 0.6-1.2% | 10.2-13.5% | | 0.7-1.7% | 1.1-2.3% | 3.3-14% | |
| Pilchard (Sardine) oil | | 3.2% | 1.6% | 16.9% | | 3.6% | 2.5% | 12.9 % | |
| HEAR oil | | 0.8-13.5% | | | | 20.1-59.4% | | | 0.1-1.4% |
| Mustard Seed oil | | 7% | | | | 44.2% | | | |

The oils shown in Table 11 generally contain from about 30% to about 90% of $C_{20}$-$C_{30}$ alkyl groups in their fatty carboxylic groups by weight. The oils often exhibit substantial variations in $C_{20}$-$C_{30}$ content, and also include some $C_1$-$C_{19}$ content.

Thus, the more preferred mixtures of dialkyl quats are derived from rapeseed oil, especially high erucic rapeseed oil (HEAR oil), which typically contains 46% of $C^{22}$:1 alkyl (erucic), 1.5% of $C^{22}$:0 alkyl (behemic), and 11% of $C^{20}$:1 alkyl (gadoleic) by weight. Yet more preferred are qtars derived from hydrogenated HEAR oil in which the double bonds of the erucic is hydrogenated, resulting in behenic ($C^{22}$:0). HEAR oil or HEAR oil-derived mixtures of carboxylic acids may be used to obtain quat mixtures. The dialkyl immidazoline quat mixture obtained in this manner is preferred. It is also referred to in the examples herein as di-behenyl immidazolinium methosulfate, reflecting the prevalence of $C^{22}$:1 alkyls.

The compositions containing immidazoline quats may in the form of quat raw materials.

In general, a producer provides raw quats to manufacturers of personal care and cosmetic products, who formulate them in the final products. An important characteristic of raw quats, as well as the final products that incorporate them, is the so-called cationic activity, which measures a concentration of positive charges in a substance, product, etc. The cationic activity may be measured by several methods readily understood by those skilled in the art. One such method utilizes a standardized solution of an anionic material, such as sodium lauryl sulfate. This material is added to the solution containing the quat until full complexation of the quat's cations (the end point) has been reached. The end point can be measured potentiometrically or by the use of color indicators.

Typical tests involve titrating a sample of the quat, usually dissolved in a solvent, with the standardized solution of sodium lauryl sulfate until the endpoint is reached. As described in the co-pending and co-assigned U.S. Patent Application No. 09/438,631, incorporated by reference herein in its entirety, once the endpoint is reached, the cationic activity can be calculated according to the following formula:

$$\%\ \text{cationic activity} = \frac{\text{mL} \times \text{N} \times \text{MW} \times 100}{\text{S.wt.} \times 1000}$$

Where: mL = the number of mL of anionic material
N = the normality of the solution used
MW = the molecular weight of the quat being analyzed
S.wt.= the sample weight in grams

For additional information regarding the methodology for measuring the cationic activity, see W. Schempp and H. T. Trau, Wochenblatt fur Papierfabrikation 19, 1981, pages 726-732, or J. P. Fischer and K. Lohr, Organic Coatings Science Technology, Volume 8, pages 227-249, Marcel Dekker, Inc. April 1986), both incorporated herein by reference in their entirety.

It is desirable to provide raw quats in a concentrated form with high cationic activity, as a solid or semi-solid solution or dispersion. Without wishing to be bound by any specific theory, it is believed that a desired amount of a given quat or mixture of quats to be placed in a formulation may be measured by the cationic activity of the quat raw material. The quat raw materials with high cationic activity permit better transportation efficiency since they occupy smaller space while providing the same desired quat amounts. It is also desirable to produce raw quats that, in addition to having high cationic activity, provide for ease in commercial handling and storage. For example, the raw quat that melt at lower temperatures minimize quat decomposition and improve energy efficiency. For this purpose, it is preferred for the raw quats to be flakeable or pastillatable.

Thus, the composition containing immidazoline quats or quat mixtures described herein may be in the form of concentrated, usually solid, solutions or suspensions of the quat(s) or mixture(s) in a suitable carrier. Such compositions are called herein quats raw materials. The preferred carrier is a solvent, and the preferred solvents include isopropyl alcohol, SDA-40, propylene glycol, butylenes glycol, various fatty alcohols, and mixtures thereof. Preferably, the quat raw materials of the invention are flakeable or pastillatable solids with high quat cationic activity. The quat cationic activity is the cationic activity that is attributed to quaternary nitrogen compounds. The preferred total quat cationic activity of the quat raw materials of the invention is greater than 10%, preferably, greater than 20%, more preferably, greater than 35%, yet more preferably, greater than 50%.

The quat raw materials of the invention may include one or more desirable ingredients of final cosmetic/personal care formulations, such as emollients and the like, as well as various impurities. The list of possible ingredients may be found below.

The compositions containing immidazoline quat mixtures may also be in the form of various cosmetic and/or personal care products. Such compositions may be referred to as final product compositions. Examples of the final

product compositions include sunscreen compositions for hair and/or skin, such as lotions, gels, sprays, and the like, hand cleaners, bath compositions, suntan oils, anti-perspirant compositions, perfumes and colognes, cold creams, pre-shaves, deodorants, topical pharmaceutical ointments, skin moisturizers, facial cleansers, cleansing creams, skin gels, shampoos, hair conditioners, detergents, household cleaning products, make-up products, lipstick products, mascara, and hair coloring products. The preferred final product compositions of the invention are compositions for treating human hair, such as shampoos or conditioners.

The final product compositions including preparations for skin and hair, include immidazoline quats or immidazoline quat mixtures described herein. The amount of immidazoline quats or mixtures in the products depend on the specific application, and may vary from about 0.1% to about 40%, more preferably, from about 0.1% to about 10%, yet more preferably, from about 0.5% to about 2% by the weight of the product composition. However, different amounts of immidazoline quats or immidazoline quat mixtures may be preferred depending on the nature of the product.

The final product compositions that include the mixtures of immidazoline quats may be in the form of liquids, gels, creams, emulsions, foams, and solids; may be clear or opaque; and may be formulated as aqueous and non-aqueous preparations, including but not limited to topical preparations. Preferably, the final product compositions are dispersions or solutions in water, or in a mixture of water with a suitable secondary solvent. Suitable inert solvents include various lower alkanols and glycols. Lower alkanols having from one to four carbon atoms are suitable for use with the present invention, and lower alkanols having from two to three carbon atoms are preferred. Glycols having from three to eight carbon atoms are suitable for use with the present invention, while glycols having from three to six carbon atoms are preferred. Examples of suitable lower alkanols and glycols include methanol, ethanol, isopropanol, butanol, hexylene glycol, 1,3-butylene glycol, 1,2- and 1,3-propane diol, 2-methyl 1,3-propane diol, propylene glycol, diethylene glycol, and the like. The total amount of solvent may be up to about 98% by weight of the composition, preferably, from about 20% to about 90%, more preferably, from about 50% to about 90% by weight of the composition. Again, however, different amounts of solvent may be preferred depending on the nature of the product. If a mixture of water and a secondary solvent is used, the secondary solvent may be present in the amount of up to 90%, preferably, between about 25% and about 80% by weight of water in the composition.

In addition to the immidazoline quaternary compounds, the final product compositions may include various active and additional ingredients, both conventional and otherwise. Of course, a decision to include an ingredient and the choice of specific active and additional ingredients depends on the specific application and product formulation. Also, the line of demarcation between an "active" ingredient and an "additional ingredient" is artificial and dependent on the specific application and product type. A substance that is an "active" ingredient in one application or product may be an "additional" ingredient in another, and vice versa.

The final product compositions may include one or more active ingredients, which provide some benefit to the object of the application of the composition, for example, hair or skin. Such active ingredients may include one or more substances such as cleaning agents, hair conditioning agents, skin conditioning agents, hair styling agents, antidandruff agents, hair growth promoters, perfumes, sunscreen compounds, pigments, moisturizers, film formers, hair colors, make-up agents, detergents, thickening agents, emulsifiers, antiseptic agents, deodorant actives, surfactants and pharmaceuticals useful for topical purposes for transdermal delivery.

The choice of the active ingredient(s) depends on the nature of the desired cosmetic or personal care product. For example, the sunscreen compounds may be used in the sunscreen lotions, shampoos, medicated shampoos, hair care lotions and the like. For each type of active ingredient, one or more compounds may be present. Likewise, more than one type of active ingredient may be present.

It is believed that immidazoline quat and/or quat mixtures improves hair substantivity of hydrophobic ingredients of cosmetic and personal care products, which is typically thought of as the degree of deposition of the hydrophobic ingredient on hair and is desirable. The hydrophobic components are those that are substantially insoluble in water. Typically, such hydrophobic ingredients are soluble in oils. Thus, the compositions described herein may further include at least one hydrophobic ingredient, examples of which include botanical extracts, vitamin E, vitamin A, silicones, waxes and antioxidants.

SURFACTANTS

In addition to the immidazoline quaternary compounds and/or mixtures, other surfactants may be present in the compositions described herein, including one or more nonionic surfactants, anionic surfactants, cationic surfactants, amphoteric surfactants, zwitterionic surfactants, and mixtures thereof. For some of surfactants that may be used in combination with the compositions of the invention, please see McCutcheon's, Detergents and Emulsifiers, (1986), U.S. Pat. Nos. 5, 151, 210, 5, 151, 209, 5, 120, 532, 5,011,681, 4,788,006, 4,741,855, U.S. Pat. Nos. 4,704,272, 4,557,853, 4,421,769, 3,755,560; all incorporated herein by reference in their entirety.

Cationic surfactants

Immidazoline quaternary compounds of the invention are cationic surfactants suitable for use in various personal care products, especially hair care products such as conditioners and shampoos. In addition, other cationic surfactants may be present in the compositions of the invention. The amounts and the nature of cationic surfactants present in the compositions of the invention depend on the nature of the composition. In the final product composition, the total amount of cationic surfactants, including the immidazoline quats and mixtures thereof described herein, may vary from 0.1% to about 40%, more preferably, from about 0.1% to about 15%, yet more preferably, from about 0.5% to about 2% by the weight of the product composition. However, different amounts of cationic surfactants may be preferred depending on the nature of the product. Suitable additional cationic surfactants are disclosed in McCutcheon, Detergents & Emulsifiers, (M.C. Publishing Co. 1979); U.S. Pat. Nos. 3,155,591, 3,929,678, 3,959,461, 4,387,090, which are incorporated by reference herein.

Ammonium quats

The compositions of the invention may include quaternary ammonium cationic surfactants of the formula

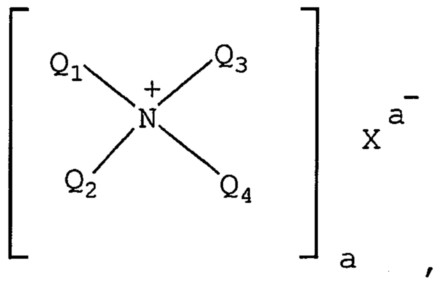

where X and a are as previously described, $Q_1$ is $C_{12}$-$C_{22}$ alkyl, $C_{12}$-$C_{22}$ alkyl amido $C_1$-$C_6$ alkylene, $C_{12}$-$C_{22}$ alkylhydroxy; $Q_2$ is $C_{12}$-$C_{22}$ alkyl, $C_{12}$-$C_{22}$ alkyl amido $C_1$-$C_6$ alkylene, $C_{12}$-$C_{22}$ alkylhydroxy, benzyl, or $C_1$-$C_6$ alkyl; $Q_3$ and $Q_4$ are independently $C_1$-$C_6$ alkyl or benzyl.

Examples of suitable quaternary ammonium surfactants include cetyl ammonium chloride, cetyl ammonium bromide, lauryl ammonium chloride, lauryl ammonium bromide, stearyl ammonium chloride, stearyl ammonium bromide, cetyl dimethyl ammonium chloride, cetyl dimethyl ammonium bromide, lauryl dimethyl ammonium chloride, lauryl dimethyl ammonium bromide, stearyl dimethyl ammonium chloride, stearyl dimethyl ammonium bromide, cetyl trimethyl ammonium chloride, cetyl trimethyl ammonium bromide, lauryl trimethyl ammonium chloride, lauryl trimethyl ammonium bromide, stearyl trimethyl ammonium chloride, stearyl trimethyl ammonium bromide, lauryl dimethyl ammonium chloride, stearyl dimethyl cetyl ditallow dimethyl ammonium chloride, dicetyl ammonium chloride, dicetyl ammonium bromide, dilauryl ammonium chloride, dilauryl ammonium bromide, distearyl ammonium chloride, distearyl ammonium bromide, dicetyl methyl ammonium chloride, dicetyl methyl ammonium bromide, dilauryl methyl ammonium chloride, dilauryl methyl ammonium bromide, distearyl methyl ammonium chloride, distearyl dimethyl ammonium chloride, distearyl methyl ammonium bromide, and mixtures thereof.

Additional quaternary ammonium salts include those wherein the $C_{12}$-$C_{22}$ alkyl is derived from a tallow fatty acid or from a coconut fatty acid. Examples of quaternary ammonium salts derived from these tallow and cococut sources include ditallow dimethyl ammonium chlroide, ditallow dimehtyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, di(coconutalkyl)dimethyl ammonium chloride, di(coconutalkyl)dimethyl ammonium bromide, tallow ammonium chloride, coconut ammonium chloride, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

More preferred quaternary ammonium surfactants are dilauryl dimethyl ammonium chloride, distearyl dimethyl ammonium chloride, dimyristyl dimethyl ammonium chloride, dipalmityl dimethyl ammonium chloride, distearyl dimethyl ammonium chloride, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, and mixtures thereof.

Fatty amines

The compositions of the invention may also include salts of primary, secondary and tertiary $C_{12}$-$C_{22}$ amines. Examples of such suitable amines include stearamido propyl dimethyl amine, diethyl amino ethyl stearamide, dimethyl stearamine, dimethyl soyamine, soyamine, tri(decyl)amine, ethyl stearylamine, ethoxylated stearylamine, dihydroxyethyl stearylamine, and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate, N-tallowpropane diamine dichloride and stearamidopropyl dimethylamine citrate. Some cationic amine surfactants useful in the compositions of the present invention are disclosed in U.S. Pat. No. 4,275,055, incorporated by reference herein.

Amidoamines

The compositions of the invention may also include aminoamides, such as disclosed in U.S. Patent Application No. 09/409,203, assigned to Croda Inc., and incorporated by reference herein.

Non-ionic surfactants

The compositions of the invention may also include various non-ionic surfactants. Among the suitable nonionic surfactants are condensation products of $C_8$-$C_{30}$ alcohols with sugar or starch polymers. These compounds can be represented by the formula $(S)_n$ --O-R, wherein S is a sugar moiety such as glucose, fructose, mannose, and galactose; n is an integer of from about 1 to about 1000, and R is $C_8$-$C_{30}$ alkyl. Examples of suitable $C_8$-$C_{30}$ alcohols from which the R group may be derived include decyl alcohol, cetyl alcohol, stearyl alcohol, lauryl alcohol, myristyl alcohol, oleyl alcohol, and the like. Specific examples of these surfactants include decyl polyglucoside and lauryl polyglucoside.

Other suitable nonionic surfactants include the condensation products of alkylene oxides with fatty acids (i.e., alkylene oxide esters of fatty acids). These materials have the general formula RCO (X) OH, wherein R is a $C_{10}$-$C_{30}$ alkyl, X is $-OCH_2CH_2-$ (derived from ethylene oxide) or- $OCH_2CHCH_3-$ (derived from propylene oxide), and n is an integer from about 1 to about 200.

Yet other suitable nonionic surfactants are the condensation products of alkylene oxides with fatty acids (i.e., alkylene oxide diesters of fatty acids) having the formula $RCO(X)_nOOCR$, wherein R is a $C_{10}$-$C_{30}$ alkyl, X is $-OCH_2CH_2-$(derived from ethylene oxide) or $-OCH_2CHCH_3-$ (derived from propylene oxide), and n is an integer from about 1 to about 200.

Yet other nonionic surfactants are the condensation products of alkylene oxides with fatty alcohols (i.e., alkylene oxide ethers of fatty alcohols) having the general formula $R(X)_nOR'$, wherein R is $C_{10}$-$C_{30}$ alkyl, , n is an integer from about 1 to about 200, and R' is H or a $C_{10}$-$C_{30}$ alkyl.

Still other nonionic surfactants are the compounds having the formula $RCO(X)_nOR'$ wherein R and R' are $C_{10}$-$C_{30}$ alkyl, X is - $OCH_2CH_2-$ (derived from ethylene oxide) or $-OCH_2CHCH_3-$ (derived from propylene oxide), and n is an integer from about 1 to about 200.

Examples of alkylene oxide-derived nonionic surfactants include ceteth-1, ceteth-2, ceteth-6, ceteth-10, ceteth-12, ceteraeth-2, ceteareth6, ceteareth-10, ceteareth-12, steareth-1, steareth-2, stearteth-6, steareth-10, steareth-12, PEG-2 stearate, PEG4 stearate, PEG6 stearate, PEG-10 stearate, PEG-12 stearate, PEG-20 glyceryl stearate, PEG-80 glyceryl tallowate, PPG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amides disclosed, for example, in U.S. Patent Nos. 2,965,576, 2,703,798, and 1,985,424, which are incorporated herein by reference.

Anionic surfactants

The compositions of the invention may also include various anionic surfactants. Several examples of suitable anionic surfactants are disclosed in U.S. Patent No. 3,929,678, which is incorporated herein by reference. Further examples of suitable anionic surfactants include alkoyl isethionates, and alkyl ether sulfates.

The alkoyl isethionates typically have the formula $RCO-OCH_2CH_2-SO_3M$, wherein R is $C_{10}$-$C_{30}$ alkyl, and M is a water-soluble cation, such as ammonium, sodium, potassium, or triethanolamine. The examples of suitable isethionates include ammonium cocoyl isethionate, sodium cocoyl isethionate, sodium lauroyl isethionate, sodium stearoyl isethionate, and mixtures thereof. Preferred for used herein are ammonium cocoyl isethionate, sodium cocoyl isethionate, and mixtures thereof.

The alkyl ether sulfates typically have the formulas $ROSO_3M$ and $RO(C_2H_4O)_xSO_3M$, where R is $C_{10}$-$C_{30}$ alkyl, x varies from about 1 to about 10, and M is a water-soluble cation such as ammonium, sodium, potassium and triethanolamine.

Yet another suitable class of anionic surfactants are alkali metal salts of $C_8$-$C_{30}$ carboxylic acids and alkylsulfonates of the formula $R_1$-$SO_3M$ (where $R_1$ is $C_8$-$C_{30}$ alkyl; preferably, $C_{12}$-$C_{22}$ alkyl, and M is a cation), including succinamates, and $C_{12}$-$C_{24}$ olefin sulfonates and carboxylates.

Amphoteric surfactants

The compositions of the invention may also include zwitterionic and amphoteric surfactants. Suitable amphoteric and zwitterionic surfactants are, for example, derivatives of mono- or di-$C_8$-$C_{24}$ secondary and tertiary amines, such as alkyl imino acetates, carboxylates, sulfonates, sulfates, phosphates, and phosphonates, including iminodialkanoates and aminoalkanoates of the formulas $RN(CH_2)_m$ $CO_2$ $M_2$ and $RNH(CH_2)_m$ $CO_2M$, where m varies from 1 to 4, R is $C_8$-$C_{30}$ alkyl; preferably, $C_{12}$-$C_{22}$ alkyl, and M is H, alkali metal, alkaline earth metal ammonium, or alkanolammonium.

Other suitable amphoteric and zwitterionic surfactants are imidazolinium and ammonium derivates. Suitable examples of such amphoteric surfactants include sodium 3-dodecylaminopropionate, sodium 3-dodecylaminopropane sulfonate, N-alkyltaurines; N-higher alkyl aspartic acids, and coamidopropyl PG-dimonium chloride phosphate. For further examples of suitable amphoteric and zwitterionic surfactants, please see U.S. Patent Nos. 2,658,072, 2, 438, 091, and 2,528,378, which are incorporated herein by reference

Yet other suitable amphoteric and zwitterionic surfactants are betaines. Examples of suitable betaines include coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine, lauryl bis-(2-hydroxyethyl) carboxymethyl betaine, stearyl bis-(2-hydroxypropyl) carboxymethyl betaine, oleyl dimethyl gammacarboxypropyl betaine, lauryl bis-(2-hydroxypropyl) alphacarboxyethyl betaine, coco dimethyl sulfopropyl betaine, stearyl dimethyl sulfopropyl betaine, lauryl dimethyl sulfoethyl betaine, lauryl bis-(2-hydroxyethyl) sulfopropyl betaine, and amidobetaines and amidosulfobetaines, oleyl betaine, and cocamidopropyl betaine.

SUNSCREEN COMPOUNDS

A wide variety of sunscreen compounds are suitable for use with the compositions of the present invention. Depending on the nature of the composition, the sunscreen compounds may be added in the amount of up to about 40% by weight of the composition, preferably, from about 1% to about 30%. However, the preferred amount may vary depending on the nature of the composition. Thus, for the final product compositions in the form of a shampoo or conditioner, the suitable sunscreen agent may be included in the amount of up to about 40% by weight of the composition, preferably, from about 0.5% to about 5%, more preferably, from about 05 to about 1.5% by weight of the composition. Suitable sunscreen compounds include, for example, p-aminobenzoic acid, its salts and its derivatives; anthranilates; salicylates; cinnamic acid derivatives; dihydroxycinnamic acid derivatives; trihydroxycinnamic acid derivatives; hydrocarbons; dibenzalacetone and benzalacetophenone; naphtholsulfonates; dihydroxy-naphtholic acid and its salts; coumarin derivatives; diazoles; quinine salts; quinoline derivatives; hydroxy- or methoxy-substituted benzophenones; uric and vilouric acids; tannic acid and its derivatives; hydroquinone; amino benzoates, salicylates, ferrulic acid derivatives, phenylbenzimidazole sulfonic acids, benzophenone sulfonic acids, thioctic acids derivatives, oil-soluble cinnamates, and benzophenones. For other suitable sunscreen compounds, please see Segarin, et al., Cosmetics Science and Technology, Chapter VIII, pages 189 et seq., incorporated herein by reference.

Specific suitable sunscreen compounds include 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl-4->bis(hydroxypropyl)!-aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethyl-aminobenzoic acid or aminobenzoate, 2-ethylhexyl p-dimethylaminobenzoate, 2-phenylbenzimidazole-5-sulfonic acid, 2-(p-dimethylaminophenyl)-5-sulfonicbenzoxazoic acid, para-aminobenzoic acid, benzophenone-1, benzophenone-1, benzophenone-2, benzophenone-3, benzophenone-4, benzophenone-5, benzophenone-6, benzophenone-8, benzophenone-12, methoxycinnamate, avobenzone, ethyl dihydroxypropyl para-aminobenzoate, glyceryl para-aminobenzoate, methyl anthranilate, octocrylene, octyl dimethyl para-aminobenzoate, octyl methoxycinnamate, octyl salicylate, zinc oxide, titanium dioxide, and red petrolatum.

EMOLLIENTS

The compositions of the invention may also include one or emollient compounds such as fats, waxes, lipids, silicones, hydrocarbons, fatty alcohols and a wide variety of solvent materials. The amount of the emollient depends on the application. For the final product compositions, emmollinets are included in the amount of up to 50% by weight of the composition, preferably, from about 0.1% to about 20%, and more preferably, from about 0.5% to about 10% by weight of the composition.

Examples of suitable emollients include $C_8$-$_{30}$ alkyl esters of $C_8$-$_{30}$ carboxylic acids; $C_{1-6}$ diol monoesters and diesters of $C_{8-30}$ carboxylic acids; monoglycerides, diglycerides, and triglycerides of $C_{8-30}$ carboxylic acids, cholesterol esters of $C_{8-30}$ carboxylic acids, cholesterol, and hydrocarbons. Examples of these materials include diisopropyl adipate, isopropyl myristate, isopropyl palmitate, ethylhexyl palmitate, isodecyl neopentanoate, $C_{12-15}$ alcohols benzoates, diethylhexyl maleate, PPG-14 butyl ether, PPG-2 myristyl ether propionate, cetyl ricinoleate, cholesterol stearate, cholesterol isosterate, cholesterol acetate, jojoba oil, cocoa butter, shea butter, lanolin, lanolin esters, mineral oil, petrolatum, and straight and branched $C_{16}$-$C_{30}$ hydrocarbons.

Also useful are straight and branched chain fatty $C_8$-$C_{30}$ alcohols, for example, stearyl alcohol, isostearyl alcohol, ehenyl alcohol, cetyl alcohol, isocetyl alcohol, and mixtures thereof. Examples of other suitable emollients are disclosed in U.S. Patent No. 4,919,934; which is incorporated herein by reference in its entirety.

Other suitable emollients are various alkoxylated ethers, diethers, esters, diesters, and trimesters. Examples of suitable alkoxylated ethers include PPG-10 butyl ether, PPG-11 butyl ether, PPG-12 butyl ether, PPG-13 butyl ether, PPG-14 butyl ether, PPG-15 butyl ether, PPG-16 butyl ether, PPG-17 butyl ether, PPG-18 butyl ether, PPG-19 butyl ether, PPG-20 butyl ether, PPG-22 butyl ether, PPG-24 butyl ether, PPG-30 butyl ether, PPG-11 stearyl ether, PPG-15 stearyl ether, PPG-10 oleyl ether, PPG-7 lauryl ether, PPG-30 isocetyl ether, PPG-10 glyceryl ether, PPG-15 glyceryl ether, PPG-10 butyleneglycol ether, PPG-15 butylene glycol ether, PPG-27 glyceryl ether, PPG-30 cetyl ether, PPG-28 cetyl ether, PPG-10 cetyl ether, PPG-10 hexylene glycol ether, PPG-15 hexylene glycol ether, PPG-10 1,2,6-hexanetriol ether, PPG-15 1,2,6-hexanetriol ether, and mixtures thereof.

Examples of alkoxylated diethers include PPG-10 1,4-butanediol diether, PPG-12 1,4-butanediol diether, PPG-14 1,4-butanediol diether, PPG-2 butanediol diether, PPG-10 1,6-hexanediol diether, PPG-12 1,6-hexanediol diether, PPG-14 hexanediol diether, PPG-20 hexanediol diether, and mixtures thereof. Preferred are those selected from the group consisting of PPG-10 1,4-butanediol diether, PPG-12 1,4-butanediol diether, PPG-10 1,6-hexandiol diether, and PPG-12 hexanediol diether, and mixtures thereof.

Examples of suitable alkoxylated diesters and trimesters are disclosed in U.S. Patent Nos. 5,382,377, 5,455,025 and 5,597,555, assigned to Croda Inc., and incorporated herein by reference.

Suitable lipids include $C_8$-$C_{20}$ alcohol monosorbitan esters, $C_8$-$C_{20}$ alcohol sorbitan diesters, $C_8$-$C_{20}$ alcohol sorbitan triesters, $C_8$-$C_{20}$ alcohol sucrose monoesters, $C_8$-$C_{20}$ alcohol sucrose diesters, $C_8$-$C_{20}$ alcohol sucrose triesters, and $C_8$-$C_{20}$ fatty alcohol esters of $C_2$ -$C_{62}$ -hydroxy acids. Examples of specific suitable lipids are sorbitan diisostearate, sorbitan dioleate, sorbitan distearate, sorbitan isosotearate, sorbitan laurate, sorbitan oleate, sorbitan palmitate, sorbitan sesquioleate, sorbitan esquistearte, sorbitan stearate, sorbitan triiostearte, sorbitan trioleate, orbitan tristeate, sucrose cocoate, sucrodilaurate, sucrose distearate, sucrose laurate, sucrose myristate, sucrose oleate, sucrose palmitate, sucrose ricinoleate, sucrose stearate, sucrose tribehenate, sucrose tristearate, myristyl lactate, stearyl lactate, isostearyl lactate, cetyl lactate, palmityl lactate, cocoyl lactate, and mixtures thereof.

Other suitable emollients include mineral oil, petrolatum, cholesterol, dimethicone, dimethiconol, stearyl alcohol, cetyl alcohol, behenyl alcohol, diisopropyl adipate, isopropyl myristate, myristyl myristate, cetyl ricinoleate, sorbitan distearte, sorbitan dilaurate, sorbitan stearate, sorbitan laurate, sucrose laurate, sucrose dilaurate, sodium isostearyl lactylate, lauryl pidolate, sorbitan stearate, stearyl acohol, cetyl alcohol, behenyl alcohol, PPG-14 butyl esther, PPG-15 stearyl ether, and mixtures thereof.

EMULSIFIERS

The compositions of the invention may also include various emulsifiers. In the final product compositions of the invention, emulsifiers may be included in the amount of up to about 10%, preferably, in the amount of from about 0.5% to about 5% by weight of the composition. The examples of suitable emulsifiers include stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, polyethyleneglycols, polypropyleneglyocis, and mixtures thereof.

ANTI-DANDRUFF

The compositions of the invention may also include antidandruff agents. The examples of suitable antidandruff agents include zinc pyrithione, sulphur, and selenium sulfide.

HAIR OXIDIZERS

The compositions of the invention may also include hair oxidizing/reducing agents. The examples of suitable hair oxidizing/reducing agents include hydrogen peroxide, perborate, thioglycolates and persulfate salts.

THICKENERS

The compositions of the invention may also include various thickeners, such as cross-linked acrylates, nonionic polyacrylamides, xanthan gum, guar gum, gellan gum, and the like; polyalkyl siloxanes, polyaryl siloxanes, and aminosilicones. In the final product compositions of the invention, thickeners may be included in the amount of up to about 10%, preferably, in the amount of from about 0.2% to about 5% by weight of the composition.

The specific examples of the suitable thickening silicon compounds include polydimethylsiloxane, phenylsilicone, polydiethylsiloxane, and polymethylphenylsiloxane. Some of the suitable silicon compounds are described in European Patent Application EP 95,238 and U.S. Patent No. 4,185,017, which are incorporated herein by reference. The compositions of the invention may also include silicone polymer materials, which provide both style retention and conditioning benefits to the hair. Such materials are described in U.S. Patent No. 4,902,499, which is incorporated herein by reference.

HAIR CONDITIONING AGENTS

The compositions of the invention may also include hydrolyzed animal protein hair conditioning agents. Croda Incorporated sells an example of a commercially available material under the tradename Crotein Q-RTM. Other examples include urea, glycerol, and propoxylated glycerols, including those described in U.S. Patent No. 4,976,953, which is incorporated by reference herein.

HAIR SETTING AGENTS

The compositions of the invention may also include a hair setting agent to impart styling benefits upon application to hair. The hair setting polymers may be homopolymers, copolymers, terpolymers, etc. For convenience in describing the polymers hereof, monomeric units present in the polymers may be referred to as the monomers from which they can be derived. The monomers can be ionic (e.g., anionic, cationic, amphoteric, zwitterionic) or nonionic.

Examples of anionic monomers include unsaturated carboxylic acid monomers such as acrylic acid, methacrylic acid, maleic acid, maleic acid half ester, itaconic acid, fumaric acid, and crotonic acid; half esters of an unsaturated polybasic acid anhydride such as succinic anhydride, phthalic anhydride or the like with a hydroxyl group-containing acrylate and/or methacrylate such as hydroxyethyl acrylate and, hydroxyethyl methacrylate, hydroxypropyl acrylate and the like; monomers having a sulfonic acid group such as styrenesulfonic acid, sulfoethyl acrylate and methacrylate, and the like; and monomers having a phosphoric acid group such as acid phosphooxyethyl acrylate and methacrylate, 3-chloro-2-acid phosphooxypropyl acrylate and methacrylate, and the like.

Examples of cationic monomers include monomers derived from acrylic acid or methacrylic acid, and a quaternarized epihalohydrin product of a trialkylamine having 1 to 5 carbon atoms in the alkyl such as (meth)acryloxypropyltrimethylammonium chloride and (meth)acryloxypropyl-triethylammonium bromide; amine derivatives of methacrylic acid or amine derivatives of methacrylamide derived from methacrylic acid or methacrylamide and a dialkylalkanolamine having $C_1$ -$C_6$ alkyl groups such as dimethylaminoethyl (meth)acrylate, diethylaminoethyl (meth)acrylate, dimethylaminopropyl (meth) acrylate, or dimethylaminopropyl (meth)acrylamide.

Examples of the amphoteric monomers include zwitterionized derivatives of the aforementioned amine derivatives of (meth)acrylic acids or the amine derivatives of (meth)acrylamide such as dimethylaminoethyl (meth) acrylate, dimethylaminopropyl(meth)acrylamide by a halogenated fatty acid salt such as potassium monochloroacetate, sodium monobromopropionate, aminomethylpropanol salt of monochloroacetic acid, triethanolamine salts of monochloroacetic acid and the like; and amine derivatives of (meth)acrylic acid or (meth)acrylamide, as discussed above, modified with propanesultone.

Examples of nonionic monomers are acrylic or methacrylic acid esters of $C_1$ -$C_{24}$ alcohols, such as methanol, ethanol, 1-propanol, 2-propanol, 1-butanol, 2-methyl-1-propanol, 1-pentanol, 2-pentanol, 3-pentanol, 2-methyl-1-butanol, 1-methyl-1-butanol, 3-methyl-1-butanol, 1-methyl-1-pentanol, 2-methyl-1-pentanol, 3-methyl-1-pentanol, t-butanol, cyclohexanol, 2-ethyl-1-butanol, 3-heptanol, benzyl alcohol, 2-octanol, 6-methyl-1-heptanol, 2-ethyl-1-hexanol, 3,5-dimethyl-1-hexanol, 3,5,5-trimethyl-1-hexanol, 1-decanol, 1-dodecanol, 1-hexadecanol, 1-octadecanol, styrene; chlorostyrene; vinyl esters such as vinyl acetate; vinyl chloride; vinylidene chloride; acrylonitrile; alpha-methylstyrene; t-butylstyrene; butadiene; cyclohexadiene; ethylene; propylene; vinyl toluene; alkoxyalkyl (meth)acrylate, methoxy ethyl (meth)acrylate, butoxyethyl (meth)acrylate; allyl acrylate, allyl methacrylate, cyclohexyl acrylate and methacrylate, oleyl acrylate and methacrylate, benzyl acrylate and methacrylate, tetrahydrofurfuryl acrylate and methacrylate, ethylene glycol di-acrylate and -methacrylate, 1,3-butyleneglycol di-acrylate and -methacrylate, diacetonacrylamide, isobornyl (meth)acrylate, n-butyl methacrylate, isobutyl methacrylate, 2-ethylhexyl methacrylate, methyl methacrylate, t-butylacrylate, t-butylmethacrylate, and mixtures thereof.

Examples of anionic hair styling polymers are copolymers of vinyl acetate and crotonic acid, terpolymers of

vinyl acetate, crotonic acid and a vinyl ester of an alpha-branched saturated aliphatic monocarboxylic acid such as vinyl neodecanoate; and copolymers of methyl vinyl ether and maleic anhydride, acrylic copolymers and terpolymers containing acrylic acid or methacrylic acid.

Examples of cationic hair styling polymers are copolymers of amino-functional acrylate monomers such as lower alkylamino alkyl acrylate or methacrylate monomers such as dimethyl aminoethylmethacrylate with compatible monomers such as N-vinylpyrrolidone or alkyl methacrylates such as methyl methacrylate and ethyl methacrylate and alkyl acrylates such as methyl acrylate and butyl acrylate.

MISCELLANEOUS COMPONENTS

The compositions of the invention may also include a wide range of miscellaneous ingredients. Some suitable miscellaneous ingredients commonly used in the cosmetic and personal care industry are described in The CTFA Cosmetic Ingredient Handbook, (2nd Ed., 1992), which is incorporated by reference herein.

Thus, the compositions of the invention may also include one or more absorbents, anti-acne agents, anti-perspirants, anticaking agents, antifoaming agents, antimicrobial agents, antioxidants, antidandruff agents, astringents, binders, buffers, biological additives, buffering agents, bulking agents, chelating agents, chemical additives, coupling agents, conditioners, colorants, cosmetic astringents, cosmetic biocides, denaturants, drug astringents, detergents, dispersants, external analgesics, film formers, foaming agents, fragrance components, humectants, keratolytics, opacifying agents, pH adjusters, preservatives, propellants, proteins, retinoids, reducing agents, sequestrants, skin bleaching agents, skin-conditioning agents (humectants, miscellaneous, and occulsive), skin soothing agents, skin healing agents, softeners, solubilizing agents, lubricants, penetrants, plastisizers, solvents and co-solvents, sunscreening additives, salts, essential oils, and vitamins.

The examples of suitable pH adjusters include sodium hydroxide, triethanoleamine, and aminomethylpropanol, and mixtures thereof. If pH adjusters are present in a final product composition, the amount may vary from about 0.01% to about 5%, preferably, from about 0.1% to about 2% by weight of the composition.

The examples of suitable film formers include glycerin/diethylene glycol myrystate copolymer, glycerin/diethylene glycol adipate copolymer, ethyl ester of PVM/MA copolymer, PVP/dimethiconylacrylate/polycarbamyl/polyglycol ester, and mixtures thereof. If the film formers are present in the final product compositions, the amount may vary from about 0.1% to about 15.0% by weight of the composition, preferably, from about 0.1% to about 2.5% by weight of the composition.

The examples of suitable vitamins include tocopherol, tocopherol acetate, retinoic acid, retinol, and retinoids.

The examples of suitable anti-acne medicaments include resorcinol, sulfur, salicylic acid, erythromycin, zinc, and benzoyl peroxide.

The examples of suitable skin bleaching or lightening agents include hydroquinone, and kojic acid. The examples of suitable aesthetic components such as fragrances, pigments, colorings, and the like, include panthenol and derivatives (e.g., ethyl panthenol), aloe vera, pantothenic acid and its derivatives, clove oil, menthol, camphor, eucalyptus oil, eugenol, menthyl lactate, witch hazel distillate, allantoin, bisabolol, and dipotassium glycyrrhizinate.

The compositions of the invention are further illustrated in the examples that follow.

EXAMPLE 1. (for reference only) Preparation of 1-methyl-1-((erucylamido-) ethyl)-2-erucyl immidazolinium methyl sulfate.

3132 g (4.62 moles) of erucic acid and 216 g (2.1 moles) of diethylenetriamine are placed in a dry stirred pressure vessel fitted with a nitrogen inlet. The vessel is purged with nitrogen and heated to 170°C for 4-5 hours. The reaction mixture is then heated to 180°C and vacuum is applied for another 4-5 hours. The reaction mixture is cooled to 95-100°C and approximately 1.5 kg of cetearyl alcohol is added. The reaction mixture is further cooled to 75-80°C and 250 g of dimethyl sulfate is slowly added with stirring. Once all dimethyl sulfate is added, the reaction mixture is held at 75-80°C for approximately one hour, providing 1-methyl-1-((erucylamido-) ethyl)-2-erucyl immidazolinium methyl sulfate as the product.

EXAMPLE 2. (for reference only) Preparation of 1-methyl-1-(erucic rapeseed-)-ethyl)-2-(erucic rapeseed-) immidazolinium methyl sulfate (mixture of dialkyl immidazoline quats of hydrogenated rapeseed oil).

1843.6g (1.88 moles) of hydrogenated rapeseed oil and 283.34g (2.75 moles) of diethylenetriamine were placed in a dry stirred pressure vessel fitted with a nitrogen inlet. The vessel was purged with nitrogen and heated to 165°C for 5 hours until a base value of 76 was reached. The reaction mixture was then heated to 190°C and vacuum was applied for 5 hours to obtain a 94% tertiary amine content. The resulting immidazoline intermediate was then cooled to 95°C and 1772g of cetearyl alcohol were added to act as solvent. The reaction mixture was further cooled to 85°C and 330g

(2.6 moles) of dimethyl sulfate were slowly added over a 30 minute period with stirring. Once all dimethyl sulfate was added, the reaction mixture was held at 85-90°C for another 60 minutes. The resulting light yellow solid product included di-hydrogenated rapeseed oil imidazoline quat and cetearyl alcohol. The cationic activity of the mixture was 54%. The product was capable of being flaked or pastillated.

EXAMPLE 3. (for reference only) Preparation of 1-methyl-1-N-(n-propyl)-2-erucyl immidazolinium methyl sulfate.

1790 g (2.64 moles) of erucic acid and 245 g (2.4 moles) of N-(n-propyl)-N-ethyleneamino)-diamine are placed in a dry stirred pressure vessel fitted with a nitrogen inlet. The vessel is purged with nitrogen and heated to 195°C for 4-5 hours. The reaction mixture is then heated to 190°C and vacuum is applied for another 4-5 hours. The reaction mixture is cooled to 100-105°C and approximately 1.6 kg of cetearyl alcohol is added. The reaction mixture is further cooled to 75-80°C and approximately 280 g of dimethyl sulfate is slowly added with stirring. Once all dimethyl sulfate is added, the reaction mixture is held at 85-90°C for approximately one hour, providing 1-methyl-1-N-(n-propyl)-2-erucyl immidazolinium methyl sulfate as the product.

EXAMPLE 4. (for reference only) Quat Raw Material 1

Quat Raw Material 1 has the following composition:

| Function | Components |
|---|---|
| Quat(s) | Mixture M4 (Table 3) |
| Solvent | Mixture of cetearyl alcohol (80%) and 1,3-butanediol (20%) |

Cationic activity of the Quat Raw Material 1 is 45%.

EXAMPLE 5. (for reference only) Quat Raw Material 2

Quat Raw Material 2 has the following composition:

| Function | Component |
|---|---|
| Quat | Mixture of Table 7 |
| Solvent | Cetyl alcohol |

Cationic activity of the Quat Raw Material 2 is 25%.

EXAMPLE 6. (for reference only) Sunscreen Lotion

A sunscreen lotion includes the following ingredients:
Phase A

| Ingredient(s) | % W/W |
|---|---|
| Di-erucic imidazoline quat | 1.0 |
| Benzophenone 3 | 6.0 |
| Cetearyl Alcohol | 4.0 |
| Crodamol OS (Octyl Stearate) | 15.0 |
| Octyl Methoxycinnamate | 7.5 |

Phase B

| Ingredient | W/W% |
|---|---|
| Water | 65.50 |

Phase C

| Ingredient | W/W% |
|---|---|
| Germaben II (preservative) | 1.0 |

The sunscreen lotion is prepared as follows. The ingredients of Phase A are combined and heated to 75°C. In a separate vessel, the ingredients of Phase B are also combined and heated to 75°C. Phase A is added to Phase B with stirring, and the stirring is continued while the combined phases are cooled to 40°C. Phase C is added, the cooling is continued to 25°C, providing the desired lotion.

EXAMPLE 7. (for reference only) Sunscreen Spray Lotion

A sunscreen spray lotion includes the following ingredients:
Phase A

| Ingredient | % W/W |
|---|---|
| Di-erucic imidazoline quat | 1.0 |
| PPG-3 Benzyl Myristate | 11.0 |
| Benzophenone 3 | 6.0 |
| Octyl Methoxycinnamate | 7.0 |
| Menthyl Anthranilate | 5.0 |
| Cromollient SCE (Di-PPG-2 Myreth-10 Adipate) | 3.0 |

Phase B

| Ingredient | W/W% |
|---|---|
| Water | 66 |
| Sodium Hydroxide | 0.1 |

Phase C

| Ingredient | W/W% |
|---|---|
| Germaben II (preservative) | 1.0 |

The sunscreen spray lotion is prepared as follows. The ingredients of Phase A are combined and heated to 75°C. In a separate vessel, the ingredients of Phase B are also combined and heated to 75°C. Phase A is added to Phase B with stirring, and the stirring is continued while the combined phases are cooled to 40°C. Phase C is added, the cooling is continued to 25°C, providing the desired lotion.

EXAMPLE 8. (for reference only) Hair conditioner

A hair conditioner includes the following ingredients:
Phase A

| Ingredient | % W/W |
|---|---|
| Di-$C_{20-24}$ Immidazoline Quat | 1.0 |
| Cromollient SCE (Di-PPG-2 Myreth-10 Adipate) | 5.0 |
| Cetyl Alcohol | 4.0 |

Phase B

| Ingredient | W/W% |
|---|---|
| Water | 89 |

Phase C

| Ingredient | W/W% |
|---|---|
| Germaben II (preservative) | 1.0 |

The hair conditioner is prepared as follows. The ingredients of Phase A are combined and heated to 75°C. In a separate vessel, the ingredients of Phase B are also combined and heated to 75°C. Phase A is added to Phase B with stirring, and the stirring is continued while the combined phases are cooled to 40°C. Phase C is added, the cooling is continued to 25°C, providing the desired lotion.

EXAMPLE 9. (for reference only) Soft & Shine Conditioner

A soft and shine conditioner includes the following ingredients.
Phase A

| Ingredient | % W/W |
|---|---|
| Water | 86.26 |
| Mixture of Di-behenyl imidazolinium Methosulfate and Cetrimonium Methasulfate (7/3 w/w) in Cetearyl alcohol (70% actives) | 2.14 |
| CRODACOL C-70 (Cetyl Alcohol) | 1.00 |
| CRODACOL S-70 (Stearyl Alcohol) | 3.00 |
| CRILLET 3 (Polysorbate 60) | 1.00 |

Part B

| Ingredient | W/W% |
|---|---|
| INCROMINE SB (Stearamidopropyl Dimethylamine) | 0.5 |
| Cyclopentasiloxane (and) Dimethicone (1) | 4.0 |
| Dimethicone (2) | 0.5 |
| Disodium EDTA | 0.2 |
| Propylene Glycol (and) Diazolidinyl Urea (and) Methylparaben (and) Propylparaben (3) | 1.00 |
| Citric acid | 0.4 |

Part C

| Ingredient | W/W% |
|---|---|
| Germaben II (Preservative) | 1.0 |

EXAMPLE 10. (for reference only) Vitamin E-containing Conditioner

The vitamin E-containing conditioner has the following ingredients.
Phase A

| Ingredient | % W/W |
|---|---|
| Deionized Water | 92.50 |
| CRODACOL S-70 (Stearyl Alcohol) | 3.80 |
| Mixture of Dibehenyl imidazolinium Methosulfate and Cetrimonium Methasulfate (7/3 w/w) in Cetearyl alcohol (70% actives) | 2.20 |

Part B

| Ingredient | W/W% |
|---|---|
| DL-α Tocopherol Acetate (1) | 0.50 |

Part C

| Ingredient | W/W% |
|---|---|
| Propylene Glycol (and) Diazolidinyl Urea (and) Methylparaben (and) Propylparaben (2) | 1.00 |

Preparation is as follows: combine Part A ingredients with mixing and heat to 75-80°C. Cool to 35°C and add Parts B and C one at a time, mixing well.

EXAMPLE 11. (for reference only) Deposition of Vitamin E on Hair (Comparative Experiment 1).

Virgin and bleached hair tresses were treated with a simple conditioning formula containing 0.5% of the vitamin and 1.5% of either mixture of Di-behenyl imidazolinium Methosulfate and Cetrimonium Methosulfate (7/3 w/w) or Behentrimonium Chloride. The actives were present at the same level. Tresses were washed for 30 seconds and rinsed, after which the deposited Vitamin E was extracted with a solvent and measured by UV absorbance.
The results are shown below

| Ingredient | Deposition of Vitamin E (mg of Vit E/100g of hair) | |
|---|---|---|
| | Virgin Hair | Bleached Hair |
| Behentrimonium Chloride | 86.3 | 57.6 |
| Mixture of Di-behenyl imidazolinium Methosulfate and Cetrimonium Methasulfate (7/3 w/w) | 180 | B0.9 |

EXAMPLE 12. (for reference only) Deposition of Vitamin E (Comparative Experiment 2).

Test conditioning shampoo formulations A and B were prepared using a mixture of Di-behenyl imidazolinium Methosulfate and Cetrimonium Methasulfate in 7/3 w/w ratio of quats to one another as the active conditioning ingredient. The added conditioning ingredient, which is generally derived from HEAR oil, contained 70% active quats by cationic activity in cetearyl alcohol. Test formulation A contained 1% of quat by cationic activity and test formulation A contained

0.5% of quat by cationic activity. Polyquaternium-10, a well-known polymeric conditioner, was used in the reference formulation C. The smaller amount of Polyquaternium-10 was used to reflect the cost benefit consideration.

Hair samples were treated with the respective conditioning shampoo for 3 minutes and rinsed off under 40°C running tap water with a flow rate of 2.51/min for 20 seconds. The total substantivity was determined by two consecutive extractions by PVCS Method # 7-1. Only trace of Vitamin E was detected in the third extraction solution.

The determined total substantivity of Vitamin E delivered from these conditioning shampoo samples is presented below:

| Ingredient//Amount added to the Test Shampoo | Average Substantivity (mg Vit E/100g hair) by PVCS Method # 7-1 | | |
|---|---|---|---|
| | First extraction | Second extraction | Total |
| Mixture of Di-behenyl imidazolinium Methosulfate and Cetrimonium Methasulfate (7/3 w/w)// 1% cationic activity | 52.7 | 23.1 | 75.8 |
| Mixture of di-behenyl imidazolinium methosulfate and cetrimonium methasulfate (7/3 w/w ratio) in cetearyl alcohol (70% actives)// 0.5% cationic activity | 46.4 | 44.6 | 91.0 |
| Polyquaternium-10// 0 .3% by cationic activity | 24.3 | 15.6 | 39.9 |

The conditioning shampoo samples containing di-behenyl imidazolinium methosulfate showed better deposition of Vitamin E onto hair surface than the sample containing Polyquaternium-10. Also, the deposition of Vitamin E on hair surface was enhanced by an increase in the concentration of di-behenyl imidazolinium methosulfate in the formulation.

Unless stated to the contrary, any use of the words such as "including," "containing," "comprising," "having" and the like, means "including without limitation" and shall not be construed to limit any general statement that it follows to the specific or similar items or matters immediately following it. Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

**1.** A cosmetic or personal care composition comprising:

(a) a mixture of dialkyl immidazoline quats wherein at least a portion of the mixture includes at least one dialkyl immidazoline quat having at least one $C_{16}$-$C_{30}$ alkyl group; the $C_{16}$-$_{30}$ substitution content of the mixture being from about 10% to about 95% with respect to $C_{10+}$ reference substitution range, and

(b) at least one active ingredient selected from the group consisting of a surfactant, emollient, sunscreen compound, emulsifier, anti-dandruff agent, hair oxidizer/reducer, thickener, hair conditioning agent, hair setting agent, hair styling agent, cleansing agent, skin conditioning agent, hair growth promoter, perfume, pigment, moisturizer, film former, hair color, make-up agent, deodorant active, and pharmaceutical useful for topical purposes,

wherein, said personal care composition is selected from the group consisting of a shampoo, hair conditioner, sunscreen formulation, suntan oil, bath composition, hand cleaner, anti-perspirant composition, perfume, cologne, cold cream, pre-shave, deodorant, topical pharmaceutical ointment, skin moisturizer, facial cleanser, cleansing cream, skin gel, make-up product, lipstick product, mascara and hair coloring product.

**2.** The cosmetic or personal care composition of claim 1, wherein said $C_{16-30}$ substitution content is from about 15% to about 80%.

**3.** The cosmetic or personal care composition of claim 2, wherein said $C_{16-30}$ substitution content is from about 20% to about 70%.

4. The cosmetic or personal care composition of claim 3, wherein said $C_{16-30}$ substitution content is from about 35% to about 60%.

5. The cosmetic or personal care composition of claim 1 wherein said composition comprises a mixture of dialkyl immidazoline quats wherein at least a portion of the mixture includes at least one dialkyl immidazoline quat having at least one $C_{20}$-$C_{30}$ alkyl group; the $C_{20-30}$ substitution content of the mixture being from about 10% to about 95% with respect to $C_{10+}$ reference substitution range.

6. The cosmetic or personal care composition of claim 5, wherein said $C_{20-30}$ substitution content is from about 15% to about 80%.

7. The cosmetic or personal care composition of claim 6, wherein said $C_{20-30}$ substitution content is from about 20% to about 70%.

8. The cosmetic or personal care composition of claim 7, wherein said $C_{20-30}$ substitution content is from about 35% to about 60%.

9. The cosmetic or personal care composition of claim 1, wherein said composition comprises a mixture of dialkyl immidazoline quats wherein at least a portion of the mixture includes at least one dialkyl immidazoline quat having at least one $C_{20}$-$C_{24}$ alkyl group; the $C_{20-24}$ substitution content of said mixture being from about 10% to about 95% with respect to $C_{10+}$ reference substitution range.

10. The cosmetic or personal care composition of claim 9, wherein said $C_{20-24}$ substitution content is from about 15% to about 80%.

11. The cosmetic or personal care composition of claim 10, wherein said $C_{20-24}$ substitution content is from about 20% to about 70%.

12. The cosmetic or personal care composition of claim 11, wherein said $C_{20-24}$ substitution content is from about 35% to about 60%.

13. The cosmetic or personal care composition of claims 1, 5 or 9 which is substantially free from monoalkyl immidazoline quats.

14. The cosmetic or personal care composition of claims 1, 5 or 9, wherein the dialkyl immidazoline quats of the mixture have the formula (I) :

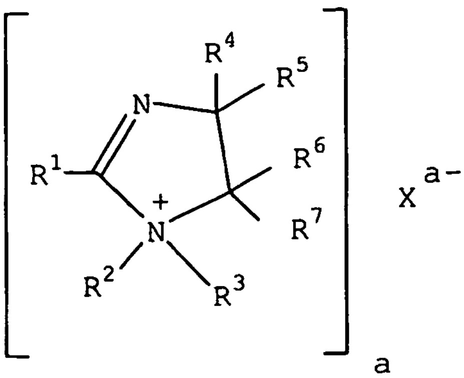

(I) ,

where

X is a salt-forming anion selected from the group consisting of chloride, bromide, iodide, fluoride, sulfate, methyl sulfate, methanebenzylsulfonate, phosphate, nitrite, nitrate, carboxylate, and mixtures thereof;

a is the ionic charge of X;
$R^1$, $R^2$, and $R^3$ are independently hydrogen, $C_1$-$C_{30}$ alkyl, $C_1$-$C_{30}$ alkylhydroxy, $C_1$-$C_{30}$ alkyl amido $R_{(C1-C6)}$, $C_1$-$C_{30}$ alkylaryl amido $R_{(C1-C6)}$ or $C_1$-$C_{30}$ alkylhydroxy amido $R_{(C1-C6)}$, $R_{(C1-C6)}$ being $C_1$-$C_6$ alkylene or benzyl;
two of $R^1$, $R^2$, and $R^3$ are independently $C_{10}$-$C_{30}$ alkyl, $C_{10}$-$C_{30}$ alkylhydroxy, $C_{10}$-$C_{30}$ alkyl amido $R_{(C1-C6)}$, $C_{10}$-$C_{30}$ alkylaryl amido $R_{(C1-C6)}$ or $C_{10}$-$C_{30}$ alkylhydroxy amido $R_{(C1-C6)}$;
the remaining one of $R^1$, $R^2$ and $R^3$ is hydrogen, $C_1$-$C_8$ alkyl, $C_1$-$C_8$ alkylhydroxy, $C_1$-$C_8$ alkyl amido $R_{(C1-C6)}$, $C_1$-$C_8$ alkylaryl amido $R_{(C1-C6)}$ or $C_1$-$C_8$ alkylhydroxy amido $R_{(C1-C6)}$;
$R^4$, $R^5$, $R^6$, and $R^7$, same or different, are independently hydrogen, alkyl, arylalkyl, alkylaryl, fluoro, bromo, chloro, iodo, acetoxy, alkylacetoxy, arylacetoxy, carboxy, alkylcarboxy, hydroxy or alkoxyhydroxy.

**15.** The cosmetic or personal care composition of claim 14, wherein $R^1$ is $C_{10}$-$C_{30}$ alkyl or $C_{10}$-$C_{30}$ alkylhydroxy, $R^2$ is $C_1$-$C_6$ alkyl, $R^3$ is $C_{10}$-$C_{30}$ alkyl amido $R_{(C1-C6)}$ or $C_{10}$-$C_{30}$ alkylhydroxy amido $R_{(C1-C6)}$, and $R^4$, $R^5$, $R^6$, and $R^7$ are independently hydrogen or $C_1$-$C_8$ alkyl.

**16.** The cosmetic or personal care composition of claim 15, wherein $R^1$ is $C_{10}$-$C_{30}$ alkyl, $R^2$ is methyl, $R^3$ is $C_{10}$-$C_{30}$ alkylhydroxy amido $C_1$-$C_3$ alkylene, $R^4$, $R^5$, $R^6$, and $R^7$ are hydrogen, and X is chloride or methyl sulfate.

**17.** The cosmetic or personal care composition of claim 15, wherein $R^1$ is $C_{10}$-$C_{30}$ alkyl, $R^2$ is methyl, $R^3$ is $C_{10}$-$C_{30}$ alkyl amido $C_1$-$C_3$ alkylene, $R^4$, $R^5$, $R^6$, and $R^7$ are hydrogen, and X is chloride or methyl sulfate.

**18.** The cosmetic or personal care composition of claim 17, wherein $R^3$ is $C_{10}$-$C_{30}$ alkyl amido ethylene.

**19.** The cosmetic or personal care composition of claim 14, wherein, for said portion of said mixture, $R^1$ is $C_{16}$-$C_{30}$ alkyl or $C_{16}$-$C_{30}$ alkylhydroxy, $R^2$ is $C_1$-$C_3$ alkyl, $R^3$ is $C_{10}$-$C_{30}$ alkyl amido $R_{(C1-C6)}$, and $R^4$, $R^5$, $R^6$, and $R^7$ are hydrogen.

**20.** The cosmetic or personal care composition of claim 14, wherein, for said portion of said mixture, $R^1$ is $C_{16}$-$C_{30}$ alkyl or $C_{16}$-$C_{30}$ alkylhydroxy, $R^2$ is $C_1$-$C_3$ alkyl, $R^3$ is $C_{16}$-$C_{30}$ alkyl amido $R_{(C1-C6)}$, and $R^4$, $R^5$, $R^6$, and $R^7$ are hydrogen.

**21.** The cosmetic or personal care composition of claim 14, wherein, for said portion of said mixture, $R^1$ is $C_{10}$-$C_{30}$ alkyl or $C_{10}$-$C_{30}$ alkylhydroxy, $R^2$ is $C_1$-$C_3$ alkyl, $R^3$ is $C_{16}$-$C_{30}$ alkyl amido $R_{(C1-C6)}$, and $R^4$, $R^5$, $R^6$, and $R^7$ are hydrogen.

**22.** The cosmetic or personal care composition of claim 14, wherein, for said portion of said mixture, $R^1$ is $C_{20}$-$C_{30}$ alkyl or $C_{20}$-$C_{30}$ alkylhydroxy, $R^2$ is $C_1$-$C_3$ alkyl, $R^3$ is $C_{10}$-$C_{30}$ alkyl amido $R_{(C1-C6)}$, and $R^4$, $R^5$, $R^6$, and $R^7$ are hydrogen.

**23.** The cosmetic or personal care composition of claim 14, wherein, for said portion of said mixture, $R^1$ is $C_{20}$-$C_{30}$ alkyl or $C_{20}$-$C_{30}$ alkylhydroxy, $R^2$ is $C_1$-$C_3$ alkyl, $R^3$ is $C_{20}$-$C_{30}$ alkyl amido $R_{(C1-C6)}$, and $R^4$, $R^5$, $R^6$, and $R^7$ are hydrogen.

**24.** The cosmetic or personal care composition of claim 14, wherein, for said portion of said mixture, $R^1$ is $C_{10}$-$C_{30}$ alkyl or $C_{10}$-$C_{30}$ alkylhydroxy, $R^2$ is $C_1$-$C_3$ alkyl, $R^3$ is $C_{20}$-$C_{30}$ alkyl amido $R_{(C1-C6)}$, and $R^4$ , $R^5$, $R^6$, and $R^7$ are hydrogen.

**25.** The cosmetic or personal care composition of claim 14, wherein, for said portion of said mixture, $R^1$ is $C_{20}$-$C_{24}$ alkyl or $C_{20}$-$C_{24}$ alkylhydroxy, $R^2$ is $C_1$-$C_3$ alkyl, $R^3$ is $C_{10}$-$C_{30}$ alkyl amido $R_{(C1-C6)}$, and $R^4$, $R^5$, $R^6$, and $R^7$ are hydrogen.

**26.** The cosmetic or personal care composition of claim 14, wherein, for said portion of said mixture, $R^1$ is $C_{20}$-$C_{24}$ alkyl or $C_{20}$-$C_{24}$ alkylhydroxy, $R^2$ is $C_1$-$C_3$ alkyl, $R^3$ is $C_{20}$-$C_{24}$ alkyl amido $R_{(C1-C6)}$, and $R^4$, $R^5$, $R^6$, and $R^7$ are hydrogen.

**27.** The cosmetic or personal care composition of claim 14, wherein, for said portion of said mixture, $R^1$ is $C_{10}$-$C_{30}$ alkyl or $C_{10}$-$C_{30}$ alkylhydroxy, $R^2$ is $C_1$-$C_3$ alkyl, $R^3$ is $C_{20}$-$C_{24}$ alkyl amido $R_{(C1-C6)}$, and $R^4$ , $R^5$, $R^6$, and $R^7$ are hydrogen.

**28.** The cosmetic or personal care composition of claim 1, wherein said composition comprises a mixture of dialkyl immidazoline quats of the formula (II):

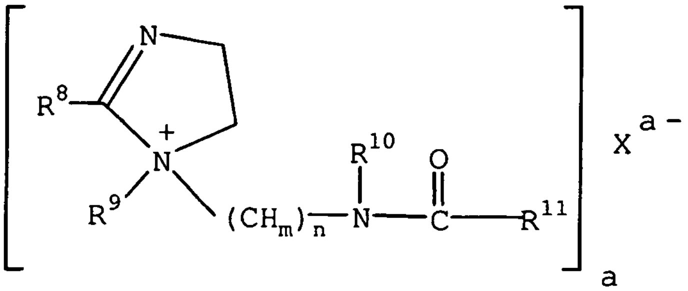

(II)

,

wherein

X is a salt-forming anion selected from the group consisting of chloride, bromide, iodide, fluoride, sulfate, methyl sulfate, methanebenzylsulfonate, phosphate, nitrite, nitrate, carboxylate, and mixtures thereof;
a is the ionic charge of X;
n varies from 1 to 3;
m is 1 or 2;
$R^8$ and $R^{11}$, same or different, are independently $C_{16}$-$C_{30}$ alkyl or $C_{16}$-$C_{30}$ alkylhydroxy;
$R^9$ is hydrogen or $C_1$-$C_3$ alkyl;
$R^{10}$ is hydrogen, alkyl, arylalkyl, alkylaryl, fluoro, bromo, chloro, iodo, acetoxy, alkylacetoxy, arylacetoxy, carboxy, alkylcarboxy, hydroxy or alkoxyhydroxy; and,
wherein at least a portion of the mixture includes at least one dialkyl immidazoline quat in which at least one of $R^8$ and $R^{11}$ is $C_{16}$-$C_{24}$ alkyl or $C_{16}$-$C_{24}$ alkylhydroxy ; the $C_{16-24}$ substitution content of said mixture being from about 10% to about 95% with respect to $C_{16-30}$ reference substitution range.

**29.** The cosmetic or personal care composition of claim 28, wherein said $C_{16-24}$ substitution content is from about 15% to about 80%.

**30.** The cosmetic or personal care composition of claim 29, wherein said $C_{16-24}$ substitution content is from about 20% to about 70%.

**31.** The cosmetic or personal care composition of claim 30, wherein said $C_{16-29}$ substitution content is from about 35% to about 60%.

**32.** The cosmetic or personal care composition of claim 28, wherein, for the dialkyl immidazoline quats of the mixture, $R^9$ is $C_1$-$C_3$ alkyl, $R^{10}$ is hydrogen, n is 2, and m is 2.

**33.** The cosmetic or personal care composition of claim 32, wherein, for the dialkyl immidazoline quats of said portion of said mixture, $R^8$ is $C_{16}$-$C_{24}$ alkyl or $C_{16}$-$C_{24}$ alkylhydroxy, and $R^{11}$ is $C_{16}$-$C_{30}$ alkyl or $C_{16}$-$C_{30}$ alkylhydroxy.

**34.** The cosmetic or personal care composition of claim 32, wherein, for the dialkyl immidazoline quats of said portion of said mixture, $R^8$ is $C_{16}$-$C_{24}$ alkyl or $C_{16}$-$C_{24}$ alkylhydroxy, and $R^{11}$ is $C_{16}$-$C_{24}$ alkyl or $C_{16}$-$C_{24}$ alkylhydroxy.

**35.** The cosmetic or personal care composition of claim 32, wherein, for the dialkyl immidazoline quats of said portion of said mixture, $R^8$ is $C_{16}$-$C_{30}$ alkyl or $C_{16}$-$C_{30}$ alkylhydroxy, and $R^{11}$ is $C_{16}$-$C_{24}$ alkyl or $C_{16}$-$C_{24}$ alkylhydroxy.

**36.** The cosmetic or personal care composition of claim 28, wherein, for said at least one dialkyl immidazoline quat of said portion of said mixture, $R^8$ and $R^{11}$ are both straight chain alkyl radicals of the formula $-C_{21}H_{43}$, $R^9$ is methyl, $R^{10}$ is hydrogen n is 2, and m is 2.

**37.** The cosmetic or personal care composition of claim 1, wherein said composition comprises a mixture of dialkyl immidazoline quats of the formula (II):

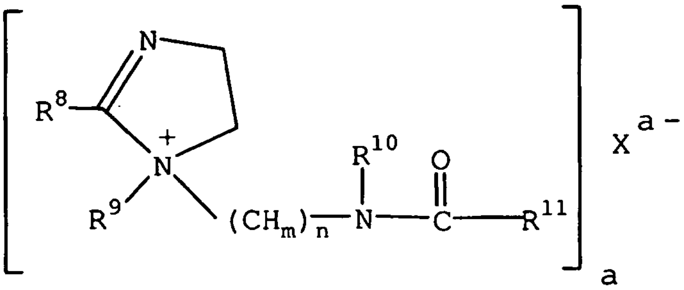

(II) ,

wherein

X is a salt-forming anion selected from the group consisting of chloride, bromide, iodide, fluoride, sulfate, methyl sulfate, methanebenzylsulfonate, phosphate, nitrite, nitrate, carboxylate, and mixtures thereof;
a is the ionic charge of X;
n varies from 1 to 3;
m is 1 or 2;
$R^8$ and $R^{11}$, same or different, are independently $C_{16}$-$C_{30}$ alkyl or $C_{16}$-$C_{30}$ alkylhydroxy;
$R^9$ is hydrogen or $C_1$-$C_3$ alkyl;
$R^{10}$ is hydrogen, alkyl, arylalkyl, alkylaryl, fluoro, bromo, chloro, iodo, acetoxy, alkylacetoxy, arylacetoxy, carboxy, alkylcarboxy, hydroxy or alkoxyhydroxy; and,
wherein at least a portion of the mixture includes at least one dialkyl immidazoline quat in which at least one of $R^8$ and $R^{11}$ is $C_{20}$-$C_{24}$ alkyl or $C_{20}$-$C_{24}$ alkylhydroxy; the $C_{20}$-$_{24}$ substitution content of said mixture being from about 10% to about 95% with respect to $C_{16}$-$_{30}$ reference substitution range.

**38.** The cosmetic or personal care composition of claim 37, wherein said $C_{20-24}$ substitution content is from about 15% to about 80%.

**39.** The cosmetic or personal care composition of claim 38, wherein said $C_{20-24}$ substitution content is from about 20% to about 70%.

**40.** The cosmetic or personal care composition of claim 39, wherein said $C_{20-24}$ substitution content is from about 35% to about 60%.

**41.** The cosmetic or personal care composition of claim 37, wherein, for the dialkyl immidazoline quats of the mixture, $R^9$ is $C_1$-$C_3$ alkyl, $R^{10}$ is hydrogen, n is 2, and m is 2.

**42.** The cosmetic or personal care composition of claim 41, wherein, for the dialkyl immidazoline quats of said portion of said mixture, $R^8$ is $C_{20}$-$C_{24}$ alkyl or $C_{20}$-$C_{24}$ alkylhydroxy, and $R^{11}$ is $C_{16}$-$C_{30}$ alkyl or $C_{16}$-$C_{30}$ alkylhydroxy.

**43.** The cosmetic or personal care composition of claim 41, wherein, for the dialkyl immidazoline quats of said portion of said mixt ure, $R^8$ is $C_{20}$-$C_{24}$ alkyl or $C_{20}$-$C_{24}$ alkylhydroxy, and $R^{11}$ is $C_{20}$-$C_{24}$ alkyl or $C_{20}$-$C_{24}$ alkylhydroxy.

**44.** The cosmetic or personal care composition of claim 41, wherein, for the dialkyl immidazoline quats of said portion of said mixture, $R^8$ is $C_{16}$-$C_{30}$ alkyl or $C_{16}$-$C_{30}$ alkylhydroxy, and $R^{11}$ is $20C_{24}$ alkyl or $C_{20}$-$C_{24}$ alkylhydroxy.

**45.** The cosmetic or personal care composition of claim 37, wherein, for said at least one dialkyl immidazoline quat of said portion of said mixture, $R^8$ and $R^{11}$ are both straight chain alkyl radicals of the formula $-C_{21}H_{43}$, $R^9$ is methyl, $R^{10}$ is hydrogen n is 2, and m is 2.

**46.** The cosmetic or personal care composition of claim 27 or 36, wherein $R^9$ is methyl.

**47.** The cosmetic or personal care composition of claim 27 or 36, wherein $R^{10}$ is hydrogen.

**48.** The cosmetic or personal care composition of claim 27 or 36, wherein n is 2, and m is 2.

**49.** The cosmetic or personal care composition of claim 1, wherein said at least one dialkyl immidazoline quat is of the formula (IA)

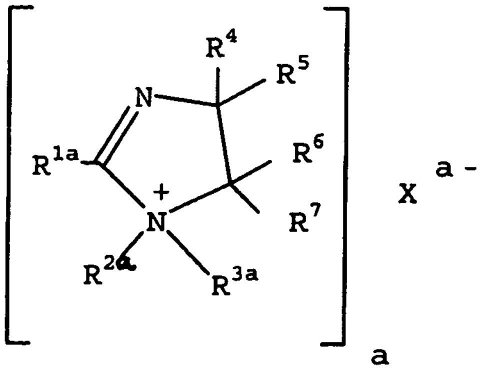

(IA)

,

where

X is a salt-forming anion selected from the group consisting of chloride, bromide, iodide, fluoride, sulfate, methyl sulfate, methanebenzylsulfonate, phosphate, nitrite, nitrate, carboxylate, and mixtures thereof;
a is the ionic charge of X;
$R^{1a}$, $R^{2a}$, and $R^{3a}$ are independently hydrogen, $C_1$-$C_{36}$ alkyl, $C_1$-$C_{36}$ alkylhydroxy, $C_1$-$C_{36}$ alkyl amido $R_{(C1-C6)}$, $C_1$-$C_{36}$ alkylaryl amido $R_{(C1-C6)}$ or $C_1$-$C_{36}$ alkylhydroxy amido $R_{(C1-C6)}$, $R_{(C1-C6)}$ being $C_1$-$C_6$ alkylene or benzyl;
two of $R^{1a}$, $R^{2a}$, and $R^{3a}$ are independently $C_{10}$-$C_{36}$ alkyl, $C_{10}$-$C_{36}$ alkylhydroxy, $C_{10}$-$C_{36}$ alkyl amido $R_{(C1-C6)}$, $C_{10}$-$C_{36}$ alkylaryl amido $R_{(C1-C6)}$ or $C_{10}$-$C_{36}$ alkylhydroxy amido $R_{(C1-C6)}$;
the remaining one of $R^{1a}$, $R^{2a}$ and $R^{3a}$ is hydrogen, $C_1$-$C_8$ alkyl, $C_1$-$C_8$ alkylhydroxy, $C_1$-$C_8$ alkyl amido $R_{(C1-C6)}$, $C_1$-$C_8$ alkylaryl amido $R_{(C1-C6)}$ or $C_1$-$C_8$ alkylhydroxy amido $R_{(C1-C6)}$; $R^4$, $R^5$, $R^6$, and $R^7$, same or different, are independently hydrogen, alkyl, arylalkyl, alkylaryl, fluoro, bromo, chloro, iodo, acetoxy, alkylacetoxy, arylacetoxy, carboxy, alkylcarboxy, hydroxy or alkoxyhydroxy;
with the proviso that the dialkyl immidazoline quat does not have the formula

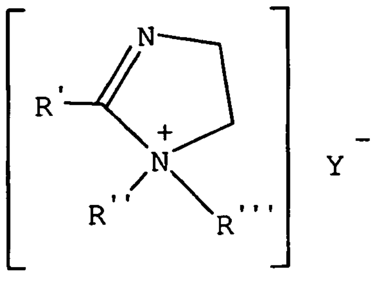

,

where
R' is $C_{11}$-$C_{22}$ alkyl or $C_{13}$-$C_{24}$ -alkyl hydroxy; R" is $C_1$-$C_6$ alkyl; and R"' is $C_{12}$-$C_{20}$ alkyl or $C_{11}$-$C_{22}$ alkyl amido $C_1$-$C_3$ alkylene.

**50.** The cosmetic or personal care composition of claim 49, wherein $R^{1a}$ is $C_{24}$-$C_{36}$ alkyl, $R^{2a}$ is independently hydrogen or $C_1$-$C_3$ alkyl, $R^{3a}$ has the structure

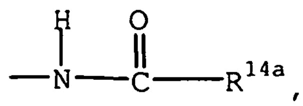

where $R^{14a}$, which may be same or different from $R^{1a}$, is $C_{24}$-$C_{36}$ alkyl; $R^4$, $R^5$ , $R^6$, and $R^7$ are all hydrogen; n varies from 1 to 3; and m is 1 or 2.

**51.** The cosmetic or personal care composition of claims 1, 5, 9, 28, or 37, further comprising at least one hydrophobic ingredient.

**52.** The cosmetic or personal care composition of claim 51, wherein said at least one hydrophobic ingredient is selected from the group consisting of botanical extracts, vitamin E, vitamin A, silicons, waxes and antioxidants.

**53.** The cosmetic or personal care composition of claim 52, wherein said at least one hydrophobic ingredient is vitamin E.

**54.** The cosmetic or personal care composition of claims 1, 5, 9, 28, or 37, further comprising one or more preservatives, fragrances, foam boosters, conditioners and emollients.

**55.** The cosmetic or personal care composition of claims 1, 5, 9, 28, or 37, further comprising at least one active ingredient present in the amount of between about 0.20 and about 40.0 percent by weight of the composition.

**56.** The cosmetic or personal care composition of claim 55, wherein said active ingredient selected from the group consisting of a sunscreen, pigment, moisturizer, film former, detergent, thickening agent, emulsifier, antiseptic agent, conditioner or deodorant.

**57.** The cosmetic or personal care composition of claims 1, 5, 9, 28, or 37, further comprising at least one additional surfactant present in the amount from about 1% to about 75% by weight of the composition.

**58.** The cosmetic or personal care composition of claim 57, wherein said additional surfactant is selected from the group consisting of non-ionic surfactants, anionic surfactants, cationic surfactants and amphoteric surfactants.

**59.** The cosmetic or personal care composition of claims 1, 5, 9, 28, or 37, which is a raw material quat.

**60.** The cosmetic or personal care composition of claim 59, further comprising a carrier.

**61.** The cosmetic or personal care composition of claim 60 having a cationic activity of greater than 20%.

**62.** The cosmetic or personal care composition of claim 61, wherein said cationic activity is greater than 35%.

**63.** The cosmetic or personal care composition of claim 62, wherein said cationic activity is greater than 50%.

**64.** The cosmetic or personal care composition of claim 1, 5, 9, 28, or 37, wherein said cosmetic or personal care product is a non-aqueous topical formulation.

**65.** The cosmetic or personal care composition of claim 1, 5, 9, 28 or 37, wherein said cosmetic or personal care product is an aqueous topical formulation.

## Patentansprüche

**1.** Kosmetische oder Körperpflegezusammensetzung, umfassend:

(a) eine Mischung aus Dialkylimidazolinquats, wobei zumindest ein Teil der Mischung zumindest ein Dialkylimidazolinquat mit zumindest einer $C_{16}$-$C_{30}$ Alkylgruppe beinhaltet; wobei der $C_{16}$-$_{30}$ Substitutionsgehalt der Mischung von etwa 10% bis etwa 95% bezüglich eines $C_{10+}$-Referenzsubstitutionsbereichs beträgt, und
(b) zumindest einen aktiven Inhaltsstoff ausgewählt aus der Gruppe bestehend aus einem grenzflächenaktivem

Stoff, Weichmacher, Sonnenschutzverbindung, Emulgator, Antischuppenmittel, Haaroxidationsverringerer, Verdicker, Haarspülmittel, Haarfestigermittel, Haarstylingmittel, Reinigungsmittel, Hautspülmittel, Haarwachstumsförderer, Parfum, Pigment, Feuchtigkeitsmittel, Filmbildner, Haarfarbe, Make-up Mittel, Deodorantaktivmittel, und für körperbehandlungsbezogene Zwecke dienliche Pharmazeutika,

wobei die Körperpflegezusammensetzung ausgewählt ist aus der Gruppe bestehend aus einem Shampoo, Haarspülung, Sonnenschutzformulierung, Sonnenöl, Badezusammensetzung, Handreiniger, Antiperspirantzusammensetzung, Parfum, Kölnischwasser, Coldcream, Preshave, Deodorant, körperbehandlungsbezogene pharmazeutische Salbe, Hautfeuchtigkeitsmittel, Gesichtsreinigungsmittel, Reinigungscreme, Hautgel, Make-up Produkt, Lippenstiftprodukt, Mascara und Haarfärbeprodukt.

**2.** Kosmetische oder Körperpflegezusammensetzung nach Anspruch 1, wobei der $C_{16-30}$ Substitutionsgehalt von etwa 15% bis etwa 80% beträgt.

**3.** Kosmetische oder Körperpflegezusammensetzung nach Anspruch 2, wobei der $C_{16-30}$ Substitutionsgehalt von etwa 20% bis etwa 70% beträgt.

**4.** Kosmetische oder Körperpflegezusammensetzung nach Anspruch 3, wobei der $C_{16-30}$ Substitutionsgehalt von etwa 35% bis etwa 60% beträgt.

**5.** Kosmetische oder Körperpflegezusammensetzung nach Anspruch 1, wobei die Zusammensetzung eine Mischung aus Dialkylimidazolinquats umfasst, wobei zumindest ein Teil der Mischung zumindest ein Dialkylimidazolinquat mit zumindest einer $C_{20}$-$C_{30}$ Alkylgruppe beinhaltet; wobei der $C_{20}$-$C_{30}$ Substitutionsgehalt der Mischung von etwa 10% bis etwa 95% bezüglich eines $C_{10+}$ Referenzsubstitutionsbereichs beträgt.

**6.** Kosmetische oder Körperpflegezusammensetzung nach Anspruch 5, wobei der $C_{20}$-$C_{30}$ Substitutionsgehalt von etwa 15% bis etwa 80% beträgt.

**7.** Kosmetische oder Körperpflegezusammensetzung nach Anspruch 6, wobei der $C_{20-30}$ Substitutionsgehalt von etwa 20% bis etwa 70% beträgt.

**8.** Kosmetische oder Körperpflegezusammensetzung nach Anspruch 7, wobei der $C_{20-30}$ Substitutionsgehalt von etwa 35% bis etwa 60% beträgt.

**9.** Kosmetische oder Körperpflegezusammensetzung nach Anspruch 1, wobei die Zusammensetzung eine Mischung aus Dialkylimidazolinquats umfasst, wobei zumindest ein Teil der Mischung zumindest ein Dialkylimidazolinquat mit zumindest einer $C_{20}$-$C_{24}$ Alkylgruppe beinhaltet; wobei der $C_{20-24}$ Substitutionsgehalt der Mischung von etwa 10% bis etwa 95% bezüglich eines $C_{10+}$ Referenzsubstitutionsbereichs beträgt.

**10.** Kosmetische oder Körperpflegezusammensetzung nach Anspruch 9, wobei der $C_{20-24}$ Substitutionsgehalt von etwa 15% bis etwa 80% beträgt.

**11.** Kosmetische oder Körperpflegezusammensetzung nach Anspruch 10, wobei der $C_{20-24}$ Substitutionsgehalt von etwa 20% bis etwa 70% beträgt.

**12.** Kosmetische oder Körperpflegezusammensetzung nach Anspruch 11, wobei der $C_{20-24}$ Substitutionsgehalt von etwa 35% bis etwa 60% beträgt.

**13.** Kosmetische oder Körperpflegezusammensetzung nach Anspruch 1, 5 oder 9, welche im Wesentlichen frei von Monoalkylimidazolinquats ist.

**14.** Kosmetische oder Körperpflegezusammensetzung nach Anspruch 1, 5 oder 9, wobei die Dialkylimidazolinquats der Mischung die Formel (I) aufweisen:

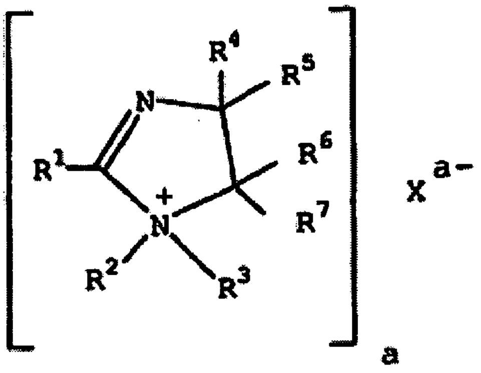

(I)

,

wobei

X ein salzbildendes Anion darstellt, ausgewählt aus der Gruppe bestehend aus Chlorid, Bromid, Jodid, Fluorid, Sulfat, Methylsulfat, Methanbenzylsulfonat, Phosphat, Nitrit, Nitrat, Carboxylat, und Mischungen davon;
a ist die Ionenladung von X;
$R^1$, $R^2$ und $R^3$ sind unabhängig voneinander Wasserstoff, $C_1$-$C_{30}$ Alkyl, $C_1$-$C_{30}$ Alkylhydroxy, $C_1$-$C_{30}$ Alkylamido $R_{(C1-C6)}$, $C_1$-$C_{30}$ Alkylarylamido $R_{(C1-C6)}$ oder $C_1$-$C_{30}$ Alkylhydroxyamido $R_{(C1-C6)}$, wobei $R_{(C1-C6)}$ $C_1$-$C_6$ Alkylen oder Benzyl darstellt;
zwei aus $R^1$, $R^2$ und $R^3$ sind unabhängig voneinander $C_{10}$-$C_{30}$ Alkyl, $C_{10}$-$C_{30}$ Alkylhydroxy, $C_{10}$-$C_{30}$ Alkylamido $R_{(C1-C6)}$, $C_{10}$-$C_{30}$ Alkylarylamido $R_{(C1-C6)}$ oder $C_{10}$-$C_{30}$ Alkylhydroxyamido $R_{(C1-C6)}$ ;
das eine verbleibende aus $R^1$, $R^2$ und R3 ist Wasserstoff, $C_1$-$C_8$ Alkyl, $C_1$-$C_8$ Alkylhydroxy, $C_1$-$C_8$ Alkylamido $R_{(C1-C6)}$, $C_1$-$C_8$ Alkylarylamido $R_{(C1-C6)}$ oder $C_1$-$C_8$ Alkylhydroxyamido $R_{(C1-C6)}$;
$R^4$, $R^5$, $R^6$ und $R^7$, gleich oder unterschiedlich, sind unabhängig voneinander Wasserstoff, Alkyl, Arylalkyl, Alkylaryl, Fluor, Brom, Chlor, Jod, Acetoxy, Alkylacetoxy, Arylacetoxy, Carboxy, Alkylcarboxy, Hydroxy oder Alkoxyhydroxy.

**15.** Kosmetische oder Körperpflegezusammensetzung nach Anspruch 14, wobei $R^1$ $C_{10}$-$C_{30}$ Alkyl oder $C_{10}$-$C_{30}$ Alkylhydroxy ist, $R^2$ $C_1$-$C_6$ Alkyl ist, $R^3$ $C_{10}$-$C_{30}$ Alkylamido $R_{(C1-C6)}$ oder $C_{10}$-$C_{30}$ Alkylhydroxyamido $R_{(C1-C6)}$ ist, und $R^4$, $R^5$, $R^6$ und $R^7$ sind unabhängig voneinander Wasserstoff oder $C_1$-$C_8$ Alkyl.

**16.** Kosmetische oder Körperpflegezusammensetzung nach Anspruch 15, wobei $R^1$ $C_{10}$-$C_{30}$ Alkyl ist, $R^2$ Methyl ist, $R^3$ $C_{10}$-$C_{30}$ Alkylhydroxyamido $C_{1-C3}$ Alkylen ist, $R^4$ , $R^5$, $R^6$ und $R^7$ Wasserstoff sind, und X Chlorid oder Methylsulfat ist.

**17.** Kosmetische oder Körperpflegezusammensetzung nach Anspruch 15, wobei $R^1$ $C_{10}$-$C_{30}$ Alkyl ist, $R^2$ Methyl ist, $R^3$ $C_{10}$-$C_{30}$ Alkylamido $C_1$-$C_3$ Alkylen ist, $R^4$, $R^5$, $R^6$ und $R^7$ Wasserstoff sind, und X Chlorid oder Methylsulfat ist.

**18.** Kosmetische oder Körperpflegezusammensetzung nach Anspruch 17, wobei $R^3$ $C_{10}$-$C_{30}$ Alkylamidoethylen ist.

**19.** Kosmetische oder Körperpflegezusammensetzung nach Anspruch 14, wobei für den Teil der Mischung $R^1$ $C_{16}$-$C_{30}$ Alkyl oder $C_{16}$-$C_{30}$ Alkylhydroxy ist, $R^2$ $C_1$-$C_3$ Alkyl ist, $R^3$ $C_{10}$-$C_{30}$ Alkylamido $R_{(C1-C6)}$ ist, und $R^4$, $R^5$, $R^6$ und $R^7$ Wasserstoff sind.

**20.** Kosmetische oder Körperpflegezusammensetzung nach Anspruch 14, wobei für den Teil der Mischung $R^1$ $C_{16}$-$C_{30}$ Alkyl oder $C_{16}$-$C_{30}$ Alkylhydroxy ist, $R^2$ $C_1$-$C_3$ Alkyl ist, $R^3$ $C_{16}$-$C_{30}$ Alkylamido $R_{(C1-C6)}$ ist, und $R^4$, $R^5$, $R^6$ und $R^7$ Wasserstoff sind.

**21.** Kosmetische oder Körperpflegezusammensetzung nach Anspruch 14, wobei für den Teil der Mischung $R^1$ $C_{10}$-$C_{30}$ Alkyl oder $C_{10}$-$C_{30}$ Alkylhydroxy ist, $R^2$ $C_1$-$C_3$ Alkyl ist, $R^3$ $C_{16}$-$C_{30}$ Alkylamido $R_{(C1-C6)}$ ist, und $R^4$, $R^5$, $R^6$ und $R^7$ Wasserstoff sind.

**22.** Kosmetische oder Körperpflegezusammensetzung nach Anspruch 14, wobei für den Teil der Mischung $R^1$ $C_{20}$-$C_{30}$

Alkyl oder $C_{10}$-$C_{30}$ Alkylhydroxy ist, $R^2$ $C_1$-$C_3$ Alkyl ist, $R^3C_{10}$-$C_{30}$ Alkylamido $R_{(C1-C6)}$ ist, und $R^4$, $R^5$, $R^6$ und $R^7$ Wasserstoff sind.

**23.** Kosmetische oder Körperpflegezusammensetzung nach Anspruch 14, wobei für den Teil der Mischung $R^1$ $C_{20}$-$C_{30}$ Alkyl oder $C_{20}$-$C_{30}$ Alkylhydroxy ist, $R^2$ $C_1$-$C_3$ Alkyl ist, $R^3$ $C_{20}$-$C_{30}$ Alkylamido $R_{(C1-C6)}$ ist, und $R^4$ , $R^5$, $R^6$ und $R^7$ Wasserstoff sind.

**24.** Kosmetische oder Körperpflegezusammensetzung nach Anspruch 14, wobei für den Teil der Mischung $R^1$ $C_{10}$-$C_{30}$ Alkyl oder $C_{20}$-$C_{30}$ Alkylhydroxy ist, $R^2$ $C_1$-$C_3$ Alkyl ist, $R^3$ $C_{20}$-$C_{30}$ Alkylamido $R_{(C1-C6)}$ ist, und $R^4$, $R^5$, $R^6$ und $R^7$ Wasserstoff sind.

**25.** Kosmetische oder Körperpflegezusammensetzung nach Anspruch 14, wobei für den Teil der Mischung $R^1$ $C_{20}$-$C_{24}$ Alkyl oder $C_{20}$-$C_{24}$ Alkylhydroxy ist, $R^2$ $C_1$-$C_3$ Alkyl ist, $R^3$ $C_{10}$-$C_{30}$ Alkylamido $R_{(C1-C6)}$ ist, und $R^4$, $R^5$, $R^6$ und $R^7$ Wasserstoff sind.

**26.** Kosmetische oder Körperpflegezusammensetzung nach Anspruch 14, wobei für den Teil der Mischung $R^1$ $C_{20}$-$C_{24}$ Alkyl oder $C_{20}$-$C_{24}$ Alkylhydroxy ist, $R^2$ $C_1$-$C_3$ Alkyl ist, $R^3$ $C_{20}$-$C_{24}$ Alkylamido $R_{(C1-C6)}$ ist, und $R^4$, $R^5$, $R^6$ und $R^7$ Wasserstoff sind.

**27.** Kosmetische oder Körperpflegezusammensetzung nach Anspruch 14, wobei für den Teil der Mischung $R^1$ $C_{10}$-$C_{30}$ Alkyl oder $C_{10}$-$C_{30}$ Alkylhydroxy ist, $R^2$ $C_1$-$C_3$ Alkyl ist, $R^3$ $C_{20}$-$C_{24}$ Alkylamido $R_{(C1-C6)}$ ist, und $R^4$, $R^5$, $R^6$ und $R^7$ Wasserstoff sind.

**28.** Kosmetische oder Körperpflegezusammensetzung nach Anspruch 1, wobei die Zusammensetzung eine Mischung aus Dialkylimidazolinquats der Formel (II) umfasst:

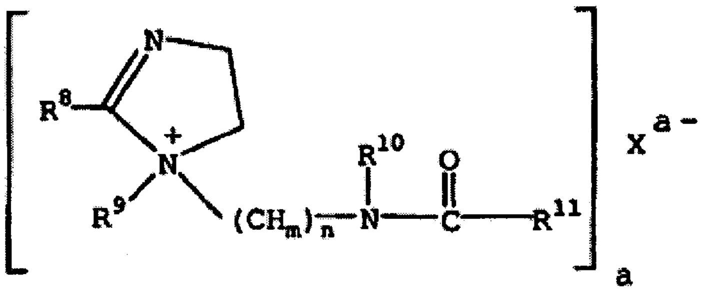

(II)

wobei

X ein salzbildendes Anion darstellt, ausgewählt aus der Gruppe bestehend aus Chlorid, Bromid, Jodid, Fluorid, Sulfat, Methylsulfat, Methanbenzylsulfonat, Phosphat, Nitrit, Nitrat, Carboxylat, und Mischungen davon;
a ist die Ionenladung von X;
n variiert von 1 bis 3;
m ist 1 oder 2;
$R^8$ und $R^{11}$, gleich oder unterschiedlich, sind unabhängig voneinander $C_{16}$-$C_{30}$ Alkyl oder $C_{16}$-$C_{30}$ Alkylhydroxy;
$R^9$ ist Wasserstoff oder $C_1$-$C_3$ Alkyl;
$R^{10}$ ist Wasserstoff, Alkyl, Arylalkyl, Alkylaryl, Fluor, Brom, Chlor, Jod, Acetoxy, Alkylacetoxy, Arylacetoxy, Carboxy, Alkylcarboxy, Hydroxy oder Alkoxyhydroxy; und,
wobei zumindest ein Teil der Mischung zumindest ein Dialkylimidazolinquat beinhaltet, bei welchem zumindest eines aus $R^8$ und $R^{11}$ $C_{16}$-$C_{24}$ Alkyl oder $C_{16}$-$C_{24}$ Alkylhydroxy ist; wobei der $C_{16-24}$ Substitutionsgehalt der Mischung von etwa 10% bis etwa 95% bezüglich eines $C_{16-30}$ Referenzsubstitutionsbereichs beträgt.

**29.** Kosmetische oder Körperpflegezusammensetzung nach Anspruch 28, wobei der $C_{16-24}$ Substitutionsgehalt von etwa 15% bis etwa 80% beträgt.

**30.** Kosmetische oder Körperpflegezusammensetzung nach Anspruch 29, wobei der $C_{16-24}$ Substitutionsgehalt von etwa 20% bis etwa 70% beträgt.

**31.** Kosmetische oder Körperpflegezusammensetzung nach Anspruch 30, wobei der $C_{16-24}$ Substitutionsgehalt von etwa 35% bis etwa 60% beträgt.

**32.** Kosmetische oder Körperpflegezusammensetzung nach Anspruch 28, wobei für die Dialkylimidazolinquats der Mischung $R^9$ $C_1$-$C_3$ Alkyl ist, $R^{10}$ Wasserstoff ist, n gleich 2 ist und m gleich 2 ist.

**33.** Kosmetische oder Körperpflegezusammensetzung nach Anspruch 32, wobei für die Dialkylimidazolinquats des Teils der Mischung $R^8$ $C_{16}$-$C_{24}$ Alkyl oder $C_{16}$-$C_{24}$ Alkylhydroxy ist und $R^{11}$ $C_{16}$-$C_{30}$ Alkyl oder $C_{16}$-$C_{30}$ Alkylhydroxy ist.

**34.** Kosmetische oder Körperpflegezusammensetzung nach Anspruch 32, wobei für die Dialkylimidazolinquats des Teils der Mischung $R^8$ $C_{16}$-$C_{24}$ Alkyl oder $C_{16}$-$C_{24}$ Alkylhydroxy ist und $R^{11}$ $C_{16}$-$C_{24}$ Alkyl oder $C_{16}$-$C_{24}$ Alkylhydroxy ist.

**35.** Kosmetische oder Körperpflegezusammensetzung nach Anspruch 32, wobei für die Dialkylimidazolinquats des Teils der Mischung $R^8$ $C_{16}$-$C_{30}$ Alkyl oder $C_{16}$-$C_{30}$ Alkylhydroxy ist und $R^{11}$ $C_{16}$-$C_{24}$ Alkyl oder $C_{16}$-$C_{24}$ Alkylhydroxy ist.

**36.** Kosmetische oder Körperpflegezusammensetzung nach Anspruch 28, wobei für das zumindest eine Dialkylimidazolinquat des Teils der Mischung $R^8$ und $R^{11}$ beide geradkettige Alkylradikale der Formel $-C_{21}H_{43}$ sind, $R^9$ Methyl ist, $R^{10}$ Wasserstoff ist, n gleich 2 ist und m gleich 2 ist.

**37.** Kosmetische oder Körperpflegezusammensetzung nach Anspruch 1, wobei die Zusammensetzung eine Mischung aus Dialkylimidazolinquats der Formel (II) umfasst:

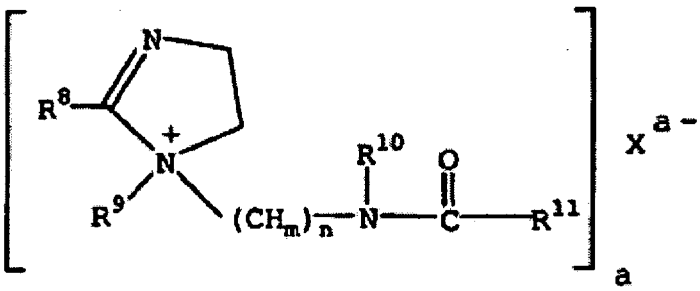

(II) ,

wobei

X ein salzbildendes Anion ausgewählt aus der Gruppe bestehend aus Chlorid, Bromid, Jodid, Fluorid, Sulfat, Methylsulfat, Methanbenzylsulfonat, Phosphat, Nitrit, Nitrat, Carboxylat, und Mischungen davon darstellt;
a ist die Ionenladung von X;
n variiert von 1 bis 3;
m ist 1 oder 2;
$R^8$ und $R^{11}$, gleich oder unterschiedlich, sind unabhängig voneinander $C_{16}$-$C_{30}$ Alkyl oder $C_{16}$-$C_{30}$ Alkylhydroxy;
$R^9$ ist Wasserstoff oder $C_1$-$C_3$ Alkyl;
$R^{10}$ ist Wasserstoff, Alkyl, Arylalkyl, Alkylaryl, Fluor, Brom, Chlor, Jod, Acetoxy, Alkylacetoxy, Arylacetoxy, Carboxy, Alkylcarboxy, Hydroxy oder Alkoxyhydroxy; und,
wobei zumindest ein Teil der Mischung zumindest ein Dialkylimidazolinquat beinhaltet, bei welchem zumindest eines aus $R^8$ und $R^{11}$ $C_{20}$-$C_{24}$ Alkyl oder $C_{20}$-$C_{24}$ Alkylhydroxy ist; wobei der $C_{20-24}$ Substitutionsgehalt der Mischung von etwa 10% bis etwa 95% bezüglich eines $C_{16-30}$ Referenzsubstitutionsbereichs beträgt.

**38.** Kosmetische oder Körperpflegezusammensetzung nach Anspruch 37, wobei der $C_{20-24}$ Substitutionsgehalt von etwa 15% bis etwa 80% beträgt.

**39.** Kosmetische oder Körperpflegezusammensetzung nach Anspruch 38, wobei der $C_{20-24}$ Substitutionsgehalt von etwa 20% bis etwa 70% beträgt.

**40.** Kosmetische oder Körperpflegezusammensetzung nach Anspruch 39, wobei der $C_{20-24}$ Substitutionsgehalt von etwa 35% bis etwa 60% beträgt.'

**41.** Kosmetische oder Körperpflegezusammensetzung nach Anspruch 37, wobei für die Dialkylimidazolinquats der Mischung $R^9$ $C_1$-$C_3$ Alkyl ist, $R^{10}$ Wasserstoff ist, n gleich 2 ist und m gleich 2 ist.

**42.** Kosmetische oder Körperpflegezusammensetzung nach Anspruch 41, wobei für die Dialkylimidazolinquats des Teils der Mischung $R^8$ $C_{20}$-$C_{24}$ Alkyl oder $C_{20}$-$C_{24}$ Alkylhydroxy ist, und $R^{11}$ $C_{16}$-$C_{30}$ Alkyl oder $C_{16}$-$C_{30}$ Alkylhydroxy ist.

**43.** Kosmetische oder Körperpflegezusammensetzung nach Anspruch 41, wobei für die Dialkylimidazolinquats des Teils der Mischung $R^8$ $C_{20}$-$C_{24}$ Alkyl oder $C_{20}$-$C_{24}$ Alkylhydroxy ist, und $R^{11}$ $C_{20}$-$C_{24}$ Alkyl oder $C_{20}$-$C_{24}$ Alkylhydroxy ist.

**44.** Kosmetische oder Körperpflegezusammensetzung nach Anspruch 41, wobei für die Dialkylimidazolinquats des Teils der Mischung $R^8$ $C_{16}$-$C_{30}$ Alkyl oder $C_{16}$-$C_{30}$ Alkylhydroxy ist, und $R^{11}$ $C_{20}$-$C_{24}$ Alkyl oder $C_{20}$-$C_{24}$ Alkylhydroxy ist.

**45.** Kosmetische oder Körperpflegezusammensetzung nach Anspruch 37, wobei für das zumindest eine Dialkylimidazolinquat des Teils der Mischung $R^8$ und $R^{11}$ beide geradkettige Alkylradikale der Formel $-C_{21}H_{43}$ sind, $R^9$ Methyl ist, $R^{10}$ Wasserstoff ist, n gleich 2 ist und m gleich 2 ist.

**46.** Kosmetische oder Körperpflegezusammensetzung nach Anspruch 27 oder 36, wobei $R^9$ Methyl ist.

**47.** Kosmetische oder Körperpflegezusammensetzung nach Anspruch 27 oder 36, wobei $R^{10}$ Wasserstoff ist.

**48.** Kosmetische oder Körperpflegezusammensetzung nach Anspruch 27 oder 36, wobei n gleich 2 ist und m gleich 2 ist.

**49.** Kosmetische oder Körperpflegezusammensetzung nach Anspruch 1, wobei das zumindest eine Dialkylimidazolinquat der Formel (IA) ist

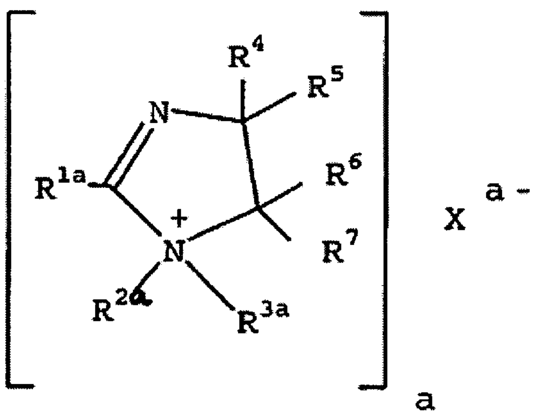

(IA) ,

wobei

X ein salzbildendes Anion ausgewählt aus der Gruppe bestehend aus Chlorid, Bromid, Jodid, Fluorid, Sulfat,

Methylsulfat, Methanbenzylsulfonat, Phosphat, Nitrit, Nitrat, Carboxylat und Mischungen davon darstellt; a ist die Ionenladung von X; $R^{1a}$, $R^{2a}$ und $R^{3a}$ sind unabhängig voneinander Wasserstoff, $C_1$-$C_{36}$ Alkyl, $C_1$-$C_{36}$ Alkylhydroxy, $C_1$-$C_{36}$ Alkylamido $R_{(C1-C6)}$, $C_1$-$C_{36}$ Alkylarylamido $R_{(C1-C6)}$ oder $C_1$-$C_{36}$ Alkylhydroxyamido $R_{(C1-C6)}$, wobei $R_{(C1-C6)}$ $C_1$-$C_6$ Alkylen oder Benzyl darstellt; zwei aus $R^{1a}$, $R^{2a}$ und $R^{3a}$ sind unabhängig voneinander $C_{10}$-$C_{30}$ Alkyl, $C_{10}$-$C_{36}$ Alkylhydroxy, $C_{10}$-$C_{36}$ Alkylamido $R_{(C1-C6)}$, $C_{10}$-$C_{36}$ Alkylarylamido $R_{(C1-C6)}$ oder $C_{10}$-$C_{36}$ Alkylhydroxyamido $R_{(C1-C6)}$; das eine verbleibende aus $R^{1a}$, $R^{2a}$ und $R^{3a}$ ist Wasserstoff, $C_1$-$C_8$ Alkyl, $C_1$-$C_8$ Alkylhydroxy, $C_1$-$C_8$ Alkylamido $R_{(C1-C6)}$, $C_1$-$C_8$ Alkylarylamido $R_{(C1-C6)}$ oder $C_1$-$C_8$ Alkylhydroxyamido $R_{(C1-C6)}$; $R^4$, $R^5$, $R^6$ und $R^7$, gleich oder unterschiedlich, sind unabhängig voneinander Wasserstoff, Alkyl, Arylalkyl, Alkylaryl, Fluor, Brom, Chlor, Jod, Acetoxy, Alkylacetoxy, Arylacetoxy, Carboxy, Alkylcarboxy, Hydroxy oder Alkoxyhydroxy; mit der Vorgabe, dass das Dialkylimidazolinquat nicht die Formel

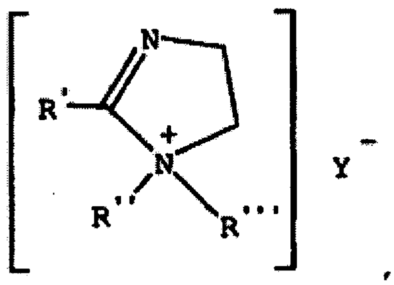

aufweist, wobei R' $C_{11}$-$C_{22}$ Alkyl oder $C_{13}$-$C_{24}$-Alkylhydroxy ist; R" $C_1$-$C_6$ Alkyl ist; und R"' $C_{12}$-$C_{20}$ Alkyl oder $C_{11}$-$C_{22}$ Alkylamido $C_1$-$C_3$ Alkylen ist.

**50.** Kosmetische oder Körperpflegezusammensetzung nach Anspruch 49, wobei $R^{1a}$ $C_{24}$-$C_{36}$ Alkyl ist, $R^{2a}$ unabhängig Wasserstoff oder $C_1$-$C_3$ Alkyl ist, $R^{3a}$ die Struktur

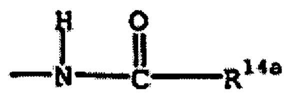

aufweist, wobei $R^{14a}$, welches gleich oder verschieden von $R^{1a}$ sein kann, $C_{24}$-$C_{36}$ Alkyl ist; $R^4$, $R^5$, $R^6$ und $R^7$ alle Wasserstoff sind; n von 1 bis 3 variiert; und m gleich 1 oder 2 ist.

**51.** Kosmetische oder Körperpflegezusammensetzung nach Anspruch 1, 5, 9, 28 oder 37, ferner umfassend zumindest einen hydrophoben Inhaltsstoff.

**52.** Kosmetische oder Körperpflegezusammensetzung nach Anspruch 51, wobei der zumindest eine hydrophobe Inhaltsstoff ausgewählt ist aus der Gruppe bestehend aus Pflanzenextrakten, Vitamin E, Vitamin A, Silikonen, Wachsen und Antioxidantien.

**53.** Kosmetische oder Körperpflegezusammensetzung nach Anspruch 52, wobei der zumindest eine hydrophobe Inhaltsstoff Vitamin E ist.

**54.** Kosmetische oder Körperpflegezusammensetzung nach Anspruch 1, 5, 9, 28 oder 37, ferner umfassend eines oder mehrere Konservierungsmittel, Duftstoffe, Schaumverstärker, Spülungen und Weichmacher.

**55.** Kosmetische oder Körperpflegezusammensetzung, nach Anspruch 1, 5, 9, 28 oder 37, ferner umfassend zumindest einen aktiven Inhaltsstoff, der in der Menge von etwa 0,20 bis etwa 40,0 Gewichtsprozent der Zusammensetzung vorhanden ist.

**56.** Kosmetische oder Körperpflegezusammensetzung nach Anspruch 55, wobei der aktive Inhaltsstoff ausgewählt ist aus der Gruppe bestehend aus einem Sonnenschutzmittel, Pigment, Feuchtigkeitsmittel, Filmbildner, Detergenz,

Dickungsmittel, Emulgator, Antiseptikum, Spülung oder Deodorant.

57. Kosmetische oder Körperpflegezusammensetzung nach Anspruch 1, 5, 9, 28 oder 37, ferner umfassend zumindest einen zusätzlichen grenzflächenaktiven Stoff, der in der Menge von etwa 1 Gewichtsprozent bis etwa 75 Gewichtsprozent der Zusammensetzung vorhanden ist.

58. Kosmetische oder Körperpflegezusammensetzung nach Anspruch 57, wobei der zusätzliche grenzflächenaktive Stoff ausgewählt ist aus der Gruppe bestehend aus nicht-ionischen grenzflächenaktiven Stoffen, anionischen grenzflächenaktiven Stoffen, kationischen grenzflächenaktiven Stoffen und amphoteren grenzflächenaktiven Stoffen.

59. Kosmetische oder Körperpflegezusammensetzung nach Anspruch 1, 5, 9, 28 oder 37, welche ein Rohmaterialquat ist.

60. Kosmetische oder Körperpflegezusammensetzung nach Anspruch 59, ferner einen Trägerstoff umfassend.

61. Kosmetische oder Körperpflegezusammensetzung nach Anspruch 60, die eine kationische Aktivität von größer als 20% aufweist.

62. Kosmetische oder Körperpflegezusammensetzung nach Anspruch 61, wobei die kationische Aktivität größer als 35% ist.

63. Kosmetische oder Körperpflegezusammensetzung nach Anspruch 62, wobei die kationische Aktivität größer als 50% ist.

64. Kosmetische oder Körperpflegezusammensetzung nach Anspruch 1, 5, 9, 28 oder 37, wobei das kosmetische oder Körperpflegeprodukt eine nicht-wässrige körperbehandlungsbezogene Formulierung ist.

65. Kosmetische oder Körperpflegezusammensetzung nach Anspruch 1, 5, 9, 28 oder 37, wobei das kosmetische oder Körperpflegeprodukt eine wässrige körperbehandlungsbezogene Formulierung ist.

## Revendications

1. Composition cosmétique ou de soin personnel, comprenant :

   (a) un mélange de dialkyl imidazolines quaternaires, dans laquelle au moins une partie du mélange comprend au moins une dialkyl imidazoline quaternaire contenant au moins un groupe alkyle en $C_{16}$ à $C_{30}$ ; le taux de substitution en $C_{16}$ à $C_{30}$ du mélange étant d'environ 10 % à environ 95 % par rapport à la plage de substitution de référence en $C_{10+}$, et
   (b) au moins un ingrédient actif choisi dans le groupe constitué par un tensioactif, un émollient, un composé d'écran solaire, un émulsifiant, un agent antipelliculaire, un oxydant-réducteur capillaire, un épaississant, un agent de revitalisation capillaire, un agent de finition capillaire, un agent de coiffage, un agent de nettoyage, un agent de conditionnement cutané, un agent stimulant la croissance des cheveux, un parfum, un pigment, un hydratant, un agent filmogène, un colorant capillaire, un agent de maquillage, un agent actif déodorant, et un agent pharmaceutique utile pour des applications topiques,

   dans laquelle ladite composition de soin personnel est choisie dans le groupe constitué par un shampooing, un revitalisant capillaire, une formule d'écran solaire, une huile de bronzage, une composition pour bain, un nettoyeur pour les mains, une composition anti-transpirante, un parfum, une eau de Cologne, une coldcream, un avant rasage, un déodorant, un onguent pharmaceutique topique, un hydratant cutané, un nettoyant du visage, une crème nettoyante, un gel pour la peau, un produit maquillant, un produit de rouge à lèvres, un mascara et un produit de coloration des cheveux.

2. Composition cosmétique ou de soin personnel selon la revendication 1, dans laquelle ledit taux de substitution en $C_{16}$ à $C_{30}$ est d'environ 15 % à environ 80 %.

3. Composition cosmétique ou de soin personnel selon la revendication 2, dans laquelle ledit taux de substitution en $C_{16}$ à $C_{30}$ est d'environ 20 % à environ 70 %.

4. Composition cosmétique ou de soin personnel selon la revendication 3, dans laquelle ledit taux de substitution en $C_{16}$ à $C_{30}$ est d'environ 35 % à environ 60 %.

5. Composition cosmétique ou de soin personnel selon la revendication 1, dans laquelle ladite composition comprend un mélange de dialkyl imidazolines quaternaires, dans laquelle au moins une partie du mélange comprend au moins une dialkyl imidazoline quaternaire contenant au moins un groupe alkyle en $C_{20}$ à $C_{30}$ ; le taux de substitution en $C_{20}$ à $C_{30}$ du mélange étant d'environ 10 % à environ 95 % par rapport à la plage de substitution de référence en $C_{10+}$.

6. Composition cosmétique ou de soin personnel selon la revendication 5, dans laquelle ledit taux de substitution en $C_{20}$ à $C_{30}$ est d'environ 15 % à environ 80 %.

7. Composition cosmétique ou de soin personnel selon la revendication 6, dans laquelle ledit taux de substitution en $C_{20}$ à $C_{30}$ est d'environ 20 % à environ 70 %.

8. Composition cosmétique ou de soin personnel selon la revendication 7, dans laquelle ledit taux de substitution en $C_{20}$ à $C_{30}$ est d'environ 35 % à environ 60 %.

9. Composition cosmétique ou de soin personnel selon la revendication 1, dans laquelle ladite composition comprend un mélange de dialkyl imidazolines quaternaires, dans laquelle au moins une partie du mélange comprend au moins une dialkyl imidazoline quaternaire contenant au moins un groupe alkyle en $C_{20}$ à $C_{24}$ ; le taux de substitution en $C_{20}$ à $C_{24}$ dudit mélange étant d'environ 10 % à environ 95 % par rapport à la plage de substitution de référence en $C_{10+}$.

10. Composition cosmétique ou de soin personnel selon la revendication 9, dans laquelle ledit taux de substitution en $C_{20}$ à $C_{24}$ est d'environ 15 % à environ 80 %.

11. Composition cosmétique ou de soin personnel selon la revendication 10, dans laquelle ledit taux de substitution en $C_{20}$ à $C_{24}$ est d'environ 20 % à environ 70 %.

12. Composition cosmétique ou de soin personnel selon la revendication 11, dans laquelle ledit taux de substitution en $C_{20}$ à $C_{24}$ est d'environ 35 % à environ 60 %.

13. Composition cosmétique ou de soin personnel selon la revendication 1, 5 ou 9, qui est sensiblement exempte de monoalkyl imidazoline quaternaire.

14. Composition cosmétique ou de soin personnel selon la revendication 1, 5 ou 9, dans laquelle les dialkyl imidazolines quaternaires du mélange possèdent la formule (I) :

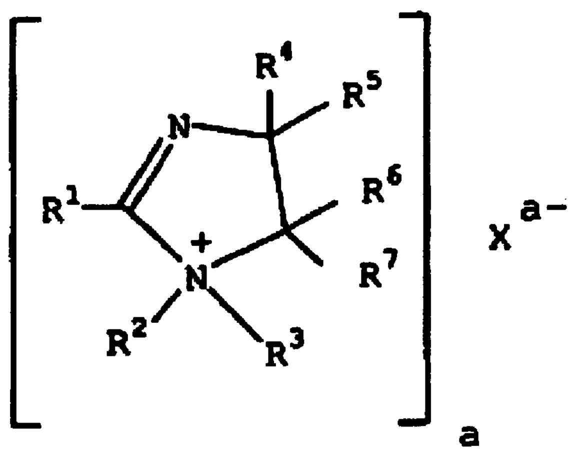

(I) ,

dans laquelle

X est un anion formant un sel choisi dans le groupe constitué par le chlorure, le bromure, l'iodure, le fluorure, le sulfate, le méthylsulfate, le méthanebenzylsulfonate, le phosphate, le nitrite, le nitrate, le carboxylate et leurs mélanges ;
a est la charge ionique de X ;
$R^1$, $R^2$ et $R^3$ sont indépendamment un hydrogène, un alkyle en $C_1$ à $C_{30}$, un alkylhydroxy en $C_1$ à $C_{30}$, un (alkyl en $C_1$ à $C_{30}$)amido $R_{(C1\ à\ C6)}$ un (alkylaryl en $C_1$ à $C_{30}$)amido $R_{(C1\ à\ C6)}$ ou un (alkylhydroxy en $C_1$ à $C_{30}$)amido $R_{(C1\ à\ C6)}$, $R_{(C1\ à\ C6)}$ étant un alkylène en $C_1$ à $C_6$ ou un benzyle ;
deux parmi $R^1$, $R^2$ et $R^3$ sont indépendamment un alkyle en $C_{10}$ à $C_{30}$, un alkylhydroxy en $C_{10}$ à $C_{30}$, un (alkyl en $C_{10}$ à $C_{30}$)amido $R_{(C1\ à\ C6)}$, un (alkylaryl en $C_{10}$ à $C_{30}$)amido $R_{(C1\ à\ C6)}$ ou un (alkylhydroxy en $C_{10}$ à $C_{30}$) amido $R_{(C1\ à\ C6)}$ ;
celui restant parmi $R^1$, $R^2$ et $R^3$ est un hydrogène, un alkyle en $C_1$ à $C_8$, un alkylhydroxy en $C_1$ à $C_8$, un (alkyl en $C_1$ à $C_8$)amido $R_{(C1\ à\ C6)}$, un (alkylaryl en $C_1$ à $C_8$)amido $R_{(C1\ à\ C6)}$ ou un (alkylhydroxy en $C_1$ à $C_8$)amido $R_{(C1\ à\ C6)}$ ;
$R^4$, $R^5$, $R^6$ et $R^7$, identiques ou différents, sont indépendamment un hydrogène, un alkyle, un arylalkyle, un alkylaryle, un fluoro, un bromo, un chloro, un iodo, un acétoxy, un alkylacétoxy, un arylacétoxy, un carboxy, un alkylcarboxy, un hydroxy ou un alcoxyhydroxy.

**15.** Composition cosmétique ou de soin personnel selon la revendication 14, dans laquelle $R^1$ est un alkyle en $C_{10}$ à $C_{30}$ ou un alkylhydroxy en $C_{10}$ à $C_{30}$, $R^2$ est un alkyle en $C_1$ à $C_6$, $R^3$ est un (alkyl en $C_{10}$ à $C_{30}$)amido $R_{(C1\ à\ C6)}$ ou un (alkylhydroxy en $C_{10}$ à $C_{30}$)amido $R_{(C1\ à\ C6)}$, et $R^4$, $R^5$, $R^6$ et $R^7$ sont indépendamment un hydrogène ou un alkyle en $C_1$ à $C_8$.

**16.** Composition cosmétique ou de soin personnel selon la revendication 15, dans laquelle $R^1$ est un alkyle en $C_{10}$ à $C_{30}$, $R^2$ est un méthyle, $R^3$ est un (alkylhydroxy en $C_{10}$ à $C_{30}$) amido (alkylène en $C_1$ à $C_3$), $R^4$, $R^5$, $R^6$ et $R^7$ sont des hydrogènes et X est un chlorure ou un méthylsulfate.

**17.** Composition cosmétique ou de soin personnel selon la revendication 15, dans laquelle $R^1$ est un alkyle en $C_{10}$ à $C_{30}$, $R^2$ est un méthyle, $R^3$ est un (alkyl en $C_{10}$ à $C_{30}$) amido(alkylène en $C_1$ à $C_3$), $R^4$, $R^5$, $R^6$ et $R^7$ sont des hydrogènes et X est un chlorure ou un méthylsulfate.

**18.** Composition cosmétique ou de soin personnel selon la revendication 17, dans laquelle $R^3$ est un (alkyl en $C_{10}$ à $C_{30}$)amidoéthylène.

**19.** Composition cosmétique ou de soin personnel selon la revendication 14, dans laquelle, pour ladite partie dudit mélange, $R^1$ est un alkyle en $C_{16}$ à $C_{30}$ ou un alkylhydroxy en $C_{16}$ à $C_{30}$, $R^2$ est un alkyle en $C_1$ à $C_3$, $R^3$ est un (alkyl en $C_{10}$ à $C_{30}$) amido $R_{(C1\ à\ C6)}$, et $R^4$, $R^5$, $R^6$ et $R^7$ sont des hydrogènes.

**20.** Composition cosmétique ou de soin personnel selon la revendication 14, dans laquelle, pour ladite partie dudit mélange, $R^1$ est un alkyle en $C_{16}$ à $C_{30}$ ou un alkylhydroxy en $C_{16}$ à $C_{30}$, $R^2$ est un alkyle en $C_1$ à $C_3$, $R^3$ est un (alkyl en $C_{16}$ à $C_{30}$)amido $R_{(C1\ à\ C6)}$, et $R^4$, $R^5$, $R^6$ et $R^7$ sont des hydrogènes.

**21.** Composition cosmétique ou de soin personnel selon la revendication 14, dans laquelle, pour ladite partie dudit mélange, $R^1$ est un alkyle en $C_{10}$ à $C_{30}$ ou un alkylhydroxy en $C_{10}$ à $C_{30}$, $R^2$ est un alkyle en $C_1$ à $C_3$, $R^3$ est un (alkyl en $C_{16}$ à $C_{30}$)amido $R_{(C1\ à\ C6)}$, et $R^4$, $R^5$, $R^6$ et $R^7$ sont des hydrogènes.

**22.** Composition cosmétique ou de soin personnel selon la revendication 14, dans laquelle, pour ladite partie dudit mélange, $R^1$ est un alkyle en $C_{20}$ à $C_{30}$ ou un alkylhydroxy en $C_{20}$ à $C_{30}$, $R^2$ est un alkyle en $C_1$ à $C_3$, $R^3$ est un (alkyl en $C_{10}$ à $C_{30}$)amido $R_{(C1\ à\ C6)}$, et $R^4$, $R^5$, $R^6$ et $R^7$ sont des hydrogènes.

**23.** Composition cosmétique ou de soin personnel selon la revendication 14, dans laquelle, pour ladite partie dudit mélange, $R^1$ est un alkyle en $C_{20}$ à $C_{30}$ ou un alkylhydroxy en $C_{20}$ à $C_{30}$, $R^2$ est un alkyle en $C_1$ à $C_3$, $R^3$ est un (alkyl en $C_{20}$ à $C_{30}$)amido $R_{(C1\ à\ C6)}$, et $R^4$, $R^5$, $R^6$ et $R^7$ sont des hydrogènes.

**24.** Composition cosmétique ou de soin personnel selon la revendication 14, dans laquelle, pour ladite partie dudit mélange, $R^1$ est un alkyle en $C_{10}$ à $C_{30}$ ou un alkylhydroxy en $C_{10}$ à $C_{30}$, $R^2$ est un alkyle en $C_1$ à $C_3$, $R^3$ est un (alkyl en $C_{20}$ à $C_{30}$)amido $R_{(C1\ à\ C6)}$, et $R^4$, $R^5$, $R^6$ et $R^7$ sont des hydrogènes.

**25.** Composition cosmétique ou de soin personnel selon la revendication 14, dans laquelle, pour ladite partie dudit mélange, $R^1$ est un alkyle en $C_{20}$ à $C_{24}$ ou un alkylhydroxy en $C_{20}$ à $C_{24}$, $R^2$ est un alkyle en $C_1$ à $C_3$, $R^3$ est un (alkyl en $C_{10}$ à $C_{30}$)amido $R_{(C1\ à\ C6)}$, et $R^4$, $R^5$, $R^6$ et $R^7$ sont des hydrogènes.

**26.** Composition cosmétique ou de soin personnel selon la revendication 14, dans laquelle, pour ladite partie dudit mélange, $R^1$ est un alkyle en $C_{20}$ à $C_{24}$ ou un alkylhydroxy en $C_{20}$ à $C_{24}$, $R^2$ est un alkyle en $C_1$ à $C_3$, $R^3$ est un (alkyl en $C_{20}$ à $C_{24}$)amido $R_{(C1\ à\ C6)}$, et $R^4$, $R^5$, $R^6$ et $R^7$ sont des hydrogènes.

**27.** Composition cosmétique ou de soin personnel selon la revendication 14, dans laquelle, pour ladite partie dudit mélange, $R^1$ est un alkyle en $C_{10}$ à $C_{30}$ ou un alkylhydroxy en $C_{10}$ à $C_{30}$, $R^2$ est un alkyle en $C_1$ à $C_3$, $R^3$ est un (alkyl en $C_{20}$ à $C_{24}$)amido $R_{(C1\ à\ C6)}$, et $R^4$, $R^5$, $R^6$ et $R^7$ sont des hydrogènes.

**28.** Composition cosmétique ou de soin personnel selon la revendication 1, dans laquelle ladite composition comprend un mélange de dialkyl imidazolines quaternaires de formule (II) :

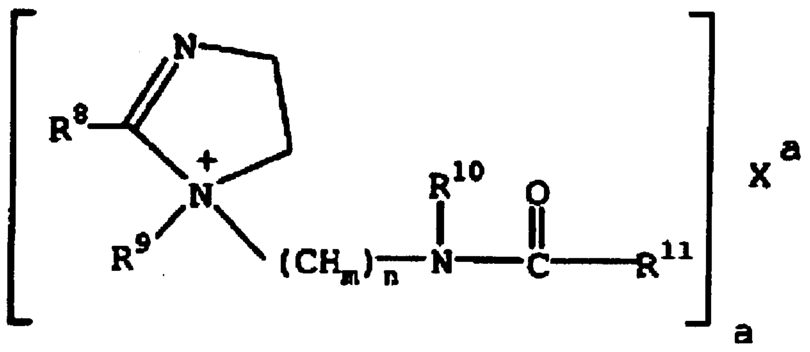

**(II)** ,

dans laquelle

X est un anion formant un sel choisi dans le groupe constitué par le chlorure, le bromure, l'iodure, le fluorure, le sulfate, le méthylsulfate, le méthanebenzylsulfonate, le phosphate, le nitrite, le nitrate, le carboxylate et leurs mélanges ;
a est la charge ionique de X ;
n varie de 1 à 3 :
m est 1 ou 2 ;
$R^8$ et $R^{11}$, identiques ou différents, sont indépendamment un alkyle en $C_{16}$ à $C_{30}$ ou un alkylhydroxy en $C_{16}$ à $C_{30}$ ;
$R^9$ est un hydrogène ou un alkyle en $C_1$ à $C_3$ ;
$R^{10}$ est un hydrogène, un alkyle, un arylalkyle, un alkylaryle, un fluoro, un bromo, un chloro, un iodo, un acétoxy, un alkylacétoxy, un arylacétoxy, un carboxy, un alkylcarboxy, un hydroxy ou un alcoxyhydroxy ; et,
dans laquelle au moins une partie du mélange comprend au moins une dialkyl imidazoline quaternaire dans laquelle au moins un parmi $R^8$ et $R^{11}$ est un alkyle en $C_{16}$ à $C_{24}$ ou un alkylhydroxy en $C_{16}$ à $C_{24}$ ; le taux de substitution en $C_{16}$ à $C_{24}$ dudit mélange étant d'environ 10 % à environ 95 % par rapport à la plage de substitution de référence en $C_{16}$ à $C_{30}$.

**29.** Composition cosmétique ou de soin personnel selon la revendication 28, dans laquelle ledit taux de substitution en $C_{16}$ à $C_{24}$ est d'environ 15 % à environ 80 %.

**30.** Composition cosmétique ou de soin personnel selon la revendication 29, dans laquelle ledit taux de substitution en $C_{16}$ à $C_{24}$ est d'environ 20 % à environ 70 %.

**31.** Composition cosmétique ou de soin personnel selon la revendication 30, dans laquelle ledit taux de substitution en $C_{16}$ à $C_{24}$ est d'environ 35 % à environ 60 %.

**32.** Composition cosmétique ou de soin personnel selon la revendication 28, dans laquelle, pour les dialkyl imidazolines quaternaires du mélange, $R^9$ est un alkyle en $C_1$ à $C_3$, $R^{10}$ est un hydrogène, n est 2 et m est 2.

**33.** Composition cosmétique ou de soin personnel selon la revendication 32, dans laquelle, pour les dialkyl imidazolines quaternaires de ladite partie dudit mélange, $R^8$ est un alkyle en $C_{16}$ à $C_{24}$ ou un alkylhydroxy en $C_{16}$ à $C_{24}$, et $R^{11}$ est un alkyle en $C_{16}$ à $C_{30}$ ou un alkylhydroxy, en $C_{16}$ à $C_{30}$.

**34.** Composition cosmétique ou de soin personnel selon la revendication 32, dans laquelle, pour les dialkyl imidazolines quaternaires de ladite partie dudit mélange, $R^8$ est un alkyle en $C_{16}$ à $C_{24}$ ou un alkylhydroxy en $C_{16}$ à $C_{24}$, et $R^{11}$ est un alkyle en $C_{16}$ à $C_{24}$ ou un alkylhydroxy en $C_{16}$ à $C_{24}$.

**35.** Composition cosmétique ou de soin personnel selon la revendication 32, dans laquelle, pour les dialkyl imidazolines quaternaires de ladite partie dudit mélange, $R^8$ est un alkyle en $C_{16}$ à $C_{30}$ ou un alkylhydroxy en $C_{16}$ à $C_{30}$, et $R^{11}$ est un alkyle en $C_{16}$ à $C_{24}$ ou un alkylhydroxy en $C_{16}$ à $C_{24}$.

**36.** Composition cosmétique ou de soin personnel selon la revendication 28, dans laquelle, pour ladite au moins une dialkyl imidazoline quaternaire de ladite partie dudit mélange, $R^8$ et $R^{11}$ sont tous les deux des radicaux alkyles à chaîne linéaire de formule $-C_{21}H_{43}$, $R^9$ est un méthyle, $R^{10}$ est un hydrogène, n est 2 et m est 2.

**37.** Composition cosmétique ou de soin personnel selon la revendication 1, dans laquelle ladite composition comprend un mélange de dialkyl imidazolines quaternaires de formule (II) :

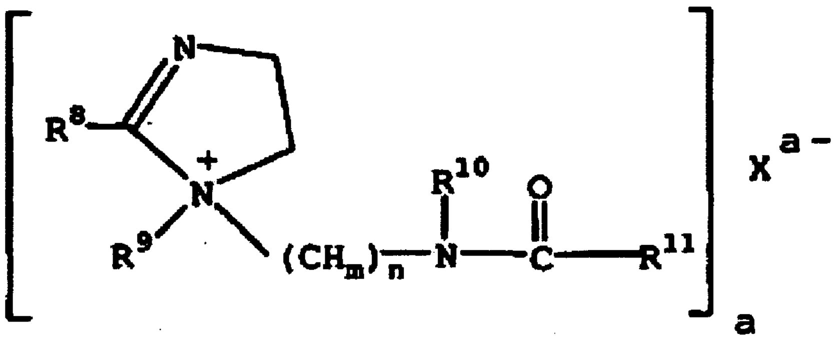

(II)

dans laquelle

X est un anion formant un sel choisi dans le groupe constitué par le chlorure, le bromure, l'iodure, le fluorure, le sulfate, le méthylsulfate, le méthanebenzylsulfonate, le phosphate, le nitrite, le nitrate, le carboxylate et leurs mélanges ;
a est la charge ionique de X ;
n varie de 1 à 3 ;
m est 1 ou 2 ;
$R^8$ et $R^{11}$, identiques ou différents, sont indépendamment un alkyle en $C_{16}$ à $C_{30}$ ou un alkylhydroxy en $C_{16}$ à $C_{30}$ ;
$R^9$ est un hydrogène ou un alkyle en $C_1$ à $C_3$ ;
$R^{10}$ est un hydrogène, un alkyle, un arylalkyle, un alkylaryle, un fluoro, un bromo, un chloro, un iodo, un acétoxy, un alkylacétoxy, un arylacétoxy, un carboxy, un alkylcarboxy, un hydroxy ou un alcoxyhydroxy ; et
dans laquelle au moins une partie du mélange comprend au moins une dialkyl imidazoline quaternaire dans laquelle au moins un parmi $R^8$ et $R^{11}$ est un alkyle en $C_{20}$ à $C_{24}$ ou un alkylhydroxy en $C_{20}$ à $C_{24}$ ; le taux de substitution en $C_{20}$ à $C_{24}$ dudit mélange étant d'environ 10 % à environ 95 % par rapport à la plage de substitution de référence en $C_{16}$ à $C_{30}$.

**38.** Composition cosmétique ou de soin personnel selon la revendication 37, dans laquelle ledit taux de substitution en $C_{20}$ à $C_{24}$ est d'environ 15 % à environ 80 %.

**39.** Composition cosmétique ou de soin personnel selon la revendication 38, dans laquelle ledit taux de substitution en $C_{20}$ à $C_{24}$ est d'environ 20 % à environ 70 %.

**40.** Composition cosmétique ou de soin personnel selon la revendication 39, dans laquelle ledit taux de substitution en $C_{20}$ à $C_{24}$ est d'environ 35 % à environ 60 %.

**41.** Composition cosmétique ou de soin personnel selon la revendication 37, dans laquelle, pour les dialkyl imidazolines quaternaires du mélange, $R^9$ est un alkyle en $C_1$ à $C_3$, $R^{10}$ est un hydrogène, n est 2 et m est 2.

**42.** Composition cosmétique ou de soin personnel selon la revendication 41, dans laquelle, pour les dialkyl imidazolines quaternaires de ladite partie dudit mélange, $R^8$ est un alkyle en $C_{20}$ à $C_{24}$ ou un alkylhydroxy en $C_{20}$ à $C_{24}$, et $R^{11}$ est un alkyle en $C_{16}$ à $C_{30}$ ou un alkylhydroxy en $C_{16}$ à $C_{30}$.

**43.** Composition cosmétique ou de soin personnel selon la revendication 41, dans laquelle, pour les dialkyl imidazolines quaternaires de ladite partie dudit mélange, $R^8$ est un alkyle en $C_{20}$ à $C_{24}$ ou un alkylhydroxy en $C_{20}$ à $C_{24}$, et $R^{11}$ est un alkyle en $C_{20}$ à $C_{24}$ ou un alkylhydroxy en $C_{20}$ à $C_{24}$.

**44.** Composition cosmétique ou de soin personnel selon la revendication 41, dans laquelle, pour les dialkyl imidazolines quaternaires de ladite partie dudit mélange, $R^8$ est un alkyle en $C_{16}$ à $C_{30}$ ou un alkylhydroxy en $C_{16}$ à $C_{30}$, et $R^{11}$ est un alkyle en $C_{20}$ à $C_{24}$ ou un alkylhydroxy en $C_{20}$ à $C_{24}$.

**45.** Composition cosmétique ou de soin personnel selon la revendication 37, dans laquelle, pour ladite au moins une dialkyl imidàzoline quaternaire de ladite partie dudit mélange, $R^8$ et $R^{11}$ sont tous, les deux des radicaux alkyles à chaîne linéaire de formule $-C_{21}H_{43}$, $R^9$ est un méthyle, $R^{10}$ est un hydrogène, n est 2 et m est 2.

**46.** Composition cosmétique ou de soin personnel selon la revendication 27 ou 36, dans laquelle $R^9$ est un méthyle.

**47.** Composition cosmétique ou de soin personnel selon la revendication 27 ou 36, dans laquelle $R^{10}$ est un hydrogène.

**48.** Composition cosmétique ou de soin personnel selon la revendication 27 ou 36, dans laquelle n est 2 et m est 2.

**49.** Composition cosmétique ou de soin personnel selon la revendication 1, dans laquelle ladite au moins une dialkyl imidazoline quaternaire est de formule (IA) :

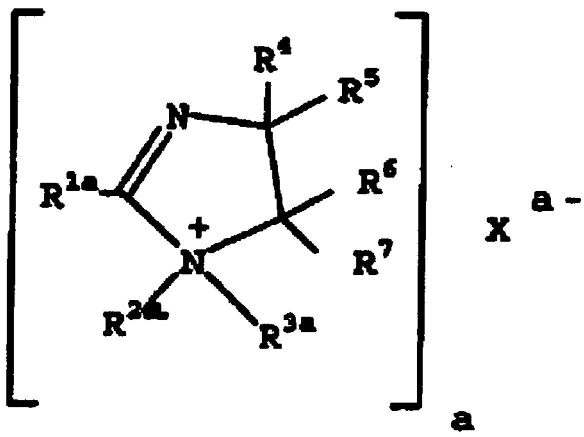

(IA)

,

dans laquelle

X est un anion formant un sel choisi dans le groupe constitué par le chlorure, le bromure, l'iodure, le fluorure, le sulfate, le méthylsulfate, le méthanebenzylsulfonate, le phosphate, le nitrite, le nitrate, le carboxylate et leurs

mélanges ;
a est la charge ionique de X ;
$R^{1a}$, $R^{2a}$ et $R^{3a}$ sont indépendamment un hydrogène, un alkyle en $C_1$ à $C_{36}$, un alkylhydroxy en $C_1$ à $C_{36}$, un (alkyl en $C_1$ à $C_{36}$)amido $R_{(C1\ à\ C6)}$, un (alkylaryl en $C_1$ à $C_{36}$) amido $R_{(C1\ à\ C6)}$ ou un (alkylhydroxy en $C_1$ à $C_{36}$) amido $R_{(C1\ à\ C6)}$, $R_{(C1\ à\ C6)}$ étant un alkylène en $C_1$ à $C_6$ ou un benzyle ;
deux parmi $R^{1a}$, $R^{2a}$ et $R^{3a}$ sont indépendamment un alkyle en $C_{10}$ à $C_{36}$, un alkylhydroxy en $C_{10}$ à $C_{36}$, un (alkyl en $C_{10}$ à $C_{36}$) amido $R_{(C1\ à\ C6)}$, un (alkylaryl en $C_{10}$ à $C_{36}$)amido $R_{(C1\ à\ C6)}$ ou un (alkylhydroxy en $C_{10}$ à $C_{36}$)amido $R_{(C1\ à\ C6)}$ ;
celui restant parmi $R^{1a}$, $R^{2a}$ et $R^{3a}$ est un hydrogène, un alkyle en $C_1$ à $C_8$, un alkylhydroxy en $C_1$ à $C_8$, un (alkyl en $C_1$ à $C_8$)amido $R_{(C1\ à\ C6)}$, un (alkylaryl en $C_1$ à $C_8$)amido $R_{(C1\ à\ C6)}$ ou un (alkylhydroxy en $C_1$ à $C_8$) amido $R_{(C1\ à\ C6)}$ ; $R^4$, $R^5$, $R^6$ et $R^7$, identiques ou différents, sont indépendamment un hydrogène, un alkyle, un arylalkyle, un alkylaryle, un fluoro, un bromo, un chloro, un iodo, un acétoxy, un alkylacétoxy, un arylacétoxy, un carboxy, un alkylcarboxy, un hydroxy ou un alcoxyhydroxy ;
à condition que la dialkyl imidazoline quaternaire ne soit pas de formule

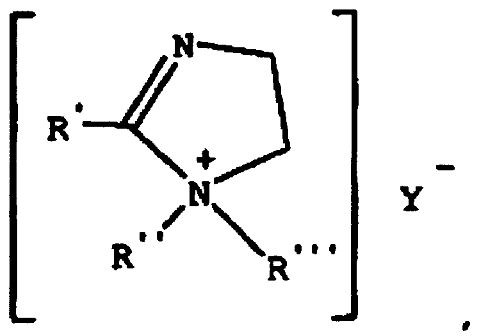

où R' est un alkyle en $C_{11}$ à $C_{22}$ ou un alkylhydroxy en $C_{13}$ à $C_{24}$ : R'' est un alkyle en $C_1$ à $C_6$ : et R''' est un alkyle en $C_{12}$ à $C_{20}$ ou un (alkyl en $C_{11}$ à $C_{22}$)amido-(alkylène en $C_1$ à $C_3$) .

**50.** Composition cosmétique ou de soin personnel selon la revendication 49, dans laquelle $R^{1a}$ est un alkyle en $C_{24}$ à $C_{36}$, $R^{2a}$ est indépendamment un hydrogène ou un alkyle en $C_1$ à $C_3$, $R^{3a}$ possède la structure

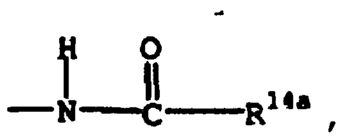

où $R^{14a}$, qui peut être identique ou différent de $R^{1a}$, est un alkyle en $C_{24}$ à $C_{36}$ ; $R^4$, $R^5$, $R^6$ et $R^7$ sont tous des hydrogènes ; n varie de 1 à 3 ; et m est 1 ou 2.

**51.** Composition cosmétique ou de soin personnel selon la revendication 1, 5, 9, 28 ou 37, comprenant en outre au moins un ingrédient hydrophobe.

**52.** Composition cosmétique ou de soin personnel selon la revendication 51, dans laquelle ledit au moins un ingrédient hydrophobe est choisi dans le groupe constitué par des extraits botaniques, la vitamine E, la vitamine A, des siliciums, des cires et des antioxydants.

**53.** Composition cosmétique ou de soin personnel selon la revendication 52, dans laquelle ledit au moins un ingrédient hydrophobe est la vitamine E.

**54.** Composition cosmétique ou de soin personnel selon la revendication 1, 5, 9, 28 ou 37, comprenant en outre un ou plusieurs conservateurs, parfums, renforçateurs de mousse, agents de conditionnement et émollients.

**55.** Composition cosmétique ou de soin personnel selon la revendication 1, 5, 9, 28 ou 37, comprenant en outre au moins un ingrédient actif présent en une quantité située entre environ 0,20 et environ 40,0 pour cent en poids de la composition.

**56.** Composition cosmétique ou de soin personnel selon la revendication 55, dans laquelle ledit ingrédient actif est choisi dans le groupe constitué par un écran solaire, un pigment, un hydratant, un agent filmogène, un détergent, un agent épaississant, un émulsifiant, un agent antiseptique, un agent de conditionnement ou un déodorant.

**57.** Composition cosmétique ou de soin personnel selon la revendication 1, 5, 9, 28 ou 37, comprenant en outre au moins un tensioactif supplémentaire présent en une quantité d'environ 1 % à environ 75 % en poids de la composition.

**58.** Composition cosmétique ou de soin personne selon la revendication 57, dans laquelle ledit tensioactif supplémentaire est choisi dans le groupe constitué par les tensioactifs non ioniques, les tensioactifs anioniques, les tensioactifs cationiques et les tensioactifs amphotères.

**59.** Composition cosmétique ou de soin personnel selon la revendication 1, 5, 9, 28 ou 37, qui est une matière première quaternaire.

**60.** Composition cosmétique ou de soin personnel selon la revendication 59, comprenant en outre un support.

**61.** Composition cosmétique ou de soin personnel selon la revendication 60, ayant une activité cationique supérieure à 20 %.

**62.** Composition cosmétique ou de soin personnel selon la revendication 61, dans laquelle ladite activité cationique est supérieure à 35 %.

**63.** Composition cosmétique ou de soin personnel selon la revendication 62, dans laquelle ladite activité cationique est supérieure à 50 %.

**64.** Composition cosmétique ou de soin personnel selon la revendication 1, 5, 9, 28 ou 37, dans laquelle ledit produit cosmétique ou de soin personnel est une formule topique non aqueuse.

**65.** Composition cosmétique ou de soin personnel selon la revendication 1, 5, 9, 28 ou 37, dans laquelle ledit produit cosmétique ou de soin personnel est une formule topique aqueuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60347170 B
- US 60403037 B
- US 4851141 A
- US 4452732 A
- US 4247538 A
- US 4206195 A
- US 4187289 A
- US 4149551 A
- US 4102795 A
- JP 58144174 A
- US 4855440 A
- US 438631 A
- US 5151210 A
- US 5151209 A
- US 5120532 A
- US 5011681 A
- US 4788006 A
- US 4741855 A
- US 4704272 A
- US 4557853 A
- US 4421769 A
- US 3755560 A
- US 3155591 A
- US 3929678 A
- US 3959461 A
- US 4387090 A
- US 4275055 A
- US 409203 A
- US 2965576 A
- US 2703798 A
- US 1985424 A
- US 2658072 A
- US 2438091 A
- US 2528378 A
- US 4919934 A
- US 5382377 A
- US 5455025 A
- US 5597555 A
- EP 95238 A
- US 4185017 A
- US 4902499 A
- US 4976953 A


### Non-patent literature cited in the description


- **Daniel Swern.** Bailey's Industrial Oil and Fat Products. John Wiley & Sons, 1979, 416-417447, 449-450452
- **W. Schempp ; H. T. Trau.** *Wochenblatt fur Papierfabrikation,* 1981, vol. 19, 726-732
- **J. P. Fischer ; K. Lohr.** Organic Coatings Science Technology. Marcel Dekker, Inc, April 1986, vol. 8, 227-249
- **McCutcheon's.** *Detergents and Emulsifiers,* 1986
- **McCutcheon.** Detergents & Emulsifiers. M.C. Publishing Co, 1979
- **Segarin et al.** Cosmetics Science and Technology. 189
- The CTFA Cosmetic Ingredient Handbook. 1992